(19) Europäisches Patentamt
European Patent Office
Office européen des brevets

(11) Publication number : **0 652 009 A1**

(12) # EUROPEAN PATENT APPLICATION

(21) Application number : **94305833.9**

(22) Date of filing : **05.08.94**

(51) Int. Cl.$^6$ : **A61K 31/47,** A61K 31/44, A61K 31/415, A61K 31/165, A61K 31/335, A61K 31/27, A61K 31/215, A61K 31/38, A61K 31/40, A61K 31/18, A61K 31/495, A61K 31/17, A61K 31/195, C07K 5/02, C07K 5/06, C07K 7/02, C12Q 1/37, G01N 33/68, A61K 38/55

(30) Priority : **09.08.93 US 104293**

(43) Date of publication of application : **10.05.95 Bulletin 95/19**

(84) Designated Contracting States : **AT BE CH DE DK ES FR GB GR IE IT LI LU NL PT SE**

(71) Applicant : **ELI LILLY AND COMPANY**
**Lilly Corporate Center**
**Indianapolis Indiana 46285 (US)**

(71) Applicant : **ATHENA NEUROSCIENCES, INC.**
**800F Gateway Boulevard**
**South San Francisco, CA 94080 (US)**

(72) Inventor : **Dovey, Harry F.**
**24 Duran Court**
**Pacifica, California 94044 (US)**
Inventor : **John, Varghese**
**1722 18th Avenue**
**San Francisco, California 94112 (US)**
Inventor : **Laguzza, Bennett Coleman**
**7340 North Grand Avenue**
**Indianapolis, Indiana 46250 (US)**
Inventor : **Lieberberg, Ivan M.**
**3085 Shasta Road**
**Berkeley, California 94708 (US)**
Inventor : **Little, Sheila Parks**
**4480 North Meridian Street**
**Indianapolis, Indiana 46208 (US)**
Inventor : **Sinha, Sukanto**
**808 Junipero Serra Drive**
**San Francisco, California 9412 (US)**

(74) Representative : **Tapping, Kenneth George**
**Lilly Industries Limited**
**European Patent Operations**
**Erl Wood Manor**
**Windlesham Surrey GU20 6PH (GB)**

(54) **Identification and use of protease inhibitors.**

(57) β-amyloid peptide (βAF) production in cell culture and in vivo is inhibited by administering aspartyl protease inhibitors, particularly inhibitors of proteases of cathepsin D. Useful aspartyl protease inhibitors can be selected in a two-step assay, where test compounds are first screened for aspartyl protease inhibition activity in vitro in noncellular assays. Those test compounds which are found to display protease inhibition activity are then tested in cellular assays for βAP production inhibition. Those test compounds which are capable of inhibiting intracellular β-amyloid production may be incorporated in pharmaceutical compositions.

EP 0 652 009 A1

The present invention relates generally to methods and compositions for inhibiting β-amyloid peptide (βAP) production in cells. In particular, the present invention relates to the screening and identification of cellular protease inhibitors, specifically aspartyl protease inhibitors, which are capable of inhibiting the intracellular production of βAP, and the use of such cellular protease inhibitors in methods for inhibiting βAP production.

Alzheimer's Disease (AD) is a degenerative brain disorder characterized clinically by progressive loss of memory, cognition, reasoning, judgment and emotional stability that gradually leads to profound mental deterioration and ultimately death. AD is a common cause of progressive mental failure (dementia) in aged humans and is believed to represent the fourth most common medical cause of death in the United States. AD has been observed in races and ethnic groups worldwide and presents a major present and future public health problem. The disease is currently estimated to affect about two to three million individuals in the United States alone. To date, AD has proven to be incurable.

The brains of individuals with AD exhibit neuronal degeneration and characteristic lesions variously referred to as amyloid plaques, vascular amyloid angiopathy, and neurofibrillary tangles. Large numbers of these lesions, particularly amyloid plaques and neurofibrillary tangles, are generally found in several areas of the human brain important for memory and cognitive function in patients with AD. Smaller numbers of these lesions in a more restricted anatomical distribution are found in the brains of most aged humans who do not have clinical AD. Amyloid plaques and vascular amyloid angiopathy also characterize the brains of individuals with Trisomy 21 (Down's Syndrome) and Hereditary Cerebral Hemorrhage with Amyloidosis of the Dutch-Type (HCHWA-D). At present, a definitive diagnosis of AD usually requires observing the aforementioned lesions in the brain tissue of patients who have died with the disease or, rarely, in small biopsied samples of brain tissue taken during an invasive neurosurgical procedure.

The principal protein constituent of the amyloid plaques and vascular amyloid angiopathy characteristic of AD and the other disorders mentioned above is an approximately 4.2 kilodalton (kD) protein of about 39-43 amino acids designated the β-amyloid peptide (βAP). βAP is an approximately 39-43 amino acid fragment of a large membrane-spanning glycoprotein, referred to as the β-amyloid precursor protein (APP).

βAP is further characterized by its relative mobility in SDS-polyacrylamide gel electrophoresis or in high performance liquid chromatography (HPLC). βAP can occur in a filamentous polymeric form which exhibits the Congo-red and thioflavin-S dye-binding and apple green birefringence characteristics of amyloid. βAP can also occur in a non-filamentous form ("preamyloid" or "amorphous" or "diffuse" deposits) in tissue, in which form no detectable birefringent staining by Congo red occurs. A portion of this protein in the insoluble form obtained from meningeal blood vessels is described in U.S. Patent No. 4,666,829.

APP is normally produced by cells in many tissues of various mammals, including humans. APP is encoded by a gene on the long arm of human chromosome 21. Knowledge of the structure of the gene encoding APP has demonstrated that βAP arises as a peptide fragment that is cleaved from APP by one or more heretofore unidentified proteases. This cleavage appears to occur intracellularly. The precise biochemical mechanism by which the βAP fragment is cleaved from APP and subsequently deposited as amyloid plaques extracellulary in the cerebral tissue and in the walls of cerebral and meningeal blood vessels is under investigation.

Several lines of evidence indicate that progressive cerebral deposition of βAP plays a seminal role in the pathogenesis of AD and can precede cognitive symptoms by years or decades. See, Selkoe, (1991) Neuron 6:487). Recently, it has been shown that βAP is released from neuronal cells grown in culture and is present in cerebrospinal fluid (CSF) of both normal individuals and AD patients. See, Seubert et al., (1992) Nature 359:325-327.

Certain inherited mutations which occur in the APP gene can cause AD and AD-related conditions. For example, missense DNA mutations at amino acid 717 of the 770-amino acid isoform of APP were found in affected members but not unaffected members of several families with a genetically determined (familial) form of AD. See, Goate et al., (1991) Nature 349:704-706; Chartier Harlan et al., (1991) Nature 353:844-846; and Murrell et al., (1991) Science 254:97-99). In addition, there is a double mutation referred to as the Swedish variant or the Swedish mutation in which $Lys_{595}$-$Met_{596}$ mutated to $Asp_{595}$-$Leu_{596}$ in the 695-amino acid isoform which is present in a Swedish family subject to a familial form of AD. See, Mullan et al., (1992) Nature Genet 1:345-347.

Genetic linkage analyses have demonstrated that these mutations, as well as certain other mutations in the APP gene, are the molecular cause of AD in the affected members of such families. Furthermore a mutation at amino acid 693 of the 770-amino acid isoform of APP has been identified as the cause of the βAP deposition disease, HCHWA-D and a change from alanine to glycine at amino acid 692 appears to cause a phenotype that resembles AD in some patients but HCHWA-D in others. See, Younkin et al., (1993) Science 259:514-516.

Despite the progress that has been made in understanding the underlying mechanisms of AD and other βAP-related diseases, there remains a need to develop compositions and methods for treatment of these diseases. Treatment methods could advantageously be based on drugs which are capable of inhibiting the generation of βAP. To identify such drugs, it is desirable to develop screening methods which are rationally based on an improved understanding of intracellular βAP production, and in particular based on an understanding of the βAP proteolytic cleavage pathway including identification of the enzyme(s) responsible. Drugs identified by such screening methods may then be used to inhibit βAP production using *in vitro* cellular models, mammals suffering from AD and other βAP-related diseases, and animal models useful for studying β-amyloid production and inhibitors thereof.

APP and βAP production are reviewed in Sinha and Lieberburg, (1992) Neurodegeneration 1:169-175 and Marx, (1993) Science 259:457-458. An apparent lysosomal APP processing pathway which produces βAP is described in Estus et al., (1992) Science 255:726-728 and Golde et al., (1992) Science 255:728-730. The release of soluble βAP into cell culture media is described in Haas et al., (1992) Nature 359:322-325 and Shoji et al., (1992) Science 258:126-129, and release into human and animal CSF is described in Seubert et al., (1992) Nature 359:325-327. Transformation of human cell lines with a mutant APP gene associated with familial AD has been shown to cause overproduction of βAP, as described in Citron et al., (1992) Nature 360:672-674 and Younkin et al., *supra*. The presence of numerous lysosomal proteases, including cathepsin D, in senile plaque associated with AD is described in Cataldo et al., (1990) Proc. Natl. Acad. Sci. USA 87:3861-3865 and (1991) Proc. Natl. Acad. Sci. USA 88:10998-11002. The nature of aspartyl proteases (also referred to as aspartic proteases) and aspartyl protease inhibitors is described in Tang and Wong, (1987) J. Cell. Biochem. 33(1):53-63; Rich, "Inhibitors of Aspartic Proteases," in *Proteinase Inhibitors*, Barrett and Salvesen, eds., Elsevier Science Publishers BV, Amsterdam, 1986, pp. 179-217; and van Noort et al., (1992) J. Biol. Chem. 264:14159-14164. The localization of cathepsin D in endosomal structures with the cell has been described in Diment et al., (1992) J. Biol. Chem. 263:6901-6907.

The present invention provides a method of inhibiting β-amyloid peptide (βAP) production comprising administering to cells an effective amount of an aspartyl protease inhibitor.

The present invention also provides a method of inhibiting βAP production comprising administering to a mammal in need thereof an effective amount of an aspartyl protease inhibitor.

The present invention also provides a method of inhibiting the deposition of amyloid plaque comprising administering to a mammal in need thereof an effective amount of an aspartyl protease inhibitor.

The present invention also provides a method of preventing or treating Alzheimer's Disease (AD) comprising administering to a mammal in need thereof an effective amount of an aspartyl protease inhibitor.

The present invention also provides a method of preventing or treating βAP-related diseases comprising administering to a mammal in need thereof an effective amount of an aspartyl protease inhibitor.

The present invention further provides the aforementioned methods wherein the aspartyl protease inhibitor is a compound of formula I

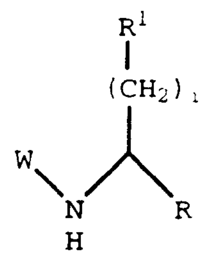

(I)

wherein:

i is 1, 2, 3, or 4;

$R^1$ is $C_1$-$C_6$ alkyl, aryl, cyclohexyl or -S-$R^{1x}$, where

$R^{1x}$ is aryl, or cyclohexyl;

W is

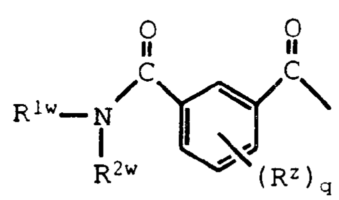

;

where

$R^{1w}$ and $R^{2W}$ are independently selected from hydrogen, $C_1$-$C_6$ alkyl, aryl($C_1$-$C_4$)alkyl, heterocycle($C_1$-$C_4$)alkyl, unsaturated heterocycle($C_1$-$C_4$)alkyl, or $R^{1w}$ and $R^{2w}$ are combined with the nitrogen atom to which they are attached to form a heterocycle or unsaturated heterocycle with the proviso that the nitrogen atom may not be quaternized;

q is 0, 1, 2, 3, or 4;

$R^z$ is hydrogen, halo, $C_1$-$C_6$ alkyl, $C_1$-$C_6$ alkoxy, $C_1$-$C_6$ alkylthio, or amino;

or W is

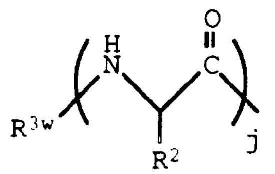

;

where

j is 1, or 2;

$R^{3w}$ is hydrogen, formyl, carbamoyl, or $-(B)_e$-X-$R^0$,

where

B is -C(O)-, or $-S(O)_k-$;

k is 0, 1, or 2;

e is 0, or 1;

X is $-(CH_2)_g-$, $-(CH_2)_m-O-(CH_2)_n-$, or $-(CH_2)_m-NR^x-(CH_2)_n-$, where

g is 0, 1, or 2;

m and n are independently 0, 1, or 2;

$R^x$ is hydrogen, or $C_1$-$C_4$ alkyl;

$R^0$ is $C_1$-$C_6$ alkyl, $C_1$-$C_4$ alkoxycarbonyl, formyl, $C_2$-$C_6$ alkanoyl, amino, trifluoromethyl, $C_1$-$C_4$ alkylamino, di($C_1$-$C_4$)alkylamino, aryl, heterocycle, or unsaturated heterocycle;

each $R^2$ is independently an amino acid side chain, $-CH_2CH_3$, $-CH_2CH_2CH_3$, $-CH_2(CH_2)_2CH_3$, $-C(CH_3)_3$, $-CH_2SO_2NH_2$, $-CH_2CN$, $-(CH_2)_r-X^1-R^{2a}$, $-(CH_2)_s-C(O)NR^{2b}R^{2c}$, or $-(CH_2)_t-S(O)_k-[1-N(R^{2d})$-tetrazol-5-yl], where

r, s and t are independently 0, 1, or 2;

$X^1$ is a bond, divalent($C_1$-$C_4$)alkyl, divalent($C_2$-$C_4$)alkenyl, divalent($C_2$-$C_4$)alkynyl, -C(O)-O-, -O-C(O)-, $-C(O)NR^{2b}-$, $-NR^{2b}-C(O)-$, $-NR^{2b}-$, -C(O)-, -O-, or $-S(O)_k-$;

$R^{2a}$ is $C_1$-$C_6$ alkyl, aryl, aryl($C_1$-$C_4$)alkyl, heterocycle, heterocycle($C_1$-$C_4$)alkyl, unsaturated heterocycle, or unsaturated heterocycle($C_1$-$C_4$)alkyl;

$R^{2b}$ is hydrogen, or $C_1$-$C_4$ alkyl;

$R^{2c}$ is amino, $C_1$-$C_6$ alkoxy, $C_1$-$C_6$ alkyl, $-(CH_2)_m$-di($C_1$-$C_4$)alkylamino, aryl, aryl($C_1$-$C_4$)alkyl, heterocycle, heterocycle($C_1$-$C_4$)alkyl, unsaturated heterocycle, or unsaturated heterocycle($C_1$-$C_4$)alkyl;

$R^{2d}$ is hydrogen, $C_1$-$C_6$ alkyl, aryl, unsaturated heterocycle, unsaturated heterocycle($C_1$-$C_4$)-alkyl, or aryl($C_1$-$C_4$)alkyl;

R is

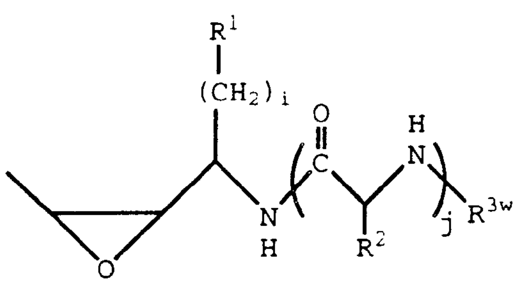

,

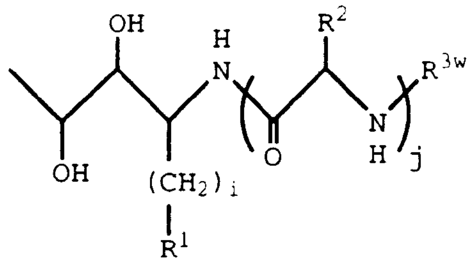

,

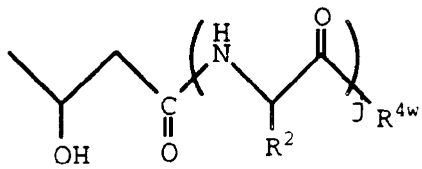

,

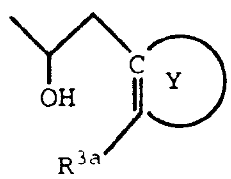

,

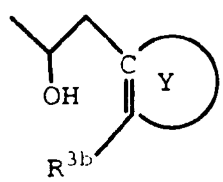

or

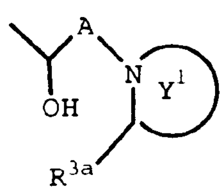

;

where:

A is $-CH_2-$ or

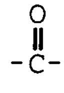

;

Y is aryl or unsaturated heterocycle;

$Y^1$ is heterocycle;

$R^{4w}$ is hydroxy or amino;

$R^{3a}$ is a group having the formula

1) $-C(O)-NR^4R^4$,

2)

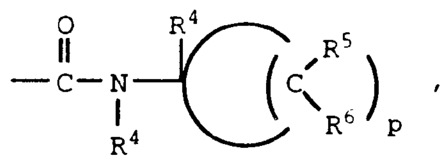

or

3)

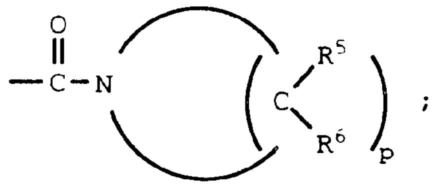

$R^{3b}$ is a group having the structure:

1)

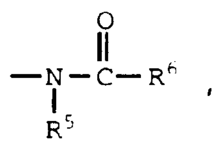

2)

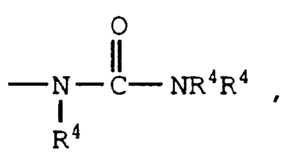

or

3)

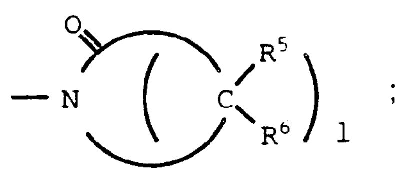

p is 4 or 5;

l is 3, 4 or 5;

$R^4$ at each occurrence is independently hydrogen, $C_1$-$C_6$ alkyl or hydroxy($C_1$-$C_4$)alkyl;

$R^5$ and $R^6$ are independently selected from hydrogen, hydroxy, $C_1$-$C_6$ alkyl, $C_1$-$C_6$ alkoxy, amino, $C_1$-$C_4$ alkylamino, hydroxy($C_1$-$C_4$)alkyl, carboxy, $C_1$-$C_4$ alkoxycarbonyl, carbamoyl, N-($C_1$-$C_4$)alkylcarbamoyl, aryl, heterocycle or unsaturated heterocycle;

with the provisos that:

(1) when R is

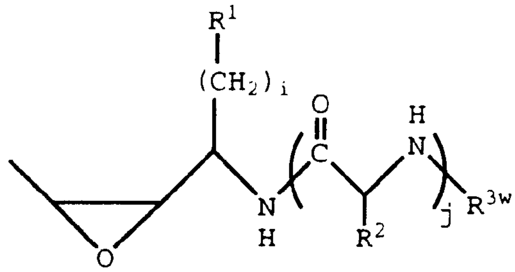

,

then

W is

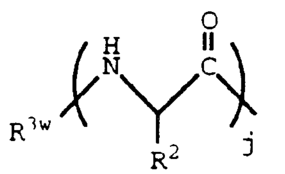

;

$R^{3w}$ is formyl, carbamoyl or $-(B)_e-X-R^0$, where

e is 1; and

B is -C(O)-;

(2) when R is

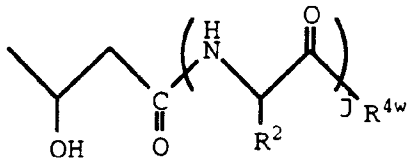

;

then

W is

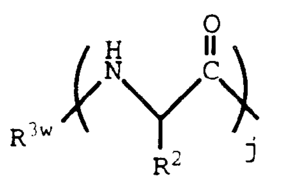

;

and

j is 2; and

(3) when R is

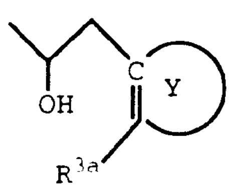

,

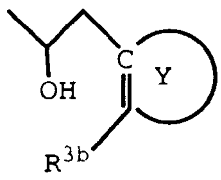

or

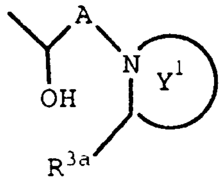

;

then

i is 1; and

$R^1$ is aryl, cyclohexyl, or -S-$R^{1x}$, where

$R^{1x}$ is aryl, or cyclohexyl;

or a pharmaceutically acceptable salt thereof.

The present invention further provides the aforementioned methods wherein the aspartyl protease inhibitor is a compound of formula II

$$R^7\text{-Gly-}(R^8)_f\text{-Thr-OH} \qquad (II)$$

wherein:

$R^7$ is $C_1$-$C_6$ alkyl, formyl or $C_2$-$C_6$ alkanoyl;

f is 1-6;

each $R^8$ is independently an amino acid residue, statine or a statine derivative;

with the provisos that:

(1) there must be at least one statine or a statine derivative;

(2) when f is 2-6, then the total number of statine and statine derivatives in -$(R^8)_f$- cannot exceed 3;

or a pharmaceutically acceptable salt thereof.

In addition, the present invention provides a process for identifying a βAP production inhibitor, comprising:

(1) administering an aspartyl protease inhibitor to a test system;

(2) measuring βAP production in the test system; and

(3) comparing βAP production in the test system to βAP production in a control test system in which no such aspartyl protease inhibitor has been administered.

Finally, the present invention provides a process of identifying βAP production inhibitors, comprising:

(1) measuring βAP production in a cellular assay; and

(2) measuring βAP production in a body fluid of a test animal.

The following terms and phrases set forth in the specification and claims are defined as follows. It is to be understood that in the field of this invention, there are a number of terms which, in fact, refer to the same moiety. The use of a particular term in the context of the present application should not be considered a limitation.

The term "β-amyloid peptide" (βAP) as used herein refers to an approximately 4.2 kD protein which, in the brains of persons afflicted with AD, Down's Syndrome, HCHWA-D, and in some normal aged subjects, forms a subunit of the amyloid filaments comprising the amyloid plaques (senile plaques) and the vascular amyloid angiopathy (amyloid deposits in small cerebral and meningeal blood vessels). Typical abbreviations for βAP used by persons in the art include Aβ, AβP and β/A4, among others. It can be found in soluble form in the extracellular fluid of cultured cells and in body fluids of mammals, such as aged humans and humans suffering from AD and βAP-related conditions.

βAP includes within its definition related βAP sequences that result from mutations in the βAP region of the normal gene and are substantially homologous to the 43-amino acid sequence. The 43-amino acid sequence for βAP is:

1
Asp Ala Glu Phe Arg His Asp Ser Gly Tyr

11
Glu Val His His Gln Lys Leu Val Phe Phe

21
Ala Glu Asp Val Gly Ser Asn Lys Gly Ala

31
Ile Ile Gly Leu Met Val Gly Gly Val Val

41
Ile Ala Thr [SEQ ID NO:1]

The term "β-amyloid precursor protein" (APP) as used herein refers to a polypeptide that is encoded by a gene of the same name localized in humans on the long arm of chromosome 21 that includes βAP within the carboxyl one-third of its length. APP is a glycosylated, single-membrane-spanning protein expressed in a wide variety of cells in many mammalian tissues. Examples of specific isotypes of APP which are known to exist in humans include the 695-amino acid polypeptide described by Kang et al., (1987) Nature 325:733-736 which is designated as the "normal" APP; the 751-amino acid polypeptide described by Ponte et al., (1988) Nature 331:525-527 (1988) and Tanzi et al.,(1988) Nature 331:528-530; and the 770-amino acid polypeptide described by Kitaguchi et al., (1988) Nature 331:530-532. Examples of specific variants of APP include point mutations which can differ in both position and phenotype. Cf, Hardy, (1992) Nature Genet. 1:233-234).

It has been observed that intracellular βAP production results from cleavage of the APP by a cellular protease. The cellular protease appears to be an aspartyl protease, more particularly cathepsin D. Furthermore, it has been observed that pepstatin, a known cathepsin D inhibitor, having the following sequence:

Iva-Val-Val-Sta-Ala-Sta-OH

where Iva is isovaleric acid and Sta is statine, is homologous with the six carboxy-terminal amino acids of βAP:

38 39 40 41 42 43
Gly-Val-Val-Ile-Ala-Thr-OH. [SEQ ID NO:2]

Preferred cathepsin D inhibitors include compounds which mimic the carboxy-terminus of βAP with inclusion of at least one statine or statine derivative, preferably at a location analogous to amino acids 41 or 42. In addition, preferred cathepsin D inhibitors are those compounds which have pharmacological properties which enhance the compound's ability to inhibit cathepsin D or the formation of βAP. Examples of pharmacological properties include oral absorption, the ability to cross a cellular membrane or the blood/brain barrier and the like.

The term "βAP-related condition" as used herein is defined as including AD (includes familial AD), Down's Syndrome, HCHWA-D, advanced aging of the brain and the like.

The terms "conditioned culture medium" and "culture medium" as used herein refer to the aqueous extracellular fluid which surrounds cells grown *in vitro* and which contains, among other constituents, proteins and peptides secreted by the cells.

The term "body fluid" as used herein refers to those fluids of a mammalian host which may contain measurable amounts of βAP and βAP fragments, specifically including blood, CSF, urine, and peritoneal fluid. The term "blood" refers to whole blood, as well as blood plasma and serum.

The term "cellular characteristic" refers to a constituent which is related to βAP production and is found in a conditioned culture medium or in a body fluid. Typical cellular characteristics include βAP and βAP fragments and other like βAP processed intermediates.

The term "Swedish mutation" refers to a mutation in the human gene encoding APP which results in an inherited, familial form of AD. The mutation occurs at $Lys_{595}$-$Met_{596}$ of the normal APP gene, where a substitution to $Asn_{595}$-$Leu_{596}$ occurs. It has been found that human cell lines transfected with this mutation will overproduce βAP, secreting the βAP into the conditioned culture medium. Other familial mutations of the APP gene which could be used to transfect cell lines to produce βAP are known in the art. Cf, Hardy, *supra*.

The term "$C_1$-$C_6$ alkyl" represents a straight or branched alkyl chain having from one to six carbon atoms. Typical $C_1$-$C_6$ alkyl groups include methyl, ethyl, propyl, isopropyl, butyl, isobutyl, *sec*-butyl, *t*-butyl, pentyl, *neo*-pentyl, hexyl and the like. The term "$C_1$-$C_6$ alkyl" includes within its definition the term "$C_1$-$C_4$ alkyl".

The term "divalent($C_1$-$C_4$)alkyl" represents a straight or branched divalent alkyl chain having from one to four carbon atoms. Typical divalent($C_1$-$C_4$)alkyl groups include methylene, ethylene, propylene, 2-methylpropylene, butylene and the like.

The term "halo" represents chloro, fluoro, bromo or iodo.

The term "halo($C_1$-$C_4$)alkyl" represents a straight or branched alkyl chain having from one to four carbon atoms with 1, 2 or 3 halogen atoms attached to it. Typical halo($C_1$-$C_4$)-alkyl groups include chloromethyl, 2-bromoethyl, 1-chloroisopropyl, 3-fluoropropyl, 2,3-dibromobutyl, 3-chloroisobutyl, iodo-*t*-butyl, trifluoromethyl and the like.

The term "$C_1$-$C_6$ alkylthio" represents a straight or branched alkyl chain having from one to four carbon atoms attached to a sulfur atom. Typical $C_1$-$C_6$ alkylthio groups include methylthio, ethylthio, propylthio, isopropylthio, butylthio and the like. The term "$C_1$-$C_6$ alkylthio" includes within its definition the term "$C_1$-$C_4$ alkylthio".

The term "$C_1$-$C_4$ alkylamino" represents a straight or branched alkyl chain having from one to four carbon atoms attached to an amino group. Typical $C_1$-$C_4$ alkylamino groups include methylamino, ethylamino, propylamino, isopropylamino, butylamino, *sec*-butylamino and the like.

The term "di($C_1$-$C_4$)alkylamino" represents a straight or branched dialkylamino chain having two alkyl chains, each having independently from one to four carbon atoms attached to a common amino group. Typical di($C_1$-$C_4$)alkylamino groups include dimethylamino, ethylmethylamino, methylisopropylamino, *t*-butylisopropylamino, di-*t*-butylamino and the like.

The term "$C_2$-$C_6$ alkanoyl" represents a straight or branched alkyl chain having from one to five carbon atoms attached to a carbonyl moiety. Typical $C_2$-$C_6$ alkanoyl groups include ethanoyl, propanoyl, isopropanoyl, butanoyl, *t*-butanoyl, pentanoyl, hexanoyl, 3-methylpentanoyl and the like.

The term "N-($C_1$-$C_4$)alkylcarbamoyl" represents a straight or branched alkyl chain having from one to four carbon atoms attached to the nitrogen atom of a carbamoyl moiety. Typical N-($C_1$-$C_4$)alkylcarbamoyl groups include N-methylcarbamoyl, N-ethylcarbamoyl, N-propylcarbamoyl, N-isopropylcarbamoyl, N-butylcarbamoyl, N-*t*-butylcarbamoyl and the like.

The term "heterocycle" refers to an unsubstituted or substituted stable 5- to 7-membered monocyclic or 7- to 10-membered bicyclic heterocyclic ring which is saturated and which consists of carbon atoms and from one to three heteroatoms selected from the group consisting of nitrogen, oxygen or sulfur, and wherein the nitrogen and sulfur heteroatoms may optionally be oxidized, and the nitrogen heteroatom may optionally be quaternized. The heterocyclic ring may be attached at any heteroatom or carbon atom which affords a stable structure. The heterocycle is unsubstituted or substituted with 1, 2 or 3 substituents independently selected from halo, hydroxy, trifluoromethyl, $C_1$-$C_4$ alkyl, $C_1$-$C_4$ alkoxy, carboxy, $C_1$-$C_4$ alkoxycarbonyl, carbamoyl or amino.

The term "unsaturated heterocycle" refers to an unsubstituted or substituted stable 5- to 7-membered monocyclic or 7- to 10-membered bicyclic heterocyclic ring which has one or more double bonds and which consists of carbon atoms and from one to three heteroatoms selected from the group consisting of nitrogen, oxygen or sulfur, and wherein the nitrogen and sulfur heteroatoms may optionally be oxidized, and the nitrogen heteroatom may optionally be quarternized. The unsaturated heterocyclic ring may be attached at any heteroatom or carbon atom which affords a stable structure. The unsaturated heterocycle is unsubstituted or substituted with 1, 2 or 3 substituents independently selected from halo, hydroxy, trifluoromethyl, $C_1$-$C_4$ alkyl, $C_1$-$C_4$ alkoxy, carboxy, $C_1$-$C_4$ alkoxycarbonyl, carbamoyl or amino.

Examples of such heterocycles and unsaturated heterocycles include piperidinyl, piperazinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazolyl, benzopyranyl, benzothiazolyl, furyl, tetrahydrofuryl, tetrahydropyranyl, thienyl, benzothienyl, thiamorpholinyl, thiamorpholinylsulfoxide, thiamorpholinylsulfone, oxadiazolyl, triazolyl, tetrahydroquinolinyl, tetrahydrisoquinolinyl, 3-methylimidazolyl, 3-methoxypyridyl, 4-chloroquinolinyl, 4-aminothiazolyl, 8-methylquinolinyl, 6-chloroquinoxalinyl, 3-ethylpyridyl, 6-methoxybenzimidazolyl, 4-hydroxyfuryl, 4-methylisoquinolinyl, 6,8-dibromoquinolinyl, 4,8-dimethylnaphthyl, 2-methyl-1,2,3,4-tetrahydroisoquinolinyl, N-methylquinolin-2-yl, 2-*t*-butoxycarbonyl-1,2,3,4-isoquinolin-7-yl and the like.

The term "aryl" represents a phenyl or naphthyl ring which is unsubstituted or substituted with 1, 2 or 3

substituents independently selected from halo, hydroxy, trifluoromethyl, $C_1$-$C_4$ alkyl, $C_1$-$C_4$ alkoxy, carboxy, $C_1$-$C_4$ alkoxycarbonyl, carbamoyl, amino or morpholino($C_1$-$C_4$)-alkoxy. Typical aryl groups include 4-methylphenyl, 3-ethylnaphthyl, 2,5-dimethylphenyl, 8-chloronaphthyl, 3-aminonaphthyl, 4-carboxyphenyl and the like.

The term "amino acid side chain" represents the distinctive atom or group bonded to an α-carbon atom also having bonded thereto a carboxyl group and an amino group. These side chains are selected from those found on the twenty naturally occurring amino acids.

The term "amino acid residue" includes within its definition the naturally occurring amino acids and the corresponding positional isomers and variants thereof. Typical positional isomers and variants include 2-aminoadipic acid (Aad), 3-aminoadipic acid (bAad), β-alanine (bAla), 2-aminobutyric acid (Abu), 4-aminobutyric acid (4Abu), 6-aminocaproic acid (Acp), 2-aminoheptanoic acid (Ahe), 2-aminoisobutyic acid (Aib), 3-aminoisobutyric acid (bAib), 2-aminopimelic acid (Apm), 2,4-diaminobutyric acid (Dbu), desmosine (Des), 2,2'-diaminopimelic acid (Dpm), 2,3-diaminopropionic acid (Dpr), N-ethylglycine (EtGly), N-ethylasparagine (EtAsn), hydroxylysine (Hyl), allohydroxylysine (aHyl), 3-hydroxyproline (3Hyp), 4-hydroxyproline (4Hyp), Isodesmosine (Ide), allo-isoleucine (alle), N-methylglycine (MeGly), N-methylisoleucine (Melle), N-methyllysine (MeLys), norvaline (Nva), norleucine (Nle), ornithine (Orn), cyanoalanine (CA), γ-carboxyglutamate, O-phosphoserine, α-naphthylalanine (NA), β-naphthylalanine (bNA), S-galactosyl cysteine, glycinamide, N-formylmethionine, tyrosine-O-sulfate and the like. These amino acid residues may be in either the D or L configuration. Unless otherwise specified, a reference to an amino acid will refer to the L configuration.

The term "protease" as used herein refers to a proteolytic enzyme which weakens or breaks the peptide linkages in certain protein substrates. The term "lysosomal protease" refers to those proteases which are characteristically found within the cellular lysosome. The term "aspartyl protease" is used herein to define a group of proteolytic enzymes of the pepsin family that usually function under acidic conditions. The occurrence of aspartyl proteases is frequently limited to particular locations in the body, such as the stomach, and cellular lysosomes. Aspartyl proteases are classified as EC3.4.23. Of particular interest to the present invention, cathepsin D is an aspartyl protease which functions in the cellular lysosomes. The nature and function of aspartyl proteases, including cathepsin D, are described in Tang and Wong, *supra*, the full disclosure of which is incorporated herein by reference.

The term "protease inhibitor" as used herein, refers to any compound, molecule, or substance which inhibits the function of a protease. In particular, cellular protease inhibitors according to the present invention inhibit the function of a cellular protease which is involved in or responsible for the intracellular processing of APP into βAP. Likewise aspartyl protease inhibitors inhibit the function of an aspartyl protease which is involved in or responsible for the intracellular processing of APP into βAP, and cathepsin D inhibitors inhibit the function of cathepsin D. It is believed that the cellular proteases which are primarily responsible for βAP production are aspartyl proteases, more specifically cathepsin D. Suitable aspartyl protease inhibitors may be selected in accordance with the selection methods of the present invention. A review of aspartyl protease inhibitors in general, and cathepsin D in particular, is found in Rich, "Inhibitors of Aspartic Proteinases," *supra*, the full disclosure of which is incorporated herein by reference.

The term "statine derivative" refers to a compound of the formula

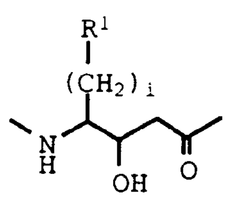

where i and $R^1$ are as defined above for formula I. Statine (Sta) is represented where i is 1 and $R^1$ is isopropyl. 4-amino-3-hydroxy-5-phenylpentanoic acid residue (AHPPA) is represented where i is 1 and $R^1$ is phenyl.

Preferred aspartyl protease inhibitors are the cathepsin D inhibitors. Preferred cathepsin D inhibitors are the compounds of formula I and formula II.

While all combinations of variables disclosed above for compounds of formula I and formula II provide compounds having the ability to inhibit aspartyl proteases, such as cathepsin D, certain compounds are preferred for such use.

Regarding the compounds of formula I, preferred compounds are those compounds wherein:

i is 1;

$R^1$ is $-CH(CH_3)_2$, or phenyl;

W is

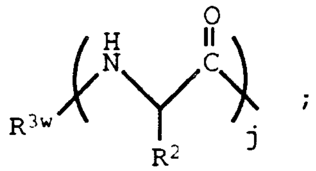

where

$R^{3w}$ is hydrogen or -B-X-$R^0$, where

B is -C(O)-, or -$S(O)_2$-;

X is -$(CH_2)_g$-, or -$(CH_2)_m$-O-$(CH_2)_n$-, where

g is 0;

m and n are independently 0, 1, or 2;

$R^0$ is $C_1$-$C_6$ alkyl, aryl, or unsaturated heterocycle;

$R^2$ is an amino acid side chain, -$CH_2CH_3$, -$CH_2CH_2CH_3$, -$CH_2(CH_2)_2CH_3$, -$CH_2CN$, or -$CH_2$-$R^{2a}$, where

$R^{2a}$ is aryl

R is

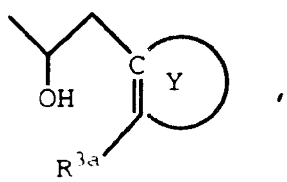

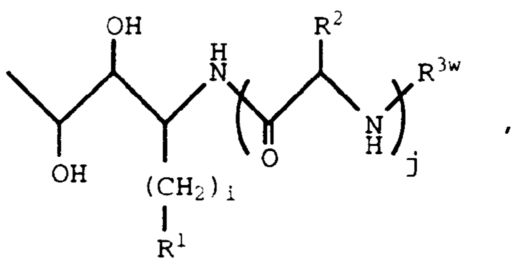

or

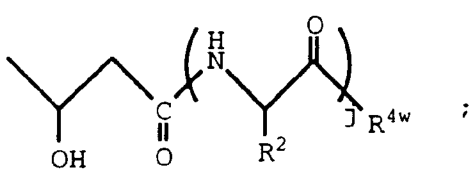

where:

$R^{4w}$ is amino;

Y is phenyl; and

$R^{3a}$ is -C(O)NH(*t*-butyl);

with the proviso that when R is

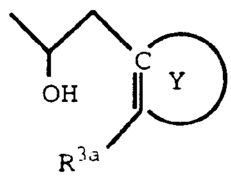 ;

then j is 2;
or a pharmaceutically acceptable salt thereof.

Of these preferred compounds of formula I, especially preferred compounds are those compounds wherein:

B is -C(O)-;
m is 1;
$R^0$ is methyl, phenyl, pyridyl, or quinolinyl;
$R^2$ is $-CH(CH_3)_2$, $-CH_2CH_2CH_3$, or $-CH_2-R^{2a}$, where
$R^{2a}$ is naphthyl; and
R is

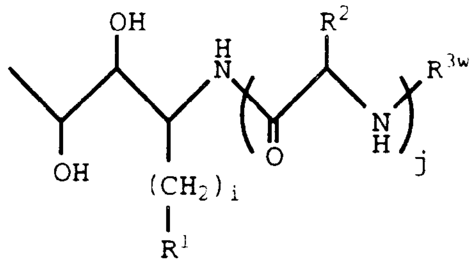 ,

or

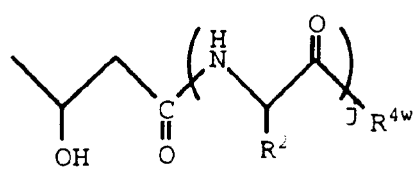 ;

where
$R^{4w}$ is amino;
or a pharmaceutically acceptable salt thereof.

The most preferred compounds of formula I are:

$CH_3C(O)$-bNA-Val-Sta-Leu-bNA-$NH_2$;
$CH_3C(O)$-NA-Nva-Sta-Nva-NA-$NH_2$;
$CH_3C(O)$-NA-Nva-AHPPA-Nva-NA-$NH_2$;
$CH_3C(O)$-NA-Val-Sta-Abu-NA-$NH_2$;

and

NA-Val-AHPPA-Leu-NA-$NH_2$;

or a pharmaceutically acceptable salt thereof.

As noted above, preferred cathepsin D inhibitors include those compounds which mimic the carboxy-terminus of βAP with inclusion of at least one statine or statine derivative, preferably at a location analogous to amino acids 41 or 42 of the carboxy-terminus of βAP. Thus, preferred compounds of formula II are those compounds wherein f is 3 or 4;
or a pharmaceutically acceptable salt thereof.

Of these preferred compounds of formula II, more preferred are those compounds of the formula

$R^7$-Gly-$(R^8)_f$-$(R^8)'$-$(R^8)''$-Thr-OH

wherein:
f is 1 or 2;

each $R^8$ is independently an amino acid residue;
$(R^8)'$ is Ile, statine or a statine derivative;
$(R^8)''$ is Ala, statine or a statine derivative;
with the proviso that at least one of $(R^8)'$ and $(R^8)''$ must be statine or a statine derivative;
or a pharmaceutically acceptable salt thereof.

Of these more preferred compounds of formula II, the most preferred compounds of formula II are:

$CH_3C(O)$-Gly-Val-Val-Sta-Ala-Thr-OH;

and

Gly-Val-Val-Sta-Ala-Thr-OH;

or a pharmaceutically acceptable salt thereof.

The peptidic compounds of formulae I and II used in the methods of the present invention may be synthesized using the classic Merrifield method of stepwise solid phase peptide synthesis. Detailed explanations of the various reactions such as the preparation of protected amino acids, chromatography, ninhydrin tests and the like can be found in Stewart and Young, 1984, Solid Phase Peptide Synthesis Second Edition (Pierce Chemical Company).

Synthesis of the peptide is started by coupling the first protected-amino acid to a resin, such as a *p*-methylbenzhydrylamine resin. The addition of each *t*-Boc-amino acid is accomplished by adding from about 1 to about 4 equivalents, preferably 2.5 equivalents of the *t*-Boc-amino acid to the resin. Typically, only 1 to about 1.5 equivalents, preferably 1.25 equivalents were used in the addition of *t*-Boc-Sta or *t*-Boc-AHPPA. *t*-Boc-Sta, *t*-Boc-AHPPA and other statine derivatives can be produced substantially in accordance with the teaching of Hui et al., 1987, J. Med. Chem. 30:1287-1295 and Boger et al., 1985, J. Med. Chem. 28:1779. This procedure is detailed in Examples 36-48, below.

A ninhydrin test may be used to detect the presence of free amino groups. The ninhydrin test is accomplished by adding one drop of a 70% phenol solution in an alcohol, such as ethanol (EtOH) to a sample of the peptide/resin mixture followed by the addition of one drop of a pyridine solution (containing 0.0002M potassium cyanide (KCN)), followed by one drop of a ninhydrin solution (0.28M in EtOH). The resultant test mixture was then heated to 110°C for two minutes. A blue or amber color indicates the presence of free amino groups while a light yellow color indicates the absence of free amino groups.

Alternatively, the ninhydrin test may be performed upon a small aliquot of the peptide/resin mixture by first washing the mixture twice with a solution of 5% triethylamine ($Et_3N$) in methylene chloride ($CH_2Cl_2$), then three times with $CH_2Cl_2$, and then concentrating the mixture under reduced pressure to provide solid dried resin. In a test tube, 2-5 mg of this solid is combined with 0.1 mL of KCN (treated with Amberlite MB-3 to remove ammonia), 0.04 mL of ninhydrin and 0.05 mL of phenol (also treated with MB-3). The resultant solution is heated to 100°C for approximately ten minutes, then chilled in cold water ($H_2O$) and combined with 1 mL of 60% EtOH. The resultant solution is mixed and filtered through a glass wool plug. The tube is rinsed twice with 0.2 mL of 0.5M tetraethylammonium chloride in $CH_2Cl_2$ and then the combined filtrates and washes are diluted to 2.0 mL by the addition of a 60% EtOH solution. The absorbance is read at 570nm against a reagent blank. A light color indicates a high percentage of coupling. The ninhydrin test is adapted from Steward and Young, *supra*, and Kaiser et al., 1970, Anal. Biochem., 34:595.

The compounds of formula I, or their precursors, may be prepared using procedures known in the art. Unless specified in a particular reaction, solvent choice is not critical so long as the solvent employed is inert to the ongoing reaction and the reactants are sufficiently solubilized to effect the desired reaction. More particularly, the compounds of formula I

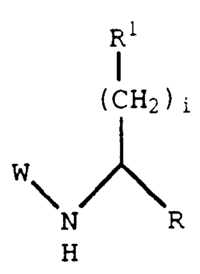

(I)

wherein:

W is

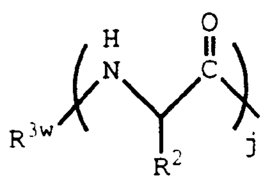

R is

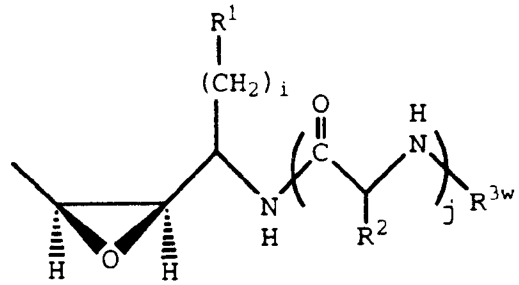

and

$R^1$, $R^{3w}$, $R^2$, i and j are as defined above, may be prepared by the following Reaction I.

<u>Reaction I</u>

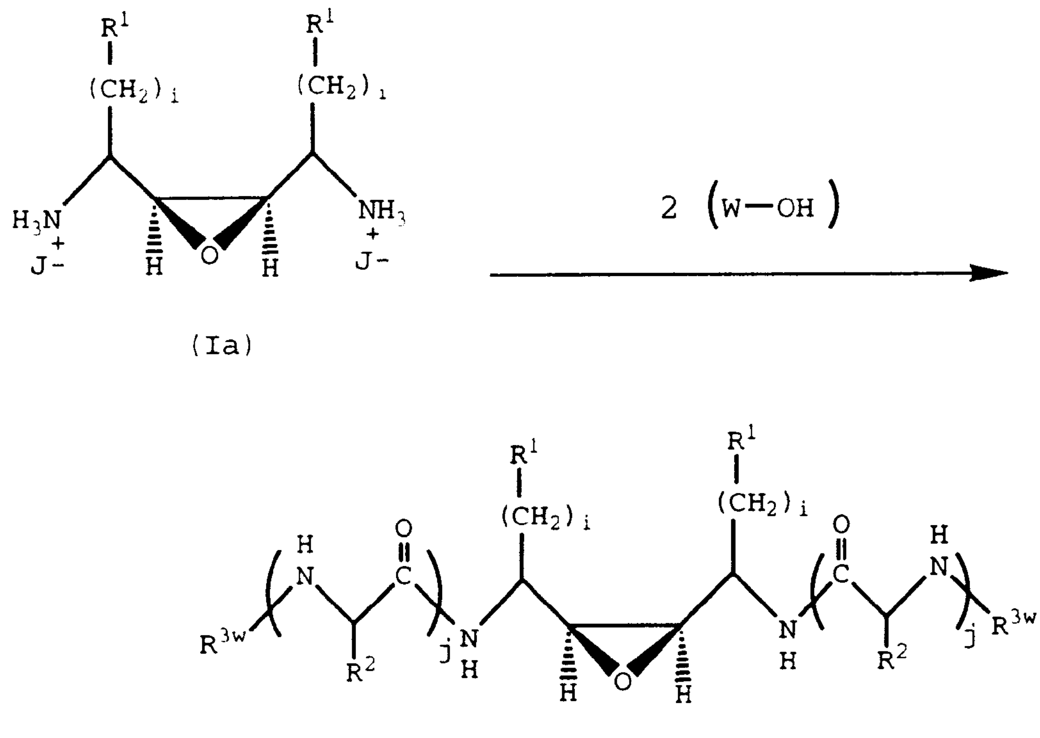

(Ia)



wherein:

J is halide, methanesulfonate, benzenesulfonate, *p*-toluenesulfonate, acetate or trifluoroacetate; and W, $R^{3w}$, $R^2$, j, i and $R^1$ are as defined above.

Reaction I, above, may be carried out by coupling a compound of formula Ia with a compound of formula W-OH, in the presence of a coupling agent and in the presence or absence of a promoting agent, preferably in the presence of a promoting agent. The carboxylic acid reactant is generally employed in an amount ranging from about equimolar proportions to about a three molar (3<u>M</u>) excess relative to the oxirane reactant, preferably in about a 2<u>M</u> excess. The coupling reagent is generally employed in an amount ranging from about equimolar

proportions to a slight excess relative to the carboxylic acid reactant. Typical solvents suitable for use in this process include dimethylformamide (DMF) or tetrahydrofuran (THF) or a DMF/THF mixture. The reaction is generally substantially complete after about 1 to 72 hours when conducted at a temperature in the range of from about -15°C to the reflux temperature of the reaction mixture. The reaction is preferably conducted at a temperature in the range of from about 0°C to about 30°C for about 24 to 48 hours. A preferred promoting agent is hydroxybenzotriazole hydrate (HOBT·$H_2O$). Typical examples of coupling reagents include the carbodiimides such as N,N'-diethylcarbodiimide, dicyclohexylcarbodiimide (DCC); the imidazoles such as carbonyldiimidazole; as well as reagents such as 1-hydroxybenzotriazole mesylate, N-ethoxycarbonyl-2-ethoxy-1,2-dihydroquinoline (EEDQ), benzotriazol-1-yloxytris(dimethylamino)phosphonium hexafluorophosphate (BOP), *o*-benzotriaza-1-yl-N,N,N',N'-tetramethyluranium hexafluorophosphate (HBTU) and benzotriazol-1-yl-oxytripyrrolidinophosphonium hexafluorophosphate (PyBOP). A preferred coupling agent is BOP. Reaction 1 is exemplified in Example 1, below.

Once the reaction is complete, the product may be isolated by procedures known in the art, for example, filtration, or the reaction solvent may be removed by extraction, evaporation or decantation. The product may be further purified, if desired, by common techniques such as crystallization or chromatography over solid supports such as silica gel or alumina.

The compounds of formula Ia used in Reaction I, above, may be prepared by the following Reaction Scheme IA:

Reaction Scheme IA:

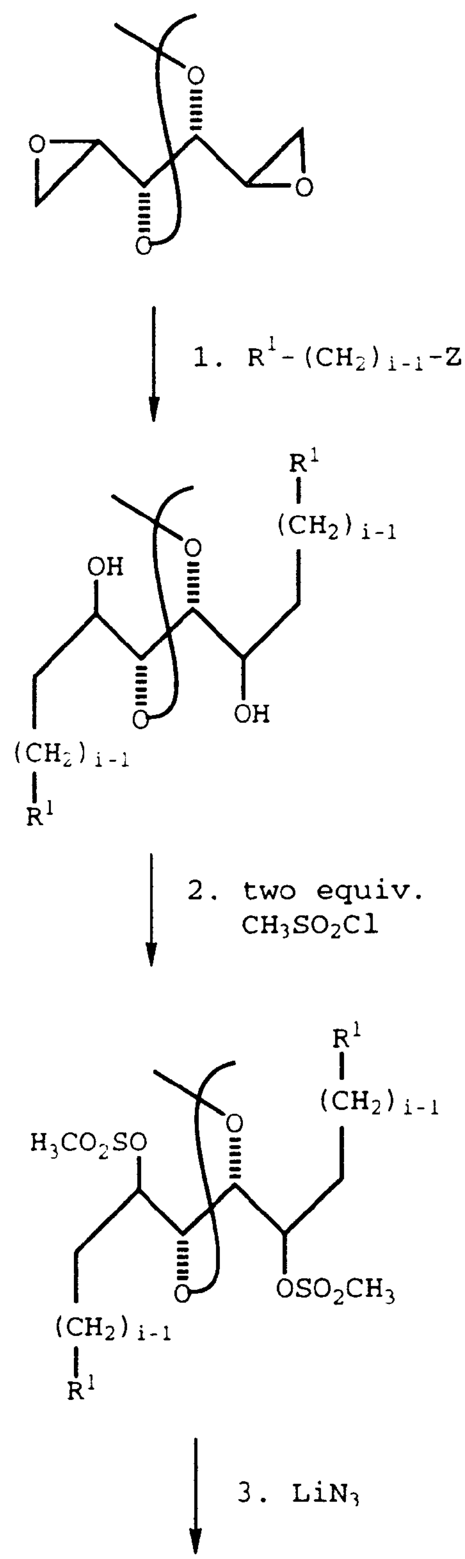

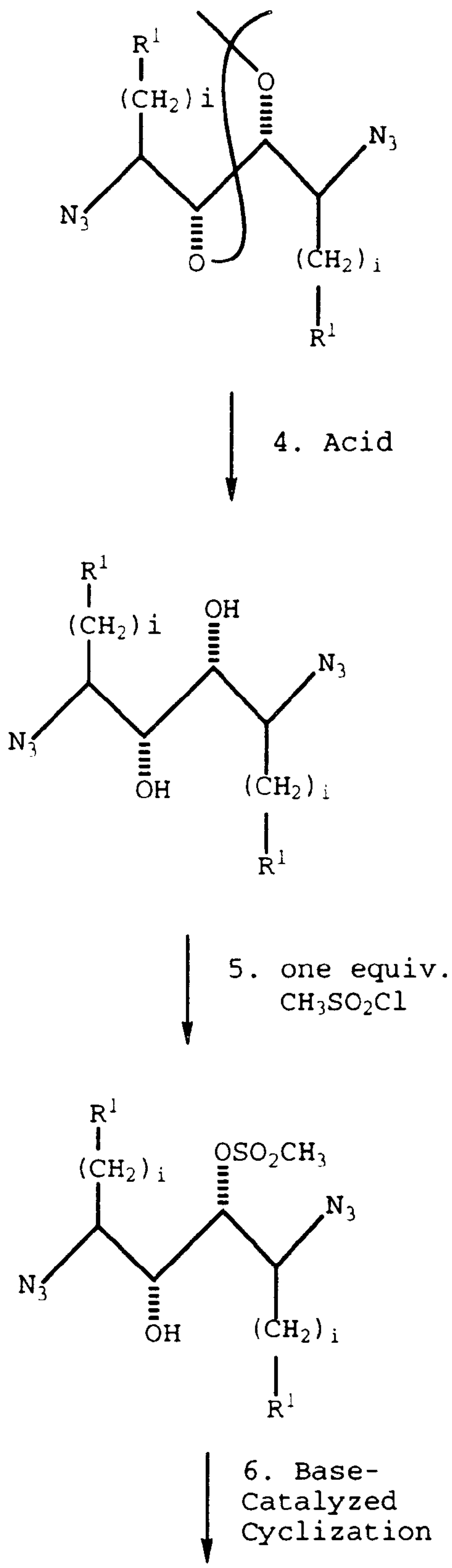
4. Acid
5. one equiv.
CH3SO2Cl
6. Base-
Catalyzed
Cyclization

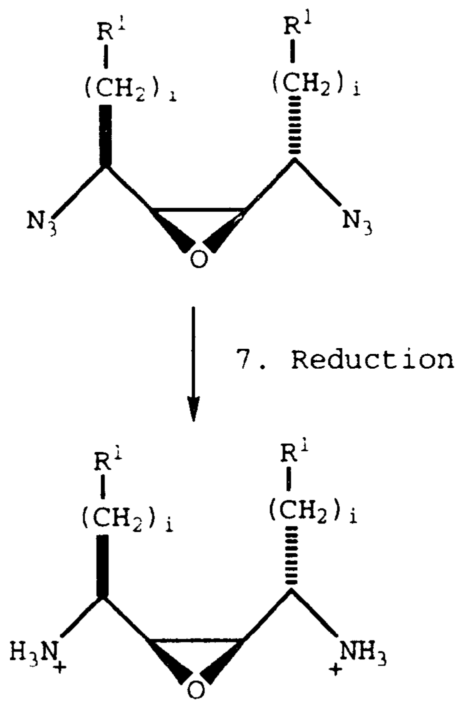


(I)

Reaction scheme IA, above, is accomplished by carrying out reactions 1-7 in sequential order. Once a reaction is complete, the intermediate compound may be isolated and/or purified using procedures known in the art as described above. Reaction scheme IA is exemplified in Preparation 1, below.

In reaction IA.1, the reaction is carried out by combining 2,2-dimethyl-4,5-dioxirane-1,3-dioxolane with a nucleophilic reagent of the formula, $R-(CH_2)_{i-1}-Z$, where Z is magnesium halide, or lithium in a mutual inert solvent. A preferred nucleophilic reagent is magnesium bromide. Better results may be obtained by catalysis with copper salts, for example copper (I) iodide (CuI) or copper (I) bromide (CuBr). The nucleophilic reagent is generally employed in an amount ranging from about equimolar proportions to about a 2M excess relative to the oxirane reactant, preferably in about a 0.5M excess. Typical solvents suitable for use in this reaction include organic solvents such as diethyl ether ($Et_2O$) or THF. The reaction is generally substantially complete after about 1 to 24 hours when conducted at a temperature in the range of from about -40°C to about 10°C. The reaction is preferably conducted at a temperature in the range of from about -5°C to about 5°C for about 2 to 6 hours.

In reaction IA.2, the reaction is carried out by combining the compound prepared in reaction IA.1, with methanesulfonyl chloride in a mutual inert solvent. The methanesulfonyl chloride is generally employed in an amount ranging from about equimolar proportions to about a 3M excess of the methanesulfonyl chloride reactant, preferably in about a 2M excess. A base, for example 2,6-lutidine or a trialkylamine such as $Et_3N$ or diisopropylethylamine and the like, may optionally, be added to promote the reaction. Preferred bases for this reaction are the trialkylamines, especially $Et_3N$. Typical solvents suitable for use in this reaction include aprotic organic solvents, such as $CH_2Cl_2$ or chloroform ($CHCl_3$). The reaction is generally substantially complete after about 15 minutes to 24 hours when conducted at a temperature in the range of from about 0°C to the reflux temperature of the reaction mixture. The reaction is preferably conducted at a temperature in the range of from about 15°C to about 30°C for about 15 minutes to 4 hours.

In reaction IA.3, the reaction is carried out by combining the compound, prepared in reaction IA.2, with azide ion in a mutual inert solvent. The azide ion may be obtained for use in this reaction from inorganic salts, such as the alkali metal azides, for example lithium azide or from organic salts such as tetramethylguanidinium azide. The azide ion is generally employed in an amount ranging from about equimolar proportions to about a 3M excess of the azide ion, preferably in about a 2M excess. An improved yield may be obtained by the addition of a base, such as 2,6-lutidine. Typical solvents suitable for use in this process include any organic solvent such as hexamethylphosphoric triamide (HMPA), DMF or N,N'-dimethylpropyleneurea (DMPU). The reaction is generally substantially complete after about 1 to 24 hours when conducted at a temperature in the range of from about 0°C to the reflux temperature of the reaction mixture. The reaction is preferably conducted

at a temperature in the range of from about 25°C to about 100°C for about 4 to 12 hours.

In reaction IA.4, the reaction is carried out by combining the compound prepared in reaction IA.3, with a strong acid in an alcoholic solvent, such as methanol (MeOH) or ethanol (EtOH). Typical acids suitable for use in this reaction include hydrohalic acids such as hydrochloric acid (HCl) or hydrobromic acid (HBr), sulfuric acid ($H_2SO_4$) and the like. Preferred acids are the hydrohalic acids, especially HCl. The acid is generally employed in a large excess, for example in an amount ranging from about a 10M excess to about a 20M excess of the acid reactant. The reaction is generally substantially complete after about 1 to 24 hours when conducted at a temperature in the range of from about 0°C to about 40°C. The reaction is preferably conducted at a temperature in the range of from about 20°C to about 30°C for about 2 to 4 hours.

In reaction IA.5, the reaction is carried out in a substantially similar manner as that described in reaction IA.2, using methanesulfonyl chloride and the compound prepared in reaction IA.4, with the exception that the compound prepared in reaction IA.4 is generally employed in an amount ranging from about equimolar proportions to about a 1M excess relative to the methanesulfonyl chloride reactant.

In reaction IA.6, the formation of the oxirane ring is carried out by base-catalyzed cyclization, preferably by combining the compound prepared in reaction IA.5 with alkoxide ion, in an appropriate solvent. The alkoxide ion is obtained from ammonium or alkali metal alkoxides and the like. Preferred alkoxide ions for this reaction are sodium methoxide (NaOMe) and potassium methoxide (KOMe). Typical solvents suitable for use in this process include any organic solvent such as MeOH or THF. The reaction is generally substantially complete after about 1 to 12 hours when conducted at a temperature in the range of from about 0°C to about 40°C. The reaction is preferably conducted at a temperature in the range of from about 20°C to about 30°C for about 1 to 2 hours.

In reaction IA.7, the reduction of the azido substituent to the corresponding amine is preferably carried out by catalytic hydrogenation, preferably by combining the compound prepared in reaction IA.6 with hydrogen gas ($H_2$) in the presence of acetic acid (HOAc) and a palladium-on-carbon catalyst (Pd/C). Typical solvents suitable for use in this reaction include any organic solvent such as ethyl acetate (EtOAc). The reaction is generally substantially complete after about 1 to 24 hours when conducted at a temperature in the range of from about 0°C to about 40°C. The reaction is preferably conducted at a temperature in the range of from about 20°C to about 30°C for about 2 to 5 hours.

Alternatively, the reduction of the azido substituent to the corresponding amine may be carried out by combining the compound prepared in reaction IA.6 with trialkyl- or triarylphosphine, in the presence of aqueous HOAc. The preferred phosphine reactant is tributylphosphine. Typical organic solvents suitable for use in this reaction include mixtures such as EtOAc/acetonitrile ($CH_3CN$) containing 10% $H_2O$. The reaction is generally substantially complete after about 1 to 12 hours when conducted at a temperature in the range of from about 0°C to about 40°C. The reaction is preferably conducted at a temperature in the range of from about 20°C to 30°C for about 0.5 to 2 hours.

The compounds of formula I wherein:

W is

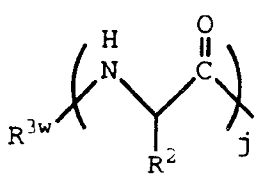

;

R is

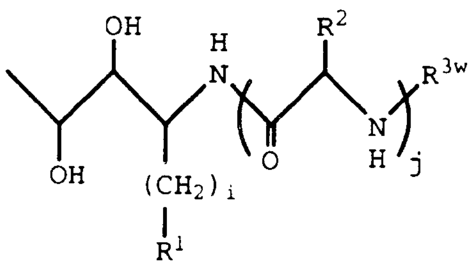

,

and

$R^1$, $R^{3w}$, $R^2$, i and j are as defined above, may be prepared by the following Reaction II.

Reaction II

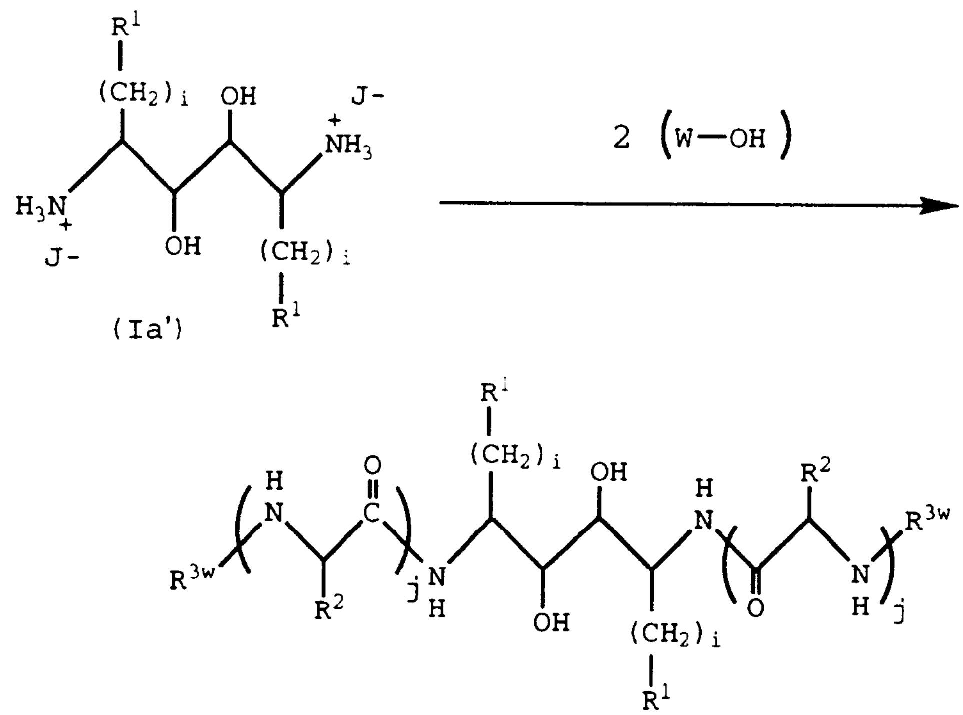

wherein:

J is halide, methanesulfonate, benzenesulfonate, *p*-toluenesulfonate, acetate or trifluoroacetate; and W, $R^{3w}$, $R^2$, j, i and $R^1$ are as defined above.

Reaction II, above, may be carried out by coupling a compound of formula Ia' with a compound of formula W-OH, in the presence of a coupling agent and in the presence or absence of a promoting agent substantially as detailed above in Reaction I. Reaction II is exemplified in Examples 33-35, below.

Alternatively, these diols may be prepared by exposing the epoxide compounds prepared in Reaction I above to aqueous acidic conditions. Also, these diols may be isolated as byproducts of the epoxide reactions.

The compounds of the formula Ia'

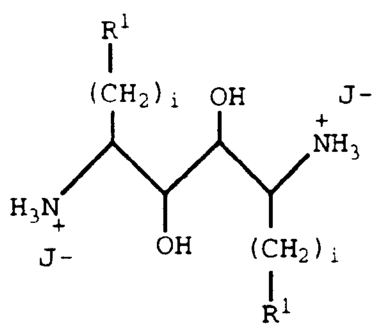

(Ia')

used in reaction II, above, may be prepared by reducing the azido intermediate prepared in Reaction IA.4, above substantially as detailed in Reaction IA.7. This procedure is exemplified in Preparation 12, below.

The compounds of the formula W-OH, where

W is

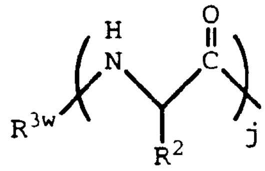

and j is 2 may be prepared by first coupling two amino acids of the general formula, $H_2N-CH(R^2)-COOH$, where each $R^2$ is independently one of the $R^2$ substituents defined above in formula I substantially in accordance with the procedure detailed above in Reaction I.

The amino-protecting group is then removed using procedures known in the art and the resulting dipeptide compound is either acylated or coupled with a compound to provide a compound having the formula:

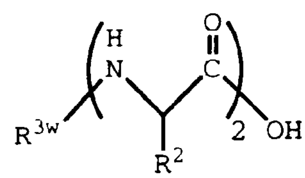

This second coupling reaction may be carried out substantially in accordance with the procedure detailed in Reaction I. The acylation may be carried out with a suitable acyl halide, isocyanate or chloroformate, preferably in the presence of an acid scavenger such as a tertiary amine, preferably $Et_3N$. The acylation reaction is carried out at a temperature of from about -20°C to about 25°C. Typical solvents for this reaction include ethers and chlorinated hydrocarbons, preferably $Et_2O$, $CHCl_3$ or $CH_2Cl_2$.

The compounds of the formula W-OH may also be prepared by reacting a suitably substituted carboxy-protected amino acid with triphosgene and then reacting the resulting compound with a compound of the formula, $R^{3w}$-H, where $R^{3w}$ is as defined above. This procedure is exemplified in Preparation 2, below.

First, the carboxy-protected amino acid reactant is combined with triphosgene in a mutual inert solvent. The carboxy-protected amino acid reactant is generally employed in an amount ranging from about equimolar proportions to about a 2M excess, preferably in about a 1M excess relative to the triphosgene. A base, for example a trialkylamine such as $Et_3N$ or diisopropylethylamine, is added to promote the reaction. Typical solvents suitable for use in this reaction include any organic solvent such as toluene. The reaction is generally substantially complete after about 6 to 24 hours when conducted at a temperature in the range of from about 25°C to the reflux temperature of the reaction mixture. The reaction is preferably conducted at a temperature in the range of from about 80°C to the reflux temperature of the reaction mixture for about 8 hours to 12 hours.

Next, the resultant compound is combined with a compound of the formula, $R^{3w}$-H, in a mutual inert solvent. Better results may be obtained by catalysis with copper salts, for example CuI or CuCl. The resultant compound is generally employed in an amount ranging from about equimolar proportions to about a 1M excess relative to the compound of the formula $R^{3w}$-H. Typical solvents suitable for use in this reaction include any organic solvent such as DMF or $CH_3CN$. The reaction is generally substantially complete after about 15 minutes to 3 hours when conducted at a temperature in the range of from about 10°C to about 40°C. The reaction is preferably conducted at a temperature of from about 15°C to about 30°C for about 15 minutes to 2 hours.

The carboxy group on the resultant compound may be deprotected using procedures known in the art, for example by hydrolysis of the ester to the corresponding carboxylic acid of the formula W-OH. The hydrolysis of the ester may be accomplished by simply combining the resultant compound with a strong acid in a mutual inert solvent. Typical solvents suitable for use in this reaction include any organic solvent such as dioxane. The reaction is generally substantially complete after about 15 minutes to 4 hours when conducted at a temperature in the range of from about 25°C to the reflux temperature of the reaction mixture. The reaction is preferably conducted at a temperature in the range of from about 60°C to about 100°C for about 15 minutes to 1 hour.

Alternatively the carboxy-protecting group may be removed using catalytic hydrogenation, preferably by combining the resultant compound with ammonium formate and Pd/C catalyst in an alcoholic solvent, such as MeOH or EtOH. The reaction is generally substantially complete after about 1 to 4 hours when conducted at a temperature in the range of from about 0°C to the reflux temperature of the reaction mixture. The reaction is preferably conducted at reflux temperature for about 2 to 5 hours.

Finally, the compounds of the formula W-OH may be prepared by reacting a suitably substituted carboxy-protected amino acid with 1,1'-carbonyldiimidazole and then reacting the resultant compound with a compound

of the formula, $R^{3w}$-H, substantially in accordance with the procedure detailed above.

Specifically, the carboxy-protected amino acid reactant is first reacted with 1,1'-carbonyldiimidazole in a mutual inert solvent. The 1,1'-carbonyldiimidazole reactant is generally employed in an amount ranging from about equimolar proportions to about a 2<u>M</u> excess relative to the carboxy-protected amino acid reactant, preferably in about equimolar proportions. Typical solvents suitable for use in this reaction include any standard organic solvent such as $CH_3CN$. The reaction is generally substantially complete after about 15 minutes to 2 hours when conducted at a temperature in the range of from about 15°C to 30°C. The reaction is preferably conducted at a temperature in the range of from about 20°C to about 25°C for about 15 minutes to 1 hour.

Next, the resultant compound is reacted with a compound of the formula, $R^{3w}$-H, in a mutual inert solvent substantially in accordance with the procedure detailed above. The carboxy group may be deprotected using the methods described above.

The compounds of formula I where R is

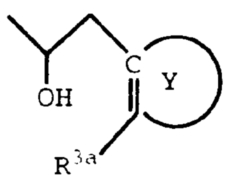 , 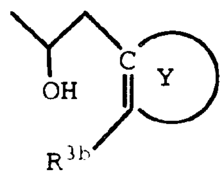 or 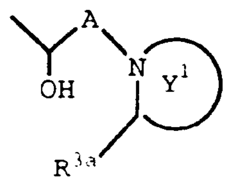 ;

and

W, $R^1$ and i are as defined above, may be prepared by the following Reaction III.

<u>Reaction III</u>

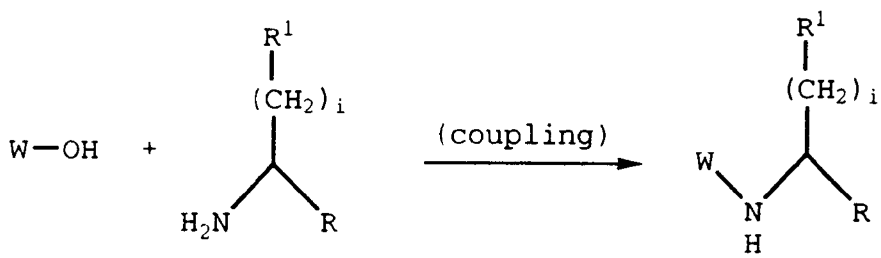


(Ib) (I)

where R, W, $R^1$, i, $R^{3a}$, $R^{3b}$, A, Y and $Y^1$ are as defined above.

Reaction III, above, may be carried out by coupling a compound of the formula, W-OH, with a compound of formula Ib. This coupling reaction is accomplished substantially in accordance with the procedure detailed in Reaction I, above. A preferred promoting agent is $HOBT \cdot H_2O$. A preferred coupling reagent is DCC. Reaction III is exemplified in Example 6.

The compounds of formula I where j is 2 may be prepared by first coupling a compound of the formula Ib with an amino-protected amino acid of the formula, $R^b$-NH-CH($R^2$)COOH, where $R^b$ is an amino-protecting group; and $R^2$ is as defined above. This reaction is accomplished substantially in accordance with the coupling reaction of Reaction I, above.

The resultant compound is deprotected and then reacted with a compound of the formula, $R^{3w}$-H, in a mutual inert solvent. Better results may be obtained by catalysis with copper salts, for example CuI or CuCl. The resultant compound is generally employed in an amount ranging from about equimolar proportions to about a 1<u>M</u> excess relative to the compound of the formula $R^{3w}$-H. Typical solvents suitable for use in this reaction include any organic solvent such as DMF or $CH_3CN$. The reaction is generally substantially complete after about 15 minutes to 3 hours when conducted at a temperature in the range of from about 10°C to about 40°C. The reaction is preferably conducted at a temperature in the range of from about 15°C to about 30°C for about 15 minutes to 2 hours.

Alternatively the resultant compound may be deprotected and then acylated with a suitable acyl halide, isocyanate or chloroformate, sulfonylated with a suitably substituted sulfonyl halide or coupled with a suitably substituted carboxylic acid reactant to provide a compound of formula I.

The compounds of the formula Ib where R is

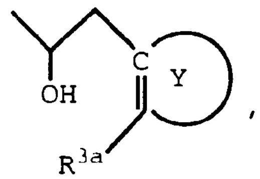

may be prepared according to the procedures shown below in Reaction Scheme IIIA.

Reaction Scheme IIIA

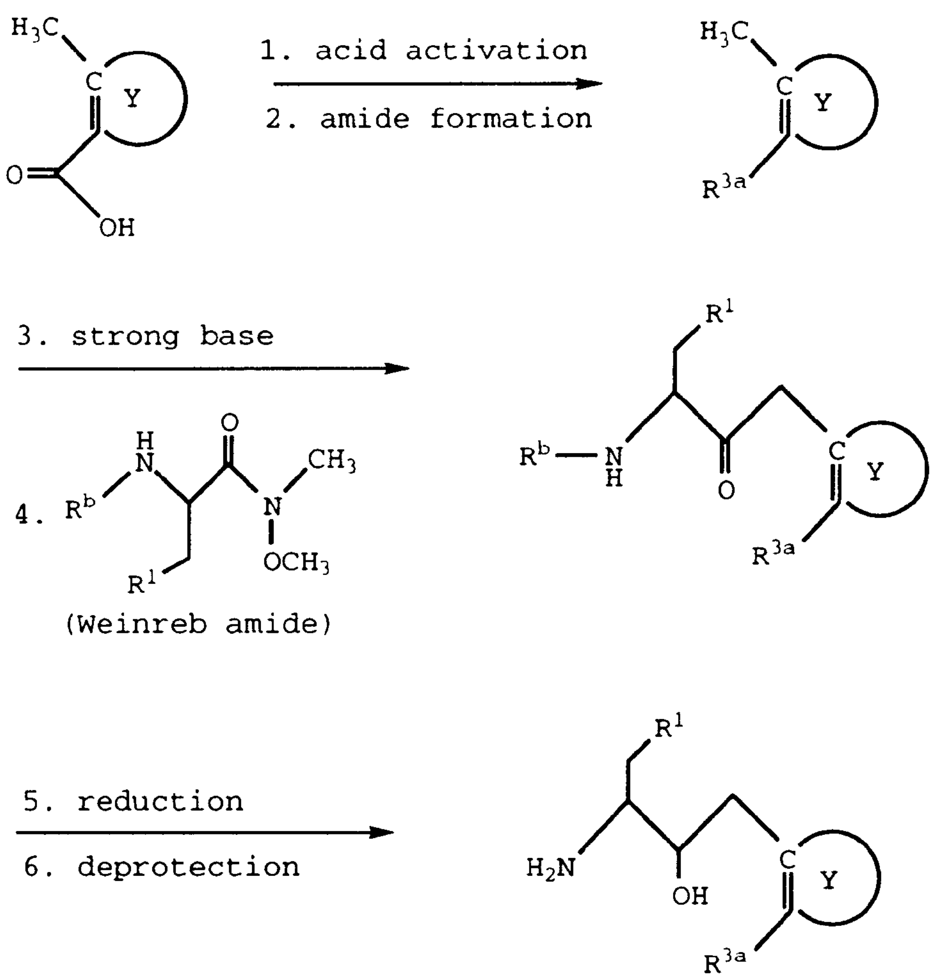


where:

$R^b$ is an amino-protecting group; and

$R^1$, $R^{3a}$ and Y are as defined above.

Reaction Scheme IIIA, is accomplished by carrying out reactions 1-6 in sequential order. Once a reaction is complete, the intermediate compound may be isolated and/or purified, if desired by procedures known in the art as described above.

In Reaction IIIA.1, the reaction is typically carried out by activating, that is, converting, a suitably substituted aryl or unsaturated heterocycle carboxylic acid to the corresponding acyl chloride or acyl bromide by reaction with thionyl chloride, thionyl bromide, phosphorous trichloride, phosphorous tribromide, phosphorous pentabromide or phosphorous pentachloride according to procedures and under conditions known in the art. Suitable aryl, heterocycle or unsaturated heterocycle carboxylic acid compounds are commercially available or prepared by standard procedures known in the art.

In Reaction IIIA.2, the acyl chloride or acyl bromide, prepared in Reaction IIIA.1, is reacted with ammonia or a primary or secondary amine having the formula

$H\text{-}NR^4R^4$,

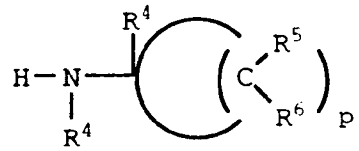

,

or

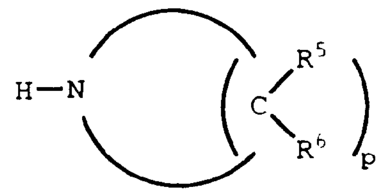

;

where $R^4$, $R^5$, $R^6$ and p are as defined above, in a nonpolar aprotic solvent or mixture of solvents in the presence or absence of an acid scavenger to afford the corresponding amide. The reaction is typically carried out at a temperature of from about -20°C to about 25°C. Typical solvents for this reaction include ethers and chlorinated hydrocarbons, preferably $Et_2O$, $CHCl_3$ or $CH_2Cl_2$. Preferably, this reaction is carried out in the presence of an acid scavenger such as a tertiary amine, preferably $Et_3N$.

In Reaction IIIA.3, the amide prepared in Reaction IIIA.2, is reacted with a strong base in the presence of a solubilizing agent to afford the corresponding anion which is then reacted in Reaction IIIA.4 with a Weinreb amide to afford a ketone. Reaction IIIA.3 is carried out in an aprotic solvent at a temperature of from about -78°C to about 0°C. Typical bases used in Reaction IIIA.3 include lithium amide bases and alkyl lithium bases, preferably $C_1$-$C_4$ alkyl lithium bases and lithium di($C_1$-$C_4$)alkylamide bases. Typical solubilizing agents for Reaction 3 are tetramethyl($C_1$-$C_4$)alkylenediamines, preferably tetramethylethylenediamine. Reaction IIIA.4 is carried out in an aprotic solvent at a temperature from about -80°C to about -40°C. Typical solvents for Reactions IIIA.3 and IIIA.4 include ethers, preferably THF. In Reaction IIIA.4, the anion is generally employed in an amount ranging from about equimolar proportions to about a 3M excess of the anion, preferably in about a 2M excess of the anion relative to the Weinreb amide reactant.

In Reaction IIIA.5, the ketone prepared in Reaction IIIA.3, is reduced to the corresponding alcohol using a suitable reducing agent. The reaction is carried out in a protic solvent at a temperature of from about -25°C to about 25°C. Typical reducing agents for this reaction include sodium borohydride, lithium borohydride, diisobutylaluminum hydride, and sodium bis(2-methoxyethoxy)aluminum hydride. A preferred reducing agent is sodium borohydride. Typical protic solvents for this reaction include alcohols, preferably EtOH. Reaction IIIA.6 is a standard amino deprotection reaction using procedures and methods known in the art to afford the corresponding amine of formula Ib, which is then used in Reaction III, above. The amine may be reacted without purification, but it is preferably purified first.

The compounds of the formula Ib where R is

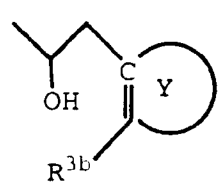

,

may be prepared according to the procedures shown below in Reaction Scheme IIIB.

Reaction Scheme IIIB

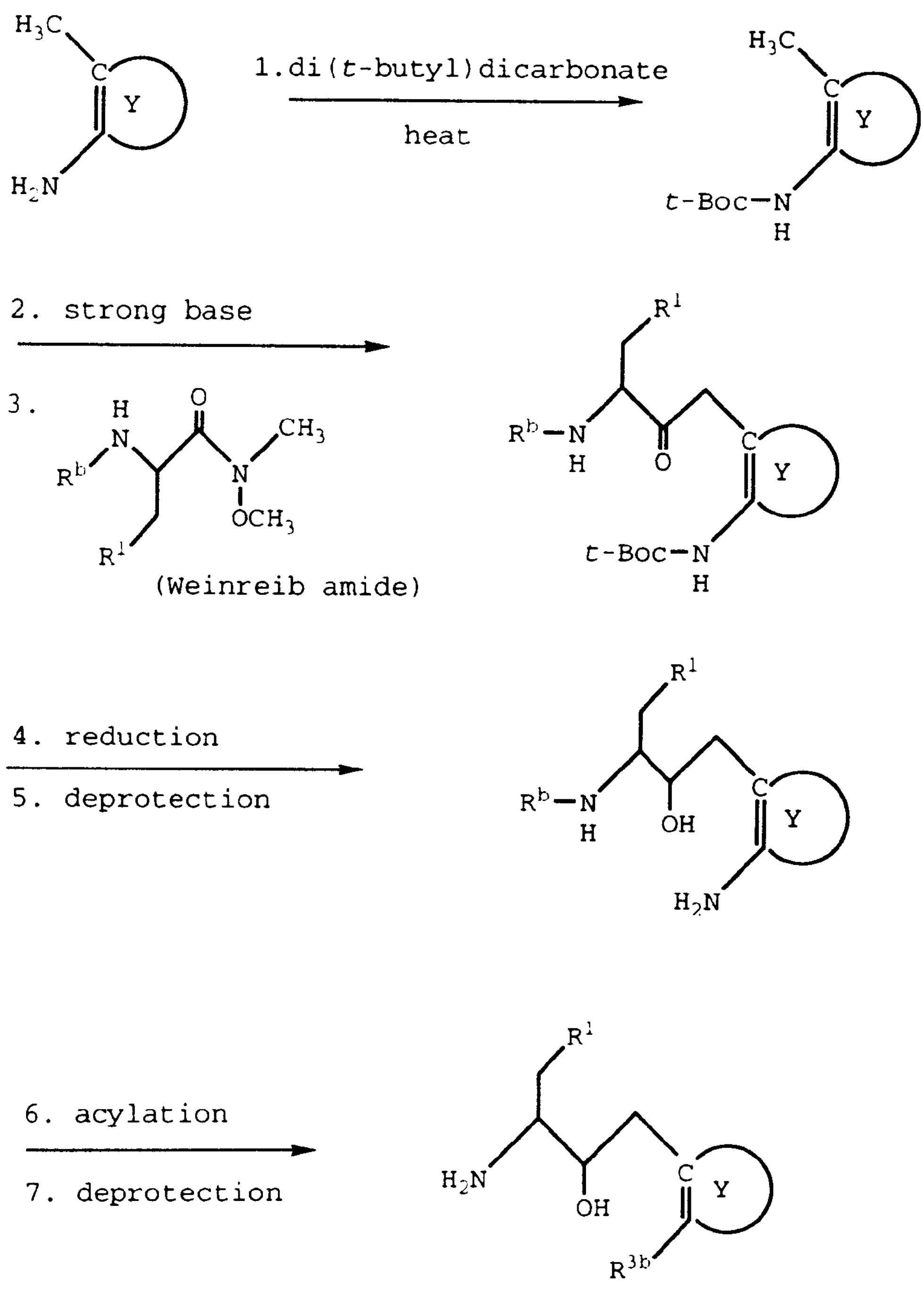


where $R^b$, $R^1$, Y and $R^{3b}$ are as defined above.

Reaction Scheme IIIB, above, is accomplished by carrying out reactions 1-7 in sequential order. Once a reaction is complete, the intermediate compound may be isolated and/or purified by procedures known in the art as described above.

In Reaction IIIB.1, a suitably substituted aryl or unsaturated heterocycle amine is protected, under standard conditions used with amino-protecting groups known in the art. Reactions IIIB.2-5 are carried out substantially as described above in Reaction Scheme IIIA.3-6, with the exception that in Reaction Scheme IIIB, an additional deprotection reaction, Reaction IIIB.5, is necessary to remove the amino-protecting group introduced in Reaction IIIB.1. This is a standard amino deprotection reaction using procedures known in the art. For example, the *t*-Boc group illustrated in Reaction Scheme IIIB.1 may be removed using a strong acid, preferably $CF_3CO_2H$.

In Reaction IIIB.6, the illustrated intermediate is acylated with a suitable acyl halide, isocyanate or chloroformate, preferably in the presence of an acid scavenger such as a tertiary amine, preferably $Et_3N$. The reaction is carried out at a temperature of from about -20°C to about 25°C. Typical solvents for this reaction include ethers and chlorinated hydrocarbons, preferably $Et_2O$, $CHCl_3$ or $CH_2Cl_2$.

Reaction IIIB.7 is a standard amino deprotection reaction using procedures known in the art to afford the corresponding amine of formula Ib which is then used in Reaction III, above. This amine may be reacted without purification, but it is preferably purified first.

The compounds of formula Ib where R is

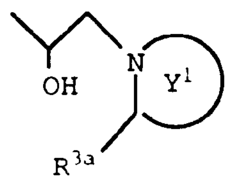
,

may be prepared according to the procedures shown below in Reaction Scheme IIIC.

Reaction Scheme IIIC

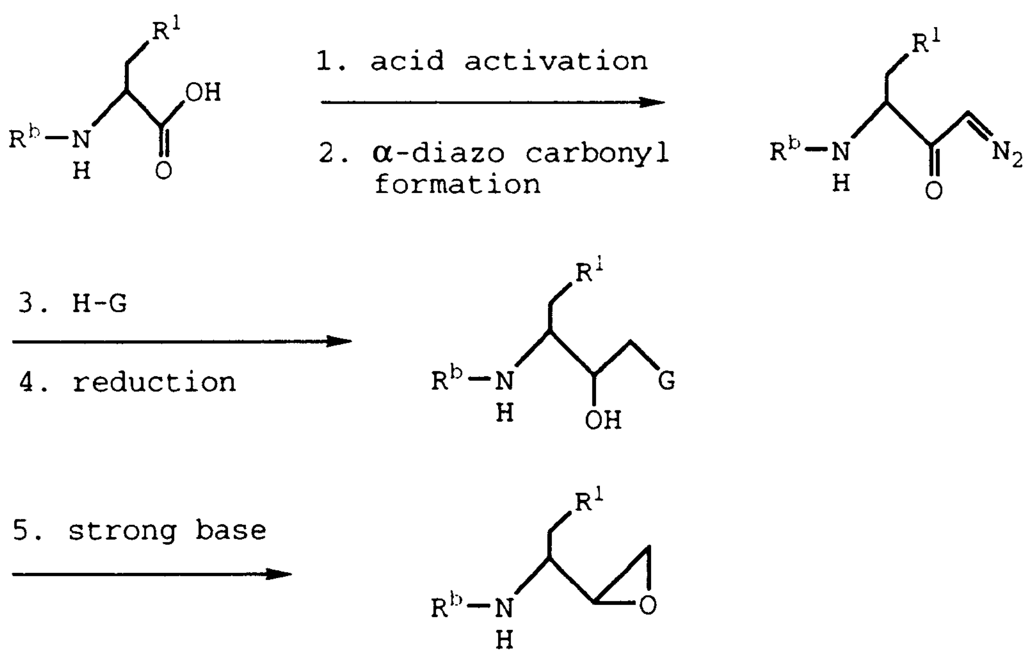

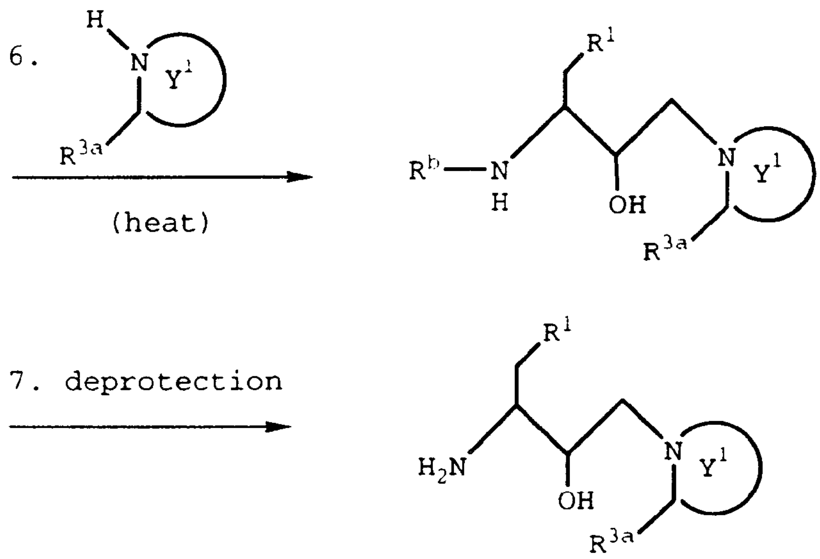


where $R^1$, $R^{3a}$, $R^b$ and $Y^1$ are as defined above; and G is halo.

Reaction Scheme IIIC, above, is accomplished by carrying out reactions 1-7 in sequential order. Once a reaction is complete, the intermediate compound may be isolated and/or purified, if desired, by procedures known in the art as described above for Reaction I. This reaction scheme is exemplified in Preparation 9, below.

Reaction IIIC.1 is carried out by activating, that is, converting, an amino-protected carboxylic acid reactant having the structure:

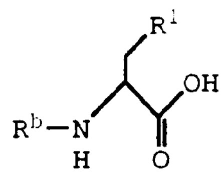

to the corresponding mixed anhydride under conditions known in the art. For example, the amino-protected carboxylic acid reactant may be reacted with a $C_1$-$C_6$ alkylchloroformate, such as isobutylchloroformate preferably in the presence of an acid scavenger. Preferred acid scavengers are the trialkylamines, preferably $Et_3N$. The reaction is typically carried out in an aprotic solvent such as EtOAc. The resulting mixed anhydride reactant is preferably used in Reaction IIIC.2 without further isolation or purification.

Reaction IIIC.2 is accomplished in two steps. First, a solution of sodium hydroxide (NaOH), covered with a layer of an ether solvent, preferably $Et_2O$, is reacted with a large excess of N-methyl-N-nitro-N-nitrosoguanidine to form a diazomethane reactant. The NaOH is preferably used as an aqueous solution of about 4-6$\underline{M}$ NaOH. Once this reaction is substantially complete, the organic layer is dried over a dessicant such as KOH. This solution is then reacted with the mixed anhydride from Reaction IIIC.1, above, to form the corresponding α-diazo carbonyl compound. The diazomethane reactant is preferably used in this reaction without isolation or purification. The reaction is typically carried out at a temperature of from about -50°C to about -20°C, preferably about -30°C.

In Reaction IIIC.3, the α-diazo carbonyl compound prepared in Reaction IIIC.2 is reacted with an acid of the formula H-G where G is halo, in an aprotic solvent such as $Et_2O$ to form an α-halo carbonyl compound. A preferred acid reactant is HCl which provides the corresponding α-chloro carbonyl compound. The reaction is typically carried out at a temperature from about -30°C to about 0°C. The acid reactant is typically added in the form of an anhydrous gas in small increments until the reaction is substantially complete. The reaction can be monitored by thin layer chromatography (TLC).

In Reaction IIIC.4, the carbonyl moiety on the compound prepared in Reaction IIIC.3 is reduced using standard conditions known in the art to form the corresponding α-chloro hydroxy compound. For example, the compound prepared in Reaction IIIC.3 may be combined with a reducing agent in a mixture of solvents. Typical reducing agents include sodium borohydride, lithium borohydride, zinc borohydride, diisobutylaluminum hydride and sodium bis(2-methoxyethoxy)aluminum hydride. A preferred reducing agent is sodium borohydride. Typical solvent mixtures include a protic and aprotic mixture such as $THF/H_2O$. The reaction is typically carried

out at a temperature from about -10°C to about 10°C, preferably about 0°C.

In Reaction IIIC.5, the α-chloro hydroxy compound prepared in Reaction IIIC.4 is treated with a strong base to form the corresponding epoxide under standard conditions known in the art. For example, the α-chloro hydroxy compound may be reacted with a KOH/EtOH mixture in an alcoholic solvent such as EtOH. The reaction is typically carried out at a temperature from about 0°C to about the reflux temperature of the solvent. Preferably the reaction is carried out at room temperature.

In Reaction IIIC.6, the epoxide prepared in Reaction IIIC.5 is reacted with a heterocyclic reactant of the formula

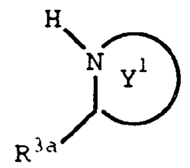

;

in a protic solvent at a temperature of from about 70°C to 100°C. Typical solvents for this reaction include the alcohols, preferably EtOH. The reaction is preferably carried out at a temperature of about 80°C.

Reaction IIIC.7 is a standard amino deprotection reaction using procedures and methods known in the art to afford the corresponding amine of formula Ib, which is then used in Reaction III, above. This amine may be reacted without purification, but it is preferably purified first.

The Weinreb amide used as a reactant in Reaction IIIA.4 and IIIB.3 is prepared by reacting an amino-protected amino acid with N-methoxy-N-methyl-amine in the presence of a promoting agent, an acid scavenger, and a coupling agent. The reaction is carried out in an aprotic solvent or mixture of solvents at a temperature of from about -25°C to 25°C. A preferred promoting agent for this reaction is $HOBT \cdot H_2O$. Preferred acid scavengers are the tertiary alkylamines, preferably $Et_3N$ or N-methylmorpholine (NMM). A preferred coupling reagent is ethyl dimethylaminopropylcarbodiimide hydrochloride. The Weinreb amide afforded by this reaction is preferably isolated prior to its use in Reactions IIIA.4 and IIIB.3.

The compounds of formula Ib, where $R^1$ is $-S-R^{1x}$, where $R^{1x}$ is aryl or cyclohexyl, are prepared using a Weinreb amide having the following structure:

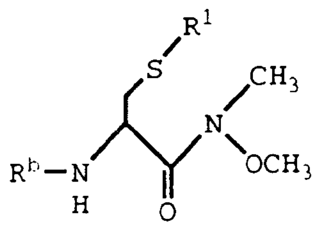

in Reactions IIIA.4 and IIIB.3. This Weinreb amide is prepared by first reacting amino-protected serine with triphenylphosphine and diethylazodicarboxylate (DEAD) in an aprotic solvent at a temperature of from about -80°C to 0°C to form the corresponding β-lactone. The reaction is typically carried out in an ether, such as THF at a temperature of from about -80°C to -50°C. Next, the lactone ring is opened to provide a compound having the structure:

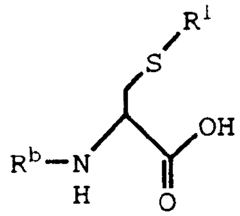

by reacting the lactone with an appropriately substituted thioanion having the structure, $-S-R^1$, where $R^1$ is as defined above for formula I. The thioanion compound is preferably formed by reacting the corresponding thiol with a strong base, such as sodium hydride or potassium hydride. This reaction is typically carried out in an

aprotic solvent at a temperature from about 0°C to about 40°C and under an inert atmosphere, such as nitrogen. Typical solvents for this reaction include ethers, preferably THF. The desired amide reactant is then formed by reacting the resulting carboxylic acid reactant with N-methoxy-N-methyl-amine in the presence of a promoting agent, an acid scavenger and a coupling agent substantially as described above.

The compounds of formula Ib where R is

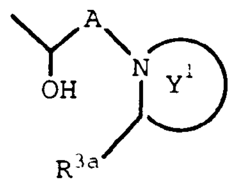 ;

A is

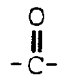 ;

and

$R^{3a}$ and $Y^1$ are as defined above;

can be prepared according to procedures known in the art. One reference that may be particularly helpful in preparing such compounds is R. Herranz et al., J. Org. Chem., 55, pp 2232-2234 (1990).

The heterocyclic reactants, used in Reaction IIIC.6 above, of the formula

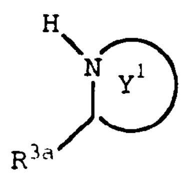 ;

can be prepared using procedures and methods known in the art. For example, the heterocyclic reactants were typically prepared from the corresponding amino-protected amino acids by acid activation followed by treatment with an alkylamine. This reaction is typically carried out in the presence of an acid scavenger, such as NMM. Removal of the amino-protecting group using standard chemical deprotecting techniques then provided the heterocyclic reactants used above in Reaction C.8. Specifically, the [3S-(*3R**, *4aR**, *8aR**)]-decahydroisoquinoline-3-N-*t*-butylcarboxamide was prepared using (2S)-1,2,3,4-tetrahydro-3-isoquinolinecarboxylic acid by the following procedure:

1) amino-protection (*t*-Boc);
2) acid activation/reaction with *t*-butylamine;
3) catalytic hydrogenation;
4) amino-deprotection.

The compounds of formula, W-OH, used in Reaction III, to the extent not commercially available, can be prepared using known procedures. More particularly, the compounds of formula, W-OH, where

W is

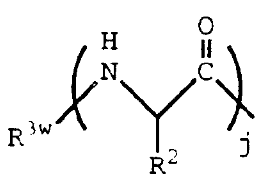 ;

may be prepared as described above in Reaction Scheme IA, above.

The compounds of the formula W-OH, where W is

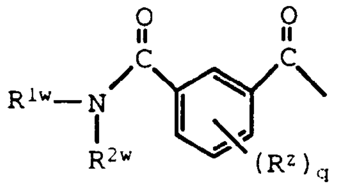
;

may be prepared according to the procedures shown below in Reaction Scheme IV.

Reaction Scheme IV

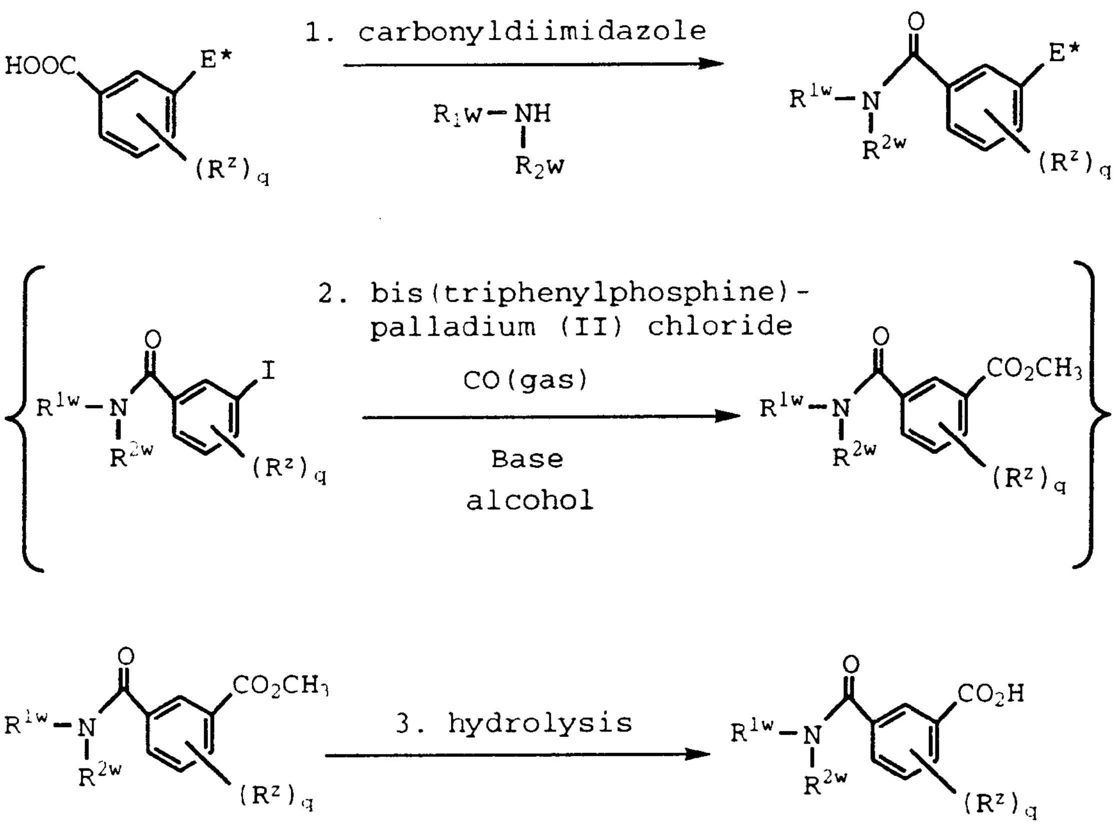


where:

q, $R^z$, $R^{1w}$ and $R^{2w}$, are as defined above; and

E* is iodo or $C_1$-$C_4$ alkoxycarbonyl.

Where E* is iodo, Reaction Scheme IV, above, is accomplished by carrying out Reactions 1-3 in sequential order. Once a reaction is complete, the intermediate compound may be isolated and/or purified by procedures known in the art as described above. Reaction IV is exemplified in Preparation 10.

Where E* is $C_1$-$C_4$ alkoxycarbonyl, the intermediate compound prepared in Reaction IV.1 is reacted according to the procedure detailed in Reaction IV.3 directly; Reaction IV.2 is not necessary.

Reaction IV.1 is a standard coupling reaction which is carried out substantially in accordance with the procedure detailed above in the Reaction I The reaction is carried out by simply combining an appropriately substituted amino moiety, $RR^0NH$, with a compound having the formula:

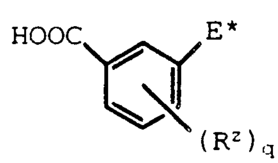

where E* is iodo or $C_1$-$C_4$ alkoxycarbonyl; and $R^z$ and q are as defined above. The reaction is carried out in a nonpolar aprotic solvent or mixture of solvents in the presence or absence of an acid scavenger to provide the corresponding amide. Typical solvents for this reaction include THF and DMF, preferably THF. The reaction is carried out at a temperature from about -30°C to about 25°C. The amine reactant is generally employed in equimolar proportions relative to the carboxylic acid reactant, in the presence of an equimolar quantity to a slight excess of the coupling reagent.

Reaction IV.2, is a carbonylation of the compound prepared in Reaction IV.1, resulting in the replacement of the iodo substituent with a $C_1$-$C_4$ alkoxycarbonyl moiety, preferably methoxycarbonyl. The reaction is carried out by simply combining the iodo intermediate from Reaction IV.2, above, a catalyst under a carbon monoxide atmosphere and in an alcoholic solvent, preferably MeOH, to provide the alkyl ester of the desired compound. This reaction is carried out in the presence of an acid scavenger, preferably dicyclohexylamine. The reaction is carried out at a temperature from about 0°C to about 25°C. A preferred catalyst is bis(triphenylphospine)palladium (II) chloride.

Reaction IV.3 is a standard hydrolysis reaction of the alkyl ester to provide the corresponding carboxylic acid reactant, W-OH, which is then used above in Reaction III. The reaction is carried out by combining the alkyl ester reactant with lithium hydroxide in a suitable solvent followed by acidification of the reaction solution to pH 2-3 to provide the desired carboxylic acid reactant of the formula W-OH, where W is

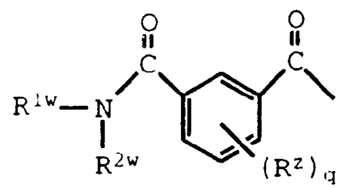

Typical solvents include a mixture of an ether and $H_2O$, preferably a THF/$H_2O$ mixture. The reaction is carried out at a temperature from about 0°C to about 35°C. The lithium hydroxide reactant is generally employed in equimolar proportions to about a 2M excess relative to the ester reactant, preferably in about a 1M excess.

It will be understood by persons in the art that in performing the processes described above it may be desirable to introduce chemical protecting groups into the reactants in order to prevent secondary reactions. Any amine, alkylamine or carboxy groups which may be present on the reactants may be protected using any standard amino- or carboxy- protecting group which does not adversely affect the remainder of the molecule's ability to react in the manner desired. Preferred amino-protecting groups are *t*-Boc and Cbz. Preferred carboxy-protecting groups are benzhydryl, benzyl and allyl. The various protective groups may then be removed simultaneously or successively using methods known in the art.

As noted above, all asymmetric forms, individual isomers and combinations thereof are considered part of this invention. Such isomers may be prepared from their respective precursors by the procedures described above, by resolving the racemic mixtures, or by separating the diastereomers. The resolution can be carried out in the presence of a resolving agent, by chromatography or by repeated crystallization or by some combination of these techniques which are known in the art. Further details regarding resolutions can be found in Jacques et al., Enantiomers, Racemates, and Resolutions, John Wiley & Sons 1981.

The compounds employed as initial starting material in the synthesis of the compounds of this invention are known and, to the extent not commercially available are readily synthesized by standard procedures commonly employed in the art.

The pharmaceutically acceptable salts of the invention are typically formed by reacting a compound of formula I with an equimolar or excess amount of acid or base. The reactants are generally combined in a mutual solvent such as $Et_2O$ or benzene, for acid addition salts, or $H_2O$ or alcohols for base addition salts. The salts normally precipitate out of solution within about one hour to about ten days and can be isolated by filtration or other conventional methods.

The following Preparations and Examples further illustrate specific aspects of the present invention. It is to be understood, however, that they are included for illustrative purposes only and are not intended to limit the scope of the invention in any respect and should not be so construed.

In the following Preparations and Examples, the terms melting point, nuclear magnetic resonance spectra, electron impact mass spectra, field desorption mass spectra, fast atom bombardment mass spectra, infrared spectra, ultraviolet spectra, elemental analysis, high performance liquid chromatography, and thin layer chromatography are abbreviated "m.p.", "NMR", "EIMS", "MS (FD)", "MS (FAB)", "IR", "UV", "Analysis", "HPLC", and "TLC", respectively. In addition, the absorption maxima listed for the IR spectra are only those of interest

and not all of the maxima observed.

In conjunction with the NMR spectra, the following abbreviations are used: "s" is singlet, "d" is doublet, "dd" is doublet of doublets, "t" is triplet, "q" is quartet, "m" is multiplet, "dm" is a doublet of multiplets and "br.s", "br.d", "br.t", and "br.m" are broad singlet, doublet, triplet, and multiplet respectively. "J" indicates the coupling constant in Hertz (Hz). Unless otherwise noted, NMR data refers to the free base of the subject compound.

NMR spectra were obtained on a Brüker Corp. 270 MHz instrument or on a General Electric QE-300 300 MHz instrument. The chemical shifts are expressed in delta (δ) values (parts per million downfield from tetramethyl-silane). MS(FD) spectra were taken on a Varian-MAT 731 Spectrometer using carbon dendrite emitters. EIMS were obtained on a CEC 21-110 instrument from Consolidated Electrodynamics Corporation. MS(FAB) spectra were obtained on a VG ZAB-3 Spectrometer. IR spectra were obtained on a Perkin-Elmer 281 instrument. UV spectra were obtained on a Cary 118 instrument. TLC was carried out on E. Merck silica gel plates. Melting points (m.p.) are uncorrected.

Preparation 1

A. 2,2-Dimethyl-4(R),5(R)-bis(1(R)-hydroxy-2-phenylethyl)-1,3-dioxolane

A solution of 14.6 mL (43.8 mmol) of phenylmagnesium bromide in 24.6 mL of $Et_2O$ was added to a cold (-40°C) solution of 0.278 g (1.46 mmol) of CuI in 2 mL of dimethylsulfide and 22 mL of THF, under nitrogen ($N_2$). After allowing this solution to stir for 5-10 minutes, a solution of 2.72 g (14.6 mmol) of 1,2:5,6-dianhydro-3,4-*o*-isopropylidene-D-mannitol in 12 mL of THF was added. The reaction mixture was then warmed to 0°C and allowed to react for 2 hours and 15 minutes, at which time a saturated ammonium chloride ($NH_4Cl$) solution was slowly added. The resulting solution was vigorously stirred for 5 minutes and then transferred to a separatory funnel containing a 2:1 mixture of $Et_2O$ and $H_2O$. The resulting layers were separated and the organic layer was reduced to dryness under reduced pressure. The resultant material was then purified using column chromatography (eluent of 2.5% acetone in $CH_2Cl_2$). The fractions containing the desired product were combined and reduced to dryness under reduced pressure to provide 4.07 g of an oil.

$^1H$ NMR ($CDCl_3$): δ 7.35 (m, 10); 3.78 (m, 4); 3.15 (dd, 2); 2.90 (d, 2); 2.70 (m, 2); 1.45 (s, 6).

MS: m/e 342 ($M^+$).

B. 2,2-Dimethyl-4(R),5(R)-bis(1(R)-methanesulfonyloxy-2-phenylethyl)-1,3-dioxolane

A solution of 2.97 mL (38.3 mmol) of methanesulfonyl chloride in 20 mL of $CH_2Cl_2$ was added to a cold (0°C) solution of 5.47 mL (39.3 mmol) of $Et_3N$ and 6.4 g (18.7 mmol) of the subtitled intermediate of Preparation 1A, in 20 mL of $CH_2Cl_2$. The reaction mixture was then warmed to room temperature. When the reaction was complete, as determined by TLC, the reaction mixture was poured into a solution of 50 mL of 0.2N HCl and 150 mL of $Et_2O$. The resulting layers were separated and the organic layer was washed with a saturated sodium bicarbonate solution and then reduced to dryness under reduced pressure. The resultant material was then purified using column chromatography (eluent of a 1:1 $Et_2O$/hexane solution). The fractions containing the desired intermediate were combined and reduced to dryness under reduced pressure.

Yield: 3.7 grams.

$^1H$ NMR ($CDCl_3$): δ 7.30 (m, 10); 4.88 (m, 2); 4.25 (d, 2); 3.10 (m, 4); 2.32 (s, 6); 1.52 (s, 6).

MS: m/e 499 ($M^+$ +1).

C. 2,2-Dimethyl-4(R),5(R)-bis(1(S)-azido-2-phenylethyl)-1,3-dioxolane

A solution of 0.784 g (16.0 mmol) of lithium azide, 4.0 g (14.6 mmol) of 18-crown-6 and 1.7 mL (14.6 mmol) of 2,6-lutidine was added to 3.6 g (7.2 mmol) of the subtitled intermediate of Preparation 1B, in 25 mL of N,N'-dimethylpropyleneurea. The reaction mixture was then heated to 95-100°C and allowed to react for approximately 7 hours. When the reaction was complete, as determined by TLC, the reaction mixture was poured into 200 mL of a 1:1 mixture of 0.1N HCl and $Et_2O$. The resulting layers were separated and the organic layer was dried with sodium sulfate ($Na_2SO_4$) and then reduced to dryness under reduced pressure to provide 2.8 g of an oil. This oil was purified using column chromatography (gradient eluent of 5-10% $CH_2Cl_2$ in toluene). The fractions containing the desired product were combined and reduced to dryness under reduced pressure to provide 1.03 g of the subtitled intermediate.

$^1H$ NMR ($CDCl_3$): δ 7.30 (m, 10); 4.12 (s, 2); 3.22 (t, 2); 3.05 (m, 4); 1.55 (s, 6).

D. 1,6-Diphenyl-2(S),5(S)-diazido-3(R),4(R)-dihydroxyhexane

To a solution of 0.370 g (0.943 mmol) of the subtitled intermediate of Preparation 1C in 4 mL of MeOH, was slowly added 1.26 mL of 12M HCl over a period of 3 hours. When the reaction was complete, as determined by TLC, the reaction mixture was diluted with 10 mL of $CH_3CN$ and concentrated two times. The resultant material was redissolved in EtOAc and then washed with a half-saturated solution of sodium bi-

carbonate ($NaHCO_3$). The resulting layers were separated and the organic layer was then reduced to dryness under reduced pressure to provide an oil. This oil was purified using column chromatography (gradient eluent of 0-20% EtOAc in $CH_2Cl_2$). The fractions containing the desired product were combined, reduced to dryness under reduced pressure and then recrystallized from a solution of $Et_2O$ and hexane to provide 0.309 g of the desired subtitled intermediate.
$^1H$ NMR ($CDCl_3$) : δ 7.25 (m, 10); 3.60 (m, 4); 3.0 (m, 4); 2.65 (d, 2).
MS: m/e 353 ($M^+$ +1).

| Analysis for $C_{18}H_{20}N_6O_2$: | | | |
|---|---|---|---|
| Calc.: | C, 61.35; | H, 5.72; | N, 23.85; |
| Found: | C, 61.15; | H, 5.73; | N, 23.70. |

E. 1,6-Diphenyl-2(S),5(S)-diazido-3(R)-methansulfonyloxy-4(R)-hvdroxyhexane

A solution of 13.5 μL (0.170 mmol) of methanesulfonyl chloride in 300 μL of $CH_2Cl_2$ was slowly added to a cold (0°C) solution of 80 mg (0.23 mmol) of the subtitled intermediate of Preparation 1D and 32 μL (0.23 mmol) of $Et_3N$ in 0.6 mL of $CH_2Cl_2$. The reaction mixture was warmed to room temperature, allowed to react for 15 minutes and then poured into 50 mL of a 3:2 mixture of $Et_2O$ and 0.1N HCl. The resulting layers were separated and the organic layer was washed with a half-saturated $NaHCO_3$ solution and then reduced to dryness under reduced pressure to provide a foam. This foam was purified using column chromatography (eluent of a 1:2 EtOAc/$CH_2Cl_2$ solution). The fractions containing the desired product were combined and reduced to dryness under reduced pressure to provide 100 mg of a 60:40 mixture of the desired subtitled intermediate and the starting material, 1,6-diphenyl-2(S),5(S)-diazido-3(R),4(R)-dihydroxyhexane.

F. 1,6-Diphenyl-2(S),5(S)-diazido-3,4-*cis*-epoxyhexane

To a cold (0°C) solution of 60 mg (0.14 mmol) of the subtitled intermediate of Preparation 1E in 1.5 mL of a 2:1 MeOH/THF solution, was added 0.325 mL of a methanolic solution of 0.5M NaOMe. The reaction mixture was then warmed to room temperature and allowed to react for one hour, followed by the addition of 3 mL of $Et_2O$ containing 2 drops of acetic acid (HOAc). The resulting solution was then poured into 65 mL of a 8:5 mixture of saturated $NaHCO_3$ and $Et_2O$. The resulting layers were separated and the organic layer was reduced to dryness under reduced pressure to provide an oil. This oil was purified using column chromatography (eluent of 20% hexane in $CH_2Cl_2$). The fractions containing the desired product were combined and reduced to dryness under reduced pressure to provide 31 mg of the desired subtitled intermediate.
$^1H$ NMR ($CDCl_3$): δ 7.30 (m, 10); 3.57 (m, 1); 3.45 (m, 1); 3.18 (m, 2); 2.94 (m, 4).

G. 1,6-Diphenyl-2(S),5(S)-diammonio-3,4-*cis*-epoxyhexane dihydrochloride

To a suspension of 350 mg of 5% Pd/C in 10 mL of a 9:1 EtOAC/HOAc solution, was added 0.368 g (1.11 mmol) of the subtitled intermediate of Preparation 1F, above. The mixture was then stirred rapidly under hydrogen ($H_2$) gas for about 3 1/2 hours. When the reaction was complete, as determined by TLC, the mixture was diluted with EtOAc and the 5% Pd/C was removed by filtration. The filtrate was then cooled overnight to provide 0.325 g of 1,6-diphenyl-2(S),5(S)-diammonio-3,4-*cis*-epoxyhexane diacetate. To a solution of 0.100 g (0.249 mmol) of the diacetate in 4 mL of a 1:1 $CH_2Cl_2$/$Et_2O$ solution, was slowly added 0.498 mL of a 1N HCl in $CH_3CN$ resulting in the formation of 0.090 g of a white solid.
$^1H$ NMR ($d_6$-DMSO): δ 8.40 (br.s, 6); 7.30 (m, 10); 3.40 (m, 4); 3.05 (m, 4).
MS: m/e 284 ($M^+$ -2Cl).

## Preparation 2

A. Benzhydryl 2(S)-ammonio-3(S)-methylpentanoate, *p*-toluenesulfonate

Diphenyldiazomethane was added to a solution of 15.03 g (49.54 mmol) of L-isoleucine, *p*-toluenesulfonate in 450 ml of a 4:5 $CH_3CN$/MeOH solution until a light pink color persisted (17.12 g (88.16 mmol) of diphenyldiazomethane), at which time 2 mL of glacial HOAc was added. The resultant solution was stirred for approximately 15 minutes at room temperature and then concentrated under reduced pressure to provide a yellow solid. This solid was then purified by recrystallization in hot $CH_3CN$ to provide 21.64 g of the desired subtitled intermediate.
$^1H$ NMR ($CD_3OD$) δ 7.67 (d, J=9 Hz, 2); 7.37 (m, 10); 7.20 (d, J=9 Hz, 2); 6.98 (s, 1); 4.13 (d, J=3 Hz, 1); 2.34 (s, 3); 2.01 (m, 1); 1.17-1.46 (m, 2); 0.80-0.97 (m, 6).

B. Benzhydryl 2(S)-amino-3(S)-methylpentanoate

To a solution of 21.64 g (46.08 mmol) of the subtitled intermediate of Preparation 2A, was added 450 mL of a saturated $NaHCO_3$ solution which resulted in the release of a gas. When the reaction was complete, the resultant layers were separated and the organic layer was reduced to dryness under reduced pressure to provide

13.61 g of the desired subtitled intermediate.

$^1H$ NMR ($CDCl_3$) δ 7.19-7.45 (m, 10); 7.93 (s, 1); 3.47 (d, J=3 Hz, 1); 1.85 (m, 1); 1.33-1.50 (m, 4); 0.92 (d, J=8 Hz, 3); 0.83 (t, J=8, 3).

C. Benzhydryl 2(S)-carbamoyl-3(S)-methylpentanoate

A solution of 4.97 g (16.7 mmol) of the subtitled intermediate of Preparation 2B and 4.7 mL (33.7 mmol) of $Et_3N$ was cannulated into a hot (60°C) solution of 2.51 g (8.46 mmol) of triphosgene in 90 mL of toluene. The temperature was then increased to 100°C and the solution was allowed to react overnight. The reaction mixture was cooled to 0°C resulting in the formation of a precipitate. This precipitate was removed by filtration and washed with a 1:1 EtOAc/hexane solution. The resulting solution was reduced to dryness under reduced pressure to provide 5.45 g of the desired subtitled intermediate of sufficient purity (ca. 90% by NMR) for use in subsequent reactions.

$^1H$ NMR ($CDCl_3$) δ 7.23-7.47 (m, 10); 6.98 (s, 1); 4.05 (d, J=3 Hz, 1); 2.05 (m, 1); 1.18 (quintet, J=8 Hz, 2); 1.00 (d, J=8 Hz, 3); 0.80 (t, J=8 Hz, 3).

D. Benzhydryl 2(S)-N-[(quinol-2-ylmethoxy)carbonyl]amino-3(S)-methylpentanoate

To a solution of 0.506 g (3.18 mmol) of 2-hydroxymethylquinoline and 0.315 g (3.18 mmol) of copper (I) chloride in anhydrous DMF, was added 1.13 g (3.50 mmol) of the subtitled intermediate of Preparation 2C. When the reaction was substantially complete, as indicated by TLC, the reaction mixture was diluted with EtOAc and washed with a half-saturated brine solution. The resulting layers were separated and the organic layer was dried over $Na_2SO_4$ and reduced to dryness under reduced pressure to provide a brown oil. This oil was purified by column chromatography (eluent of 65% hexane in EtOAc) to provide 1.23 g of the desired subtitled intermediate.

$^1H$ NMR ($CDCl_3$) δ 8.15 (d, 1); 8.05 (d, 1); 7.8 (d, 1); 7.70 (t, 1); 7.55 (t, 1); 7.45 (d, 1); 7.30 (m, 10); 6.92 (s, 1); 5.43 (d, 1); 5.40 (s, 2); 4.5 (m, 1); 2.0 (m, 1); 1.2 (m, 2); 0.9 (d, 3); 0.82 (t, 3).

E. 2(S)-N-[(Quinolin-2-ylmethoxy)carbonyl]amino-3(S)-methylpentanoic acid

The subtitled intermediate of Preparation 2D, above, was dissolved in 12 mL of dioxane containing 3 mL of concentrated HCl. The resulting solution was heated to 100°C and allowed to react for approximately 5 minutes, then cooled to room temperature and stirred until the reaction was substantially complete, as indicated by TLC. The resulting solution was reduced to dryness under reduced pressure and then redissolved in a saturated $NaHCO_3$ solution. The resulting solution was washed with 100 mL of $Et_2O$ and then acidified to pH 4 using 1N HCl. The desired product was then extracted with a solution of 10% isopropanol (iPr) in $CH_2Cl_2$, dried over $Na_2SO_4$ and filtered. The filtrate was then reduced to dryness under reduced pressure to provide 0.567 g of the desired subtitled intermediate.

$^1H$ NMR ($d_6$-DMSO) δ 8.35 (d, 1); 7.92 (d, 2); 7.70 (m, 2); 7.53 (m, 2); 5.22 (s, 2); 3.92 (m, 1); 1.78 (m, 1); 1.40 (m, 1); 1.20 (m, 1); 0.90 (m, 6).

Preparation 3

A. N-*t*-Butyl-2-methylbenzamide

To a cold (0°C) solution of 139.2 g (0.9 mol) of *o*-toluoyl chloride in 1200 mL of $CH_2Cl_2$, under $N_2$, was slowly added 180.0 g (1.8 mol) of $Et_3N$ followed by the dropwise addition of a solution containing 73.14 g (1.0 mol) of *t*-butylamine in 200 mL of $CH_2Cl_2$. The resulting reaction mixture was warmed to room temperature and allowed to react for 2.5 hours. The reaction mixture was then diluted with 1800 mL of $H_2O$. The resulting layers were separated, and the organic layer was washed sequentially with 2N NaOH, 1.0N HCl and brine, dried over $MgSO_4$, filtered and then reduced to dryness under reduced pressure to provide 167.6 g of an off-white solid. (mp 77-78°C).

Yield: 97%.

$^1H$ NMR ($CDCl_3$): δ 1.41 (s, 9H); 2.41 (s, 3H); 5.54 (br.s, 1H); 7.13-7.30 (m, 4H).

IR ($CHCl_3$): 3430, 3011, 2971, 2932, 1661, 1510, 1484, 1452, 1393, 1366, 1304, 1216, 876 $cm^{-1}$.

MS (FD): m/e 191 ($M^+$), 191 (100).

| Analysis for $C_{12}H_{17}NO$: | | | |
|---|---|---|---|
| Calcd: | C, 75.35; | H, 8.76; | N, 7.32; |
| Found: | C, 75.10; | H, 9.11; | N, 7.20. |

B. (S)-N-*t*-Butyl-2-(3-(N-benzyloxycarbonyl)amino-2-oxo-4-phenylbutyl)benzamide

To a solution of 7.0 g (36.5 mmol) of the subtitled intermediate of Preparation 3A in 200 mL of anhydrous THF, was added 12.1 mL (80.3 mmol) N,N,N',N'-tetramethylethylenediamine (TMEDA) was added via syringe. The resulting solution was cooled to -78°C and then 55.9 mL of sec-butyllithium was added dropwise via syringe while maintaining the temperature of the reaction under -60°C. The resulting reaction solution was then allowed to stir for approximately 1 hour at -78°C before the addition of a solution containing 5.00 g (14.6 mmol) of (S)-N-methoxy-N-methyl-2-(N-phenylmethyloxycarbonyl)amino-3-phenylpropanamide in 50 mL of anhydrous THF, added via cannula while maintaining the reaction temperature below -65°C. The resulting reaction mixture was warmed to -20°C, quenched using 20 mL of saturated $NH_4Cl$ and then diluted with 200 mL of $Et_2O$. The resulting layers were separated and the organic layer was washed sequentially with $H_2O$, 0.2N sodium hydrogen sulfate ($NaHSO_4$) and brine, dried over $Na_2SO_4$, filtered and then reduced to dryness under reduced pressure to provide a colorless oil. This oil was purified using flash chromatography (eluent of 25% EtOAc in $CH_2Cl_2$).

Yield: 6.08 g (88%) of a colorless foam.

$[a]_D$ -289.26° (*c* 0.12, MeOH).

$^1H$ NMR ($CDCl_3$): δ 1.38 (s, 9H); 2.99 (dd, J=15; 6 Hz, 1H); 3.24 (dd, J=15, 6 Hz, 1H); 3.89 (d, J=18 Hz, 1H); 4.16 (d, J=18 Hz, 1H); 4.72 (dd, J=15, 6 Hz, 1H); 5.00-5.09 (m, 2H); 5.56 (d, J=6 Hz, 1H); 5.93 (br.s, 1H); 7.03-7.40 (m, 14H).

IR ($CHCl_3$): 3431, 3027, 3012, 2973, 1713, 1658, 1511, 1454, 1383, 1366, 1307, 1231, 1046 $cm^{-1}$.

MS (FD): m/e 472 ($M^+$), 218 (100).

| Analysis for $C_{29}H_{32}N_2O_4$: | | | |
|---|---|---|---|
| Calcd: | C, 73.70; | H, 6.82; | N, 5.93; |
| Found: | C, 73.41; | H, 6.98; | N, 5.83. |

C. [2R-(*2R**,*3S**)]-N-*t*-Butyl-2-(3-(N-benzyloxycarbonyl)amino-2-hydroxy-4-phenylbutyl)benzamide

To a solution of 6.96 g (14.7 mmol) of the subtitled intermediate of Preparation 3B in 200 mL of absolute ethanol (EtOH), under $N_2$, was added 2.78 g (73.5 mmol) of sodium borohydride. When the reaction was substantially complete, as indicated by TLC, the reaction mixture was diluted with 200 mL of EtOAc and quenched by the dropwise addition of 20 mL of saturated $NH_4Cl$. The resulting layers were separated and the organic layer was washed sequentially with 1N HCl, saturated $NaHCO_3$ and brine, dried over $NaSO_4$, filtered and then reduced to dryness under reduced pressure to provide 6.4 g of a colorless oil. This oil was purified using flash chromatography (gradient eluent of 2-10% $CH_2Cl_2$ in EtOAc) to provide 5.12 g of the major, desired diastereomer.

Yield: 74%.

$[a]_D$ +10.38° (*c* 0.10, MeOH).

$^1H$ NMR ($CDCl_3$): δ 1.40 (s, 9H); 2.79 (dd, J=12; 3 Hz, 1H); 2.90-2.98 (m, 2H); 3.04 (44, J=12, 3 Hz, 1H); 3.70-3.81 (m, 1H); 3.97 (m, 1H); 4.96-5.08 (m, 2H); 5.10 (d, J=9 Hz, 1H); 5.88 (d, J=6 Hz, 1H); 5.93 (s, 1H); 7.13-7.42 (m, 14H).

IR ($CHCl_3$): 3431, 3028, 3012, 2971, 1773, 1643, 1515, 1454, 1367, 1229, 1028 $cm^{-1}$.

MS (FD): m/e 475 ($M^+$), 475 (100).

| Analysis for $C_{29}H_{34}N_2O_4$: | | | |
|---|---|---|---|
| Calcd: | C, 73.39; | H, 7.22; | N, 5.99; |
| Found: | C, 73.12; | H, 7.48; | N, 5.62. |

D. [2R-(*2R**,*3S**)]-N-*t*-Butyl-2-(3-amino-2-hydroxy-4-phenylbutyl)benzamide

A suspension was prepared containing 41.0 g (120 mmol) of the subtitled intermediate of Preparation 3C and 500 mg of 10% Pd/C in 150 mL of absolute EtOH. This suspension was shaken under 60 psi of $H_2$ gas in a Parr shaker apparatus. The 10% Pd/C was then removed by filtration and the resultant filtrate

was reduced to dryness under reduced pressure to provide 31.1 g of the desired subtitled intermediate as a light yellow foam. This foam was used without further purification.

Yield: 96%.

[a]$_D$ +34.68° (*c* 1.0, MeOH).

$^1$H NMR ($CDCl_3$): δ 1.46 (s, 9H); 2.71 (dd, J=13.7; 9.5 Hz, 1H); 2.84 (dd, J=13.3; 2.51 Hz, 1H); 2.95-3.06 (m, 2H); 3.23-3.29 (m, 1H); 3.84-3.90 (m, 1H); 6.23 (s, 1H); 7.19-7.37 (m, 12H).

IR ($CHCl_3$): 3440, 3382, 3007, 2970, 2934, 1643, 1516, 1454, 1367, 1213 cm$^{-1}$.

MS (FD): m/e 341 (M$^+$), 341 (100).

Preparation 4 was prepared substantially in accordance with the procedure detailed in Preparation 3A-C. The resultant compound was deprotected before further reaction as detailed in Preparation 3D.

Preparation 4

[2R-(*2R**,*3S**)]-N-*t*-Butyl-2-(3-(N-benzyloxycarbonyl)amino-2-hydroxy-4-phenylbutyl)-5-methylbenzamide

Yield: 4.84 mg (82%) of a colorless foam.

[a]$_D$ +10.31° (c 0.58, MeOH).

$^1$H NMR ($CDCl_3$): δ 1.46 (s, 9H); 2.32 (s, 3H); 2.76 (d, J=15 Hz, 1H); 2.85-2.95 (m, 2H); 3.04 (dd, J=15,5 Hz, 1H); 3.67-3.74 (m, 1H); 3.92-4.05 (m, 1H); 4.92-5.14 (m, 3H); 5.86 (d, J=7 Hz, 1H); 5.91 (s, 1H); 7.03-7.40 (m, 13H).

IR ($CHCl_3$): 3431, 3274, 3027, 3012, 2970, 1713, 1643, 1514, 1496, 1454, 1367, 1224, 1039, 910 cm$^{-1}$.

MS (FD): m/e 489(M$^+$), 205(100).

| Analysis for $C_{30}H_{36}N_2O_4$: | | | |
|---|---|---|---|
| Calcd: | C, 73.74; | H, 7.43; | N, 5.73; |
| Found: | C, 73.55; | H, 7.50; | N, 5.96. |

Deprotection provided 4.2 g (89%) of a colorless foam.

Preparation 5

A. Quinaldic acid pentafluorophenyl ester

To a suspension containing 15.0 g (86.6 mmol) of quinaldic acid and 20.8 g (113 mmol) of pentafluorophenol in 200 mL of THF, was added 18.3 g (95.3 mmol) of EDC. The resulting reaction mixture was vigorous stirred at room temperature for approximately two hours, during which time a gummy precipitate formed at the bottom of the flask. The solution was decanted from the gum, and the desired compound was extracted from the gum using $CH_2Cl_2$. The resultant solution was then diluted with hexane and washed sequentially with 0.1N $NaHSO_4$, 1N potassium carbonate ($K_2CO_3$) and brine solutions. The resulting layers were separated and the organic layer was dried over $Na_2SO_4$, filtered and then concentrated under reduced pressure to provide a pale pink solid. This solid was redissolved in 30 mL of hot $Et_2O$ and then diluted with 400 mL of hot hexane. The resulting solution was slowly cooled to room temperature to provide 21.6 g of colorless needles.

Yield: 73%.

$^1$H NMR ($CDCl_3$): δ 7.73 (t, J=7.5 Hz, 1H); 7.86 (t, J=7.9 Hz, 1H); 7.95 (d, J=8.2 Hz, 1H); 8.29-8.42 (m, 3H).

IR ($CHCl_3$): 3035, 2997, 1763, 1522, 1285, 1068, 998, 842 cm$^{-1}$.

| Analysis for $C_{16}H_6NO_2F_5$: | | | |
|---|---|---|---|
| Calcd: | C, 56.65; | H, 1.78; | N, 4.13; |
| Found: | C, 56.66; | H, 1.77; | N, 4.12. |

B. (S)-2-N-(quinolin-2-ylcarbonyl)amino-3-carbamoyl propanoic acid

A solution was prepared containing 17.9 g (51.2 mmol) of the subtitled compound of Preparation 5A, 6.99 g (46.6 mmol) of L-asparagine monohydrate and 15.7 g (186 mmol) of $NaHCO_3$ in 265 mL of $H_2O$ and 219 mL of dioxane. The resulting suspension was stirred vigorously overnight at room temperature. The reaction mixture was then concentrated under reduced pressure to remove the dioxane and the remaining

aqueous layer was acidified to pH 3 using 2N $NaHSO_4$. The desired subtitled compound was then extracted from the aqueous layer with 60 mL of a 3:1 chloroform ($CHCl_3$)/iPr solution. The organic layers were then washed with a brine solution, dried over $Na_2SO_4$, filtered and then reduced to dryness under reduced pressure to provide a colorless solid. This solid was washed with 500 mL of $Et_2O$ and 250 mL of hot hexanes and then dried at 80°C under reduced pressure for three hours to provide 10.61 g of the desired subtitled intermediate.

Yield: 79%.

$[a]_D$ +16.54° (*c* 1.01, DMSO).

$^1H$ NMR (DMSO-$d_6$): δ 2.68 (dd, J=16.0, 4.9 Hz, 1H); 2.81 (dd, J=16.0, 5.7 Hz, 1H); 4.74-4.81 (m, 1H); 6.96 (s, 1H); 7.70 (t, J=7.5 Hz, 1H); 7.85 (t, J=7.5 Hz, 1H); 8.05-8.15 (m, 3H); 8.56 (d, J=8.5 Hz, 1H); 9.12 (d, J=8.6 Hz, 1H); 12.8 (s, 1H).

IR (KBr): 3385, 3367, 3216, 1171, 1662, 1523, 1499, 1427, 780, 592 $cm^{-1}$.

MS (FD) m/e 288($M^+$), 288(100).

| Analysis for $C_{14}H_{13}N_3O_4$: | | | |
|---|---|---|---|
| Calcd: | C, 58.53; | H, 4.56; | N, 14.63; |
| Found: | C, 58.80; | H, 4.57; | N, 14.56. |

Preparations 6-8 were prepared substantially in accordance with the procedure detailed in Preparation 5A and B.

Preparation 6 (S)-2-N-(quinoxalin-2-ylmethylcarbonyl)amino-3-carbamoyl propanoic acid

Preparation 7 (S)-2-N-(naphth-2-ylmethylcarbonyl)-amino-3-carbamoyl propanoic acid

Preparation 8 (S)-2-N-(quinolin-2-ylcarbonyl)amino-3-carboxy propanoic acid

Preparation 9

A. [3S-(*3R**,*4aR**,*8aR**,*2'S**,*3'R**)]-2-[3'-N(Benzyloxycarbonyl)amino-2'-hydroxy-4'-phenyl]butyl-N(*t*-butyl)decahydroisoquinoline-3-carboxamide

A solution of [1R-(*1R**,*3S**,*1'S**,*4aS**,*8aS**)]-1-[(1'-N-benzyloxycarbonylamino-2'-phenyl)ethyl]oxirane and decahydroisoquinoline-3-N-*t*-butylcarboxamide in absolute EtOH was heated at 80°C overnight. The reaction mixture was reduced to dryness under reduced pressure to provide a residue. This residue was purified using flash chromatography (gradient eluent of 10-50% EtOAc in $CH_2Cl_2$) to provide 6.47 g (75%) of an off-white foam.

$^1H$ NMR ($CDCl_3$): δ 1.29 (s, 9H); 1.25-2.05 (m, 2H); 2.20-2.35 (m, 2H); 2.55-2.70 (m, 11H); 2.85-3.10 (m, 3H); 3.24 (br.s, 1H); 3.82 (br.s, 1H); 3.98 (br.s, 1H); 4.99 (br.s, 2H); 5.16-5.18 (m, 1H); 5.80 (br.s, 1H); 7.05-7.38 (m, 10H).

IR ($CDCl_3$): 3600-3100 (br.), 3031, 2929, 1714, 1673, 1512, 1455, 1368, 1232, 1199, 1047 $cm^{-1}$.

MS (FD): m/e 536($M^+H$), 1068(100).

B. [3S-(*3R**,4a*R**,8a*R**,*2'S**,3'*R**)]-2-[3'-Amino-2'-hydroxy-4'-phenyl]butyl-decahydroisoquinoline-3-N-*t*-butylcarboxamide

The desired subtitled compound was prepared substantially in accordance with the procedure detailed in Procedure 3D, using 6.37 g (11.91 mmol) of the subtitled intermediate of Preparation 9A and 1.2 g of 10% Pd/C in 200 mL of absolute EtOH to provide 5.09 g of a solid. This compound was used without further purification.

$^1H$ NMR ($CDCl_3$): δ 1.33 (s, 9H); 1.40-1.95 (m, 10H); 2.25-2.48 (m, 2H); 2.59-2.75 (m, 3H); 2.80-3.40 (m, 7H); 3.75-3.90 (m, 1H); 6.19 (br.s, 1H); 7.18-7.35 (m, 5H).

IR ($CDCl_3$): 3600-3100 (br.), 2929, 2865, 1671, 1515, 1455, 1367, 1245, 1047 $cm^{-1}$.

MS (FD) m/e 402($M^+$, 100).

Preparation 10

A. N-[(1'-Oxo-1'-(3"-iodo-4"-methyl)phenyl)methyl]-1,2,3,4-tetrahydroisoquinoline

To a solution containing 5.00 g (19.1 mmol) of 3-iodo-4-methylbenzoic acid and 3.40 g (21.0 mmol) of 1,1-carbonyldiimidazole in 80 mL of THF, under $N_2$, was added 2.7 mL (21.0 mmol) of 1,2,3,4-tetrahydroisoquinoline, by syringe. The resulting reaction mixture was allowed to react for approximately 2 hours and then reduced to dryness under reduced pressure to provide a residue. This residue was redissolved

in 100 mL of EtOAc, washed sequentially with saturated $NaHCO_3$ and brine solutions, dried over $Na_2SO_4$, filtered and then reduced to dryness under reduced pressure to provide a light yellow oil. This oil was purified using flash chromatography (gradient eluent of
14-20% hexane in EtOAc) to provide 6.92 g of a clear oil.
Yield: 96%.
$^1H$ NMR ($CDCl_3$): δ 2.46 (s, 3H); 2.90 (br.s, 2H); 3.65 (br.s, 1H); 3.95 (br.s, 1H); 4.85 (br.s, 1H); 7.17-7.34 (m, 6H); 7.90 (s, 1H).
IR ($CDCl_3$): 3010, 1624, 1586, 1547, 1497, 1370, 1300, 1253, 1108, 1050, 1035, 831 $cm^{-1}$.
MS (FD): m/e 377($M^+$, 100).

| Analysis for $C_{17}H_{16}NOI$: | | | | |
|---|---|---|---|---|
| Calcd: | C, 54.13; | H, 4.28; | N, 3.71; | I, 33.64; |
| Found: | C, 53.89; | H, 4.24; | N, 3.61; | I, 33.52. |

B. N-[1'-Oxo-1'-(3"-methoxycarbonyl-4"-methyl)phenyl]methyl-1,2,3,4-tetrahydroisoquinoline

A solution containing 6.35 g (16.8 mmol) of the subtitled compound of Preparation 10A, 3.35 mL (16.8 mmol) of dicyclohexylamine and 1.18 g (1.68 mmol) of bis(triphenylphosphine)palladium (II) chloride in 150 mL of anhydrous MeOH was vigorously stirred under an atmosphere of carbon monoxide. When the reaction was substantially complete, as indicated by TLC, the mixture was reduced to dryness under reduced pressure to provide a residue. This residue was redissolved in 200 mL of EtOAc and the resulting mixture filtered through celite to remove insoluble organic salts. The resultant filtrate was washed sequentially with saturated $NaHCO_3$ and brine solutions, dried over $Na_2SO_4$, filtered and then reduced to dryness under reduced pressure. The resultant material was purified using flash chromatography (gradient eluent of
14-20% EtOAc in hexane) to provide 4.54 g of a clear oil.
Yield: 87%.
$^1H$ NMR ($CDCl_3$): δ 2.64 (s, 3H); 2.90 (br.s, 2H); 3.89 (s, 3H); 3.98 (br.s, 1H); 4.59 (br.s, 1H); 4.87 (br.s, 1H); 7.05-7.51 (m, 6H); 8.03 (s, 1H).
IR ($CDCl_3$) 3010, 1722, 1626, 1584, 1497, 1436, 1306, 1268, 1236, 1210, 1149, 1109, 1085 $cm^{-1}$.
MS (FD): m/e 309($M^+$, 100).

| Analysis for $C_{19}H_{19}NO_3$: | | | |
|---|---|---|---|
| Calcd: | C, 73.77; | H, 6.19; | N, 4.53; |
| Found: | C, 73.95; | H, 6.43; | N, 4.57. |

C. N-[1'-Oxo-1'-(3"-carboxy-4"-methyl)phenyl]methyl-1,2,3,4-tetrahydroisoquinoline

To a solution containing 4.25 g (13.8 mmol) of the subtitled compound of Preparation 10B in 200 mL of a 3:1 THF/$H_2O$ mixture, was added 661 mg (27.6 mmol) of lithium hydroxide (LiOH). The reaction mixture was reacted for approximately 24 hoursand then was concentrated under reduced pressure and acidified (pH 2-3) by the dropwise addition of 1N HCl, resulting in precipitation of the crude product. The resulting suspension was combined with 75 mL of EtOAc, and the resulting aqueous and organic layers were separated. The aqueous phase was extracted twice with EtOAc and the combined organic layers were washed with brine, dried over $Na_2SO_4$, filtered and then reduced to dryness under reduced pressure to provide a residue. This residue was purified using column chromatography (silica gel; eluent of 15% MeOH in $CH_2Cl_2$) to provide 4.05 g of an off-white foam.
$^1H$ NMR ($CDCl_3$): δ 2.68 (s, 3H); 2.92 (br.s, 2H); 3.67 (br.s, 1H); 4.00 (br.s, 1H); 4.62 (br.s, 1H); 4.91 (br.s, 1H); 7.18-7.58 (m, 6H); 8.17 (s, 1H).
IR ($CDCl_3$): 3500-2500 (br.), 1699, 1625, 1584, 1498, 1445, 1371, 1301, 1237, 1202, 1167, 1060, 981, 934, 838 $cm^{-1}$.
MS (FD): m/e 295($M^+$, 100).

| Analysis for $C_{18}H_{17}NO_3$: | | | |
|---|---|---|---|
| Calcd: | C, 73.20; | H, 5.80; | N, 4.74; |
| Found: | C, 73.14; | H, 5.90; | N, 4.44. |

Preparation 11

N-[1'-Oxo-1'-(3"-carboxy-4"-ethyl)phenyl]methyl-1,2,3,4-tetrahydroisoquinoline

The titled compound was prepared substantially in accordance with Preparation 10.
Yield: 1.35 g (88%) of a white foam.
$^1$H NMR ($CDCl_3$) δ 1.27 (t, J=7.5 Hz, 3H); 2.96 (br.s, 2H); 3.10 (q, J=7.4 Hz, 2H); 3.66 (br.s, 1H); 3.99 (br.s, 1H); 4.62 (br.s, 1H); 4.89 (br.s, 1H); 7.13-7.25 (m, 4H); 7.38 (d, J=7.9 Hz, 1H); 7.58 (d, J=7.2 Hz, 1H); 8.12 (d, J=1.6 Hz, 1H). IR (KBr): 3600-2300 (br.); 1700, 1625, 1497, 1443, 1300, 1237, 1150, 1109, 1048, 933 $cm^{-1}$.
MS (FD): m/e 309($M^+$, 100).

Preparation 12

1,6-Diphenyl-2(S);5(S)-diammonio-3(R);4(R)-dihydroxyhexane

The titled compound was prepared substantially in accordance with the procedure detailed in Preparation 1G, using 285 mg (0.81 mmol) of the subtitled compound of Preparation 1D and 143 mg of Pd/C in 9 mL of EtOH containing 1 mL of concentrated HCl, to provide 175 mg (58%) of a white solid.
$^1$H NMR ($CD_3OD$): δ 1.98 (d, J=2 Hz, 2H); 2.79-3.07 (m, 4H); 3.60 (br., 2H); 3.75 (br., 2H); 7.20-7.37 (m, 10H).
MS (FD): 301 ($M^+$).

Example 1

1,6-Diphenyl-2(S);5(S)-di[N-[2(S)-N(benzyloxycarbonyl)amino-3-methyl-butanoyl]amino]-3,4-*cis*-epoxyhexane

A solution of 215 mg (0.818 mmol) of 2(S)-N(benzyloxycarbonyl)amino-3-methylbutanoic acid, 12 mg (0.082 mmol) of HOBT·$H_2O$, 104 μL (0.818 mmol) of NMM and 362 mg (0.818 mmol) of benzotriazol-1-yloxytris(dimethylamino)phosphonium hexafluorophosphate (BOP) in 2 mL of DMF was prepared and allowed to stir at room temperature for approximately 10 minutes. Concurrently, 80 μL (0.40 mmol) of N,N-dicyclohexylamine was added to a solution of 71 mg (0.20 mmol) of the subtitled intermediate of Preparation 1G in 6 mL of DMF, resulting in the formation of a white solid. This solid was removed by centrifugation and the filtrate was added to the above solution. The resulting reaction mixture was concentrated under reduced pressure to a volume of 2.0 mL and allowed to react for about 48 hours at room temperature. The reaction mixture was then diluted with 20 mL of EtOAc and the resulting solution was added to 120 mL of a 2:1 EtOAc/0.1N HCl. The resultant layers were separated and the organic layer was washed with a 1:1 mixture of saturated $NaHCO_3$/brine solutions, dried with $Na_2SO_4$, filtered and then reduced to dryness under reduced pressure to provide 110 mg of material. This material was then purified using flash chromatography (eluent of 65% toluene in EtOAc) to provide 52 mg of the desired titled product.
$^1$H NMR (80% $CDCl_3$/20% $CD_3OD$): δ 7.35 (s, 10); 7.18 (m, 10); 5.12 (s, 4); 4.20 (d, 2); 4.12 (m, 1); 3.90 (d, 1); 3.18 (m, 1); 2.82 (m, 5); 2.05 (m, 2); 0.90 (m, 9); (d, 3).
MS: m/e 749 ($M^+$ +1).

| Analysis for $C_{44}H_{52}N_4O_7$: | | | |
|---|---|---|---|
| Calc.: | C, 70.57; | H, 7.00; | N, 7.48; |
| Found: | C, 70.31; | H, 7.11; | N, 7.76. |

The following Examples 2-5 were prepared substantially in accordance with the procedure detailed in Example 1, using HOBT·$H_2O$, NMM and BOP.

Example 2

1,6-Diphenyl-2(S);5(S)-di[N-[2(S)-N(benzyloxycarbonyl)aminobutanoyl]amino]-3,4-*cis*-epoxyhexane

The titled compound was prepared using 194 mg (0.818 mmol) of 2(S)-N(benzyloxycarbonyl)aminobutanoic acid and 71 mg (0.20 mmol) of the subtitled intermediate of Preparation 1G to provide 21 mg of the desired compound.
$^1$H NMR (80% $CDCl_3$/20% $CD_3OD$): δ 7.35 (s, 10); 7.20 (m, 10); 5.15 (s, 4); 4.25 (d, 2); 4.10 (m, 1); 3.90 (d, 1); 3.15 (m, 1); 2.80 (m, 5); 1.45 (m, 4); 0.8 (m, 6).

Example 3

1,6-Diphenyl-2(S);5(S)-di[N-[2(S)-N[(quinolin-2-ylmethoxy)carbonyl]amino-3(S)-methylpentanoyl]amino]-3,4-*cis*-epoxyhexane

The titled compound was prepared using 365 mg (1.15 mmol) of the subtitled intermediate of Preparation 2E and 100 mg (0.282 mmol) of the subtitled intermediate of Preparation 1G. The resultant material was purified using reverse phase HPLC (45% $CH_3CN$/30% MeOH/20% $H_2O$ containing 0.5% $NH_4OAc$) to provide 90 mg.

$^1H$ NMR ($CD_3OD$): δ 8.25 (d, 2); 7.95 (d, 2); 7.85 (d, 2); 7.70 (m, 2); 7.55 (m, 4); 7.10 (m, 10); 5.35 (m, 4); 4.25 (m, 2); 4.20 (d, 1); 4.0 (m, 2); 3.22 (m, 1); 2.80 (m, 4); 1.8 (m, 2); 1.5 (m, 1); 1.3 (m, 1); 1.1 (m, 2); 0.8 (m, 12).
MS: m/e 879 ($M^+$ +1).

| Analysis for $C_{52}H_{58}N_6O_7$: | | | |
|---|---|---|---|
| Calc.: | C, 71.05; | H, 6.65; | N, 9.56; |
| Found: | C, 71.05; | H, 6.70; | N, 9.75. |

Example 4

1,6-Diphenyl-2(S);5(S)-di[N-[2(S)-N-[[N(methyl)-N(quinolin-2-ylmethyl)amino]carbonyl]aminobutanoyl]amino]-3,4-*cis*-epoxyhexane

The titled compound was prepared using 187 mg (0.62 mmol) of 2(S)-N-[[N(methyl)-N(quinolin-2-ylmethyl)-amino]carbamoyl]aminobutanoic acid and 63 mg (0.18 mmol) of the subtitled intermediate of Preparation 1G. The resultant material was purified using reverse phase HPLC (50% $CH_3CN$/20% MeOH/30% $H_2O$ containing 0.5% $NH_4OAc$) to provide 50 mg of the desired product.

$^1H$ NMR ($CD_3OD$): δ 8.25 (dd, 2); 7.95 (m, 2); 7.85 (m, 2); 7.70 (m, 2); 7.55 (t, 2); 7.40 (dd, 2); 7.15 (m, 10); 4.80 (ABX, 4); 4.35 (m, 2); 4.15 (m, 2); 4.0 (m, 1); 3.25 (m, 1); 3.02 (s, 3); 2.97 (s, 3); 2.80 (m, 4); 1.7 (m, 4); 0.85 (m, 6).
MS: m/e 849 ($M^+$ +1).

Example 5

1,6-Diphenyl-2(S);5(S)-di[N-[2(S)-N[[N(methyl)-N(quinolin-2-ylmethyl)amino]carbonyl]amino-3(S)-methylpentanoyl]amino]-3,4-*cis*-epoxyhexane

The titled compound was prepared using 232 mg (0.704 mmol) of 2(S)-N-[[N(methyl)-N(quinolin-2-ylmethyl)-amino]carbonyl]amino-3(S)-methylpentanoic acid and 72 mg (0.201 mmol) of the subtitled intermediate of Preparation 1G. The resultant material was purified using reverse phase HPLC (50% $CH_3CN$/20% MeOH/30% $H_2O$ containing 0.5% $NH_4OAc$).

$^1H$ NMR ($CD_3OD$) : δ 8.25 (dd, 2); 7.95 (m, 2); 7.85 (d, 2); 7.70 (m, 2); 7.55 (t, 2); 7.40 (d, 2); 7.15 (m, 10); 4.75 (ABX, 4); 4.38 (d, 1); 4.28 (t, 1); 4.20 (m, 1); 4.0 (m, 2); 3.25 (m, 1); 3.02 (s, 6); 2.75 (m, 4); 1.78 (m, 2); 1.05-1.45 (m, 4); 0.75 (m, 12).
MS: m/e 906 ($M^+$ +2).

Example 6

[1S-*(1R**,*4R**,*5S***)*]-N-[1-(2-amino-2-oxoethyl)-2-oxo-3-aza-4-phenylmethyl-5-hydroxy-6-(2-(1-*t*-butylamino-1-oxomethyl)phenyl)hexyl]-2-quinolinyl carboxamide

To a cold (-10°C) solution containing 500 mg (1.46 mmol) of the subtitled intermediate of Preparation 3D, 443 mg (1.54 mmol) of (S)-2-N(quinolin-2-ylcarbonyl)-amino-3-carbamoylpropanoic acid and 208 mg (1.54 mmol) of HOBT·$H_2O$ in 10 mL of anhydrous THF and 1.75 mL of anhydrous DMF, was added 309 mg (1.50 mmol) of dicyclohexylcarbodiimide (DCC), under $N_2$. The resulting reaction mixture was allowed to react at -10°C for approximately twenty minutes, then at 0°C for 1 hour and then overnight at room temperature. The reaction mixture was then cooled to 0°C and filtered to remove the resultant precipitate. The filtrate was concentrated under reduced pressure to provide a residue which was redissolved in 50 mL of EtOAc, washed sequentially with $H_2O$, saturated $NaHCO_3$, 5% citric acid and brine. The resulting layers were separated and the organic layer was dried over $Na_2SO_4$, filtered and then concentrated under reduced pressure to provide a foam.

This foam was purified using radial chromatography (1.0 mm plate, gradient eluent of 0-10% MeOH in $CH_2Cl_2$). Yield: 487 mg (55%).

$[\alpha]_D$ +11.93° (*c* 0.10, MeOH).

$^1H$ NMR ($CDCl_3$) : δ 1.46 (s, 9H); 2.71 (dd, J=15,6 Hz, 1H); 2.81-3.01 (m, 4H); 3.07 (dd, J=15,3 Hz, 1H); 3.75-3.78 (m, 1H); 4.28-4.32 (m, 1H); 4.95 (dd, J=12, 6 Hz, 1H); 5.74 (br.s, 1H); 6.19 (br.s, 1H); 6.32 (br.s, 1H); 6.90 (t, J=6 Hz, 1H); 7,01 (t, J=6 Hz, 1H); 7.08-7.38 (m, 6H); 7.64 (t, J=6 Hz, 1H); 7.79 (t, J=9 Hz, 1H); 7.80 (d, J=6 Hz, 1H); 8.17-8.31 (m, 3H); 9.22 (d, J=9 Hz, 1H).

$^{13}C$ NMR (75.4 MHz, $CDCl_3$): δ 28.7, 35.3, 37.1, 37.2, 50.1, 52.1, 53.5, 55.9, 74.8, 118.7, 126.0, 127.0, 127.6, 128.2, 129.3, 129.4, 130.0, 130.2, 130.3, 130.9, 137.4, 137.5, 138.2, 146.5, 148.8, 164.6, 170.4, 170.5.

IR ($CHCl_3$): 3428, 3411, 3359, 30 12, 2975, 1681, 1601, 1565, 1518, 1499, 1454, 1428, 1395, 1367, 1231, 1047 $cm^{-1}$.

MS (FD): m/e 610 ($M^+$); 221(100).

| Analysis for $C_{35}H_{39}N_5O_5$: | | | |
|---|---|---|---|
| Calcd: | C, 68.95; | H, 6.45; | N, 11.49; |
| Found: | C, 68.76; | H, 6.55; | N, 11.49. |

Example 7

[1S-*(1R*,4R*,5S*)*]-N-[1-(2-Amino-2-oxoethyl)-2-oxo-3-aza-4-phenylmethyl-5-hydroxy-6-(2-(1-*t*-butylamino-1-oxomethyl)-4-methylphenyl)hexyl]-2-quinolinyl carboxamide

The titled compound was prepared substantially in accordance with the procedure detailed in Example 6 using the subtitled intermediate of [2R-(*2R*,3S**)]-N-*t*-butyl-2-(3-(N-benzyloxycarbonyl)amino-2-hydroxy-4-phenylbutyl)-5-methylbenzamide and (S)-2-N(quinolin-2-ylcarbonyl)amino-3-carbamoylpropanoic acid, HOBT·$H_2O$ and DCC. The resultant material was purified using flash chromatography (gradient eluent of 2-8% MeOH in $CH_2Cl_2$) to provide 2.00 g of crude material which was further purified using reverse phase HPLC. Yield: 1.95 g (74%).

$[a]_D$ +28.57° (*c* 0.10, MeOH).

$^1H$ NMR ($CDCl_3$): δ 1.43 (s, 9H); 2.30 (s, 3H); 2.63-3.05 (m, 6H); 3.65-3.76 (m, 1H); 4.22-4.35 (m, 1H); 4.89-4.97(m, 1H); 5.51 (s, 1H); 5.82-5.88 (br.s, 1H); 6.09 (s, 1H); 6.20 (s, 1H); 6.84-7.24 (m, 10H); 7.63 (t, J=7.5 Hz, 1H); 7.78 (t, J=7.5 Hz, 1H); 7.85 (d, J=8 Hz, 1H); 8.19 (t, J=8.4 Hz, 1H); 8.28 (d, J=8.4 Hz, 1H); 9.20 (d, J=8.1 Hz, 1H).

IR ($CHCl_3$): 3410, 3022, 3013, 1674, 1519, 1497, 1454, 1428, 1367, 1210, 1047, 910 $cm^{-1}$.

MS (FD): m/e 624($M^+$); 234(100).

| Analysis for $C_{36}H_{41}N_5O_5$: | | | |
|---|---|---|---|
| Calcd: | C, 69.32; | H, 6.63; | N, 11.23; |
| Found: | C, 68.73; | H, 6.76; | N, 11.07. |

Example 8

A. [2R-*(2R*,3S*,6S*)*]-N-*t*-Butyl-2-(2-hydroxy-3-phenylmethyl-4-aza-5-oxo-6-N(benzyloxycarbonyl)amino)-7-carbamoylheptyl) benzamide

To a cold (0°C) solution containing 3.4 g (10 mmol) of the subtitled intermediate from Preparation 3D, 2.6 g (10 mmol) of 2-N(benzyloxycarbonyl)amino-3-carbamoyl propanoic acid, 1.09 mL (10 mmol) of NMM and 1.48 g (11 mmol) of HOBT·$H_2O$ in 20 mL of anhydrous DMF, was added 2.4 mg (11 mmol) of DCC, under $N_2$. The reaction mixture was warmed to room temperature and allowed to react overnight. The resulting reaction mixture was filtered and the desired subtitled compound was extracted from the filtrate using EtOAc. The EtOAc solution was diluted with an EtOAc/$H_2O$ mixture. The resulting layers were separated and the organic layer was washed sequentially with saturated $NaHCO_3$, 5% citric acid, saturated $NaHCO_3$ and brine solutions, dried over $MgSO_4$, filtered and then reduced to dryness under reduced pressure to provide a residue. This residue was then rinsed with an $Et_2O$/hexane mixture and then filter dried in a vacuum desiccator.

Yield: 4.4 g (79%).

B. [2R-(*2R*,3S*,6S**)]-N-*t*-Butyl-2-(2-hydroxy-3-phenylmethyl-4-aza-5-oxo-6-amino-7-carbamoylheptyl) benzamide

The subtitled compound was prepared substantially in accordance with the procedure detailed in Preparation 3D, using the subtitled compound of Example 8A and Pd/C to provide 31.1 g (96%) of a pale yellow foam.

C. [IS-(*1R*,4R*,5S**)]-N-(1-(2-amino-2-oxoethyl)-2-oxo-3-aza-4-phenylmethyl-5-hydroxy-6-(2-(1-*t*-butylamino-1-oxomethyl)phenyl)hexyl)-7-methylquinolin-2-yl carboxamide

To a solution containing 135 mg (0.5 mmol) of 2-carboxy-7-methyl quinoline and 81 mg (0.5 mmol) of 1,1-carbonyldiimidazole in 15 mL of anhydrous $CH_2Cl_2$, was added 227 mg (0.5 mmol) of the subtitled compound of Example 8B. The resulting reaction mixture was allowed to react overnight. When the reaction was substantially complete, as determined by TLC, the reaction mixture was diluted with 100 mL of $CH_2Cl_2$ and 25 mL of $H_2O$ to provide an emulsion. This emulsion was washed sequentially with saturated $NaHCO_3$, 5% citric acid, saturated $NaHCO_3$ and brine solutions. The resulting mixture was dried over $MgSO_4$ and then reduced to dryness under reduced pressure to provide a residue. This residue was slurried with $Et_2O$, isolated by filtration and then dried in a vacuum desiccator at room temperature to provide 115 mg (37%) of the desired subtitled compound.

MS (FAB): 623($M^{+1}$).

$^1$H NMR ($CDCl_3$): δ 1.46 (s, 9H); 2.6-3.1 (m, 10H); 3.69-3.75 (m, 1H); 4.22-4.32 (m,IH); 4.28-4.35 (m, 1H); 4.9 (m, 1H); 5.3-5.35 (m, 1H); 5.9-6.05, (2 br.s, 2H); 6.89-8.3 (m, 14H); 9.3 (d, 1H).

The following Examples 9 and 10 were prepared substantially in accordance with the procedure detailed in Example 8C, using the subtitled compound of Example 8B and the designated carboxy-substituted reagent.

## Example 9

[1S-(*1R*,4R*,5S**)]-N-(1-(2-Amino-2-oxoethyl)-2-oxo-3-aza-4-phenylmethyl-5-hydroxy-6-(2-(1-*t*-butylamino-1-oxomethyl)phenyl)hexyl)-8-methylquinolin-2-yl carboxamide

The titled compound was prepared using 2-carboxy-8-methyl quinoline.

Yield: 57.8%

MS (FAB): $M^{+1}$ (623+1).

$^1$H NMR ($CDCl_3$): δ 1.46 (s, 9H); 2.6-3.1 (m, 8H); 3.70-3.78 (m, 1H); 4.28-4.32 (m, 1H); 4.9 (m, 1H); 5.2 (br.s, 1H); 6.05, (br.s, 2H); 6.8-7.78, (m, 12H); 8.2-8.3 (dd, 2H).

| Analysis for $C_{36}H_{40}N_5O_5$: | | | |
|---|---|---|---|
| Calcd: | C, 69.32; | H, 6.62; | N, 11.23; |
| Found: | C, 69.10; | H, 6.81; | N, 10.94. |

## Example 10

[1S-(*1R*,4R*,5S**)]-N-(1-(2-Amino-2-oxoethyl)-2-oxo-3-aza-4-phenylmethyl-5-hydroxy-6-(2-(1-*t*-butylamino-1-oxomethyl)phenyl)hexyl)-4-methylquinolin-2-yl carboxamide

The titled compound was prepared using 2-carboxy-4-methyl quinoline.

Yield: 35%.

MS (FAB): 623($M^{+1}$).

$^1$H NMR ($CDCl_3$): δ 1.46 (s, 9H); 2.6-3.1 (m, 10H); 3.7-3.78 (m, 1H); 4.23-4.35 (m, 1H); 4.9-4.95 (m, 1H); 5.35-5.40 (br.s, 1H); 5.97-6.08 (2 br.s, 3H); 6.82-8.2 (m, 14H); 9.18-9.2 (d, 1H).

| Analysis for $C_{36}H_{40}N_5O_5$: | | | |
|---|---|---|---|
| Calcd: | C, 69.32; | H, 6.62; | N, 11.23; |
| Found: | C, 69.06; | H, 6.64; | N, 11.25. |

The following Examples 11-15 were prepared substantially in accordance with the procedure detailed in Example 6, using HOBT·$H_2O$ and DCC.

Example 11

[1S-(*1R**,*4R**,*5S**)]-N-(1-(2-Amino-2-oxoethyl)-2-oxo-3-aza-4-phenylmethyl-5-hydroxy-6-(2-(1-*t*-butylamino-1-oxomethyl)phenyl)hexyl)-2-(6,7,8,9-tetrahydronaphthyl) carboxamide

Yield: 75 mg (54%) of a colorless foam.
$^1H$ NMR ($CDCl_3$): δ 8.0 (d, 1H); 7.5-7.03 (m, 12H); 6.51 (br.s, 1H); 6.22 (br.s, 1H); 5.57 (br.s, 1H); 4.84 (m, 1H); 4.23 (m, 1H); 3.75 (m, 1H); 3.12-2.5 (m, 10H); 1.81 (m, 4H); 1.43 (s, 9H).
MS (FD): 629($M^{+1}$).

| Analysis for $C_{37}H_{36}N_4O_5$: | | | |
|---|---|---|---|
| Calcd: | C, 70.79; | H, 7.55; | N, 8.92; |
| Found: | C, 70.63; | H, 7.31; | N, 9.21. |

Example 12

[1S-(*1R**,*4R**,*5S**)]-N-(1-Isopropyl-2-oxo-3-aza-4-phenylmethyl-5-hydroxy-6-(2-(1-*t*-butylamino-1-oxomethyl)phenyl)hexyl)-2-quinoxalinyl acetamide

The desired titled compound was using the subtitled intermediates from Preparations 3D and 6 to provide 51 mg (8%) of a yellowish foam.
$^1H$ NMR ($CDCl_3$): δ 9.69 (s, 1H); 8.31 (d, 1H); 8.28-7.0 (m, 13H); 6.78 (m, 2H); 6.12 (m, 2H); 4.43 (m, 2H); 3.82 (m, 1H); 1.5 (s, 9H); 1.0 (dd, 6H).
MS (FD): 597($M^{+1}$).

Example 13

[1S-(1*R**,*4R**,*5S**)]-N-(1-Isopropyl-2-oxo-3-aza-4-phenylmethyl-5-hydroxy-6-(2-(1-*t*-butylamino-1-oxomethyl)phenyl)hexyl)-2-naphthyl acetamide

The titled compound was prepared using the subtitled intermediates from Preparations 3D and 7 to provide 9.6 mg (11%) of a tan foam.
$^1H$ NMR ($CDCl_3$): δ 8.22 (s, 1H); 7.93-6.7 (m, 15H); 6.1 (d, 1H); 6.0 (s, 1H); 4.49 (t, 1H); 4.35 (m, 1H); 3.82 (m, 1H); 3.09-2.77 (m, 4H); 2.2 (m, 1H); 1.47 (s, 9H); 0.98 (t, 6H).
MS (FD): 594($M^{+1}$).

Example 14

[1S-(*1R**,*4R**,*5S**)]-N-(1-(2-Amino-2-oxoethyl)-2-oxo-3-aza-4-phenylmethyl-5-hydroxy-6-(2-(1-*t*-butylamino-1-oxomethyl)thien-3-yl)hexyl)-2-quinolinyl carboxamide

Yield: 90 mg (50%) of a white solid.
$^1H$ NMR ($CDCl_3$): δ 1.42 (s, 9H); 2.75 (dd, J=7 Hz, 1H); 2.95-3.20 (m, 5H); 3.77 (m, 1H); 4.27 (m, 1H); 4.95 (m, 1H); 5.38 (br.s, 1H); 5.93 (br.s, 1H); 6.41 (br.s, 1H); 6.95 (m, 5H); 7.20 (m, 4H); 7.66 (t, J=8 Hz, 1H); 7.81 (t, J=8 Hz, 1H); 7.90 (d, J=8 Hz, 1H); 8.20 (m, 2H); 8.34 (d, J=8 Hz, 1H); 9.25 (d, J=8 Hz, 1H).
IR (KBr): 3302, 1667, 1498, 1427 $cm^{-1}$.
MS (FAB): mass 616.2591 (M+H).
MS (FD): m/e 615($M^+$).

| Analysis for $C_{33}H_{37}N_5O_5S$: | | | |
|---|---|---|---|
| Calcd: | C, 64.37; | H, 6.06; | N, 11.37; |
| Found: | C, 64.60; | H, 5.98; | N, 11.42. |

Example 15

[1S-(*1R**,*R**,*5S**)]-N-(1-(2-Hydroxy-2-oxoethyl)-2-oxo-3-aza-4-phenylmethyl-5-hydroxy-6-(2-(1-*t*-butylamino-1-oxomethyl)phenyl)hexyl)-2-quinolinyl carboxamide

The desired titled compound was prepared using the subtitled intermediates from Preparations 3D and 8 to provide 40 mg (40%) of a colorless solid.
$^1$H NMR (DMSO-$d_6$): δ 1.38 (s, 9H); 2.58-3.05 (m, 6H); 3.61 (br.s, 1H); 3.84 (m, 1H); 4.79 (m, 1H); 5.88 (s, 1H); 6.92 (t, J=8 Hz, 1H); 7.02 (t, J=8 Hz, 2H); 7.12-7.37 (m, 6H); 7.75 (t, J=10 Hz, 2H); 7.90 (t, J=10 Hz, 2H); 7.98 (d, J=10 Hz, 2H); 8.07-8.23 (m, 4H); 8.60 (d, J=10 Hz, 1H); 8.89 (d, J=10 Hz, 1H); 12.40 (s, 1H).
MS (FD): m/e 613(25), 612(95), 611(100).

| Analysis for $C_{35}H_{38}N_4O_6$: | | | |
|---|---|---|---|
| Calcd: | C, 68.84; | H, 6.27; | N, 9.17; |
| Found: | C, 68.61; | H, 6.44; | N, 9.10. |

The following Examples 16-19 were prepared substantially in accordance with the procedure detailed in Example 8C, using 1,1-carbonyldiimidazole.

Example 16

[1S-(*1R**,*4R**,*5S**)]-N-(1-(2-(N-2-Pyridylmethyl)amino)-2-oxoethyl]-2-oxo-3-aza-4-phenylmethyl-5-hydroxy-6-(2-(1-*t*-butylamino-1-oxomethyl)phenyl)hexyl)-2-quinolinyl carboxamide

The titled compound was prepared using 0.2 g (0.33 mmol) of the titled compound of Example 15 and 0.07 mL (0.66 mmol) of 2-aminomethylpyridine.
Yield: 33 mg (14%).
$^1$H NMR ($CDCl_3$): δ 1.48 (s, 9H); 2.77-3.16 (m, 6H); 3.80 (br.s, 1H); 4.33 (m, 1H); 4.59 (m, 2H); 5.02 (m, 1H); 5.90 (br.s, 1H); 6.23 (s, 1H); 6.90-7.09 (m, 4H); 7.15 (m, 1H); 7.20-7.42 (m, 8H); 7.57-7.70 (m, 2H); 7.82 (t, J=8 Hz, 1H); 7.91 (d, J=8 Hz, 1H); 8.18-8.40 (m, 4H); 9.35 (d, J=8 Hz, 1H).
MS (FD): m/e 701(100), 700(15), 481(28), 220(90).

| Analysis for $C_{41}H_{44}N_6O_5 \cdot 2.2H_2O$: | | | |
|---|---|---|---|
| Calcd: | C, 66.50; | H, 6.56; | N, 11.40; |
| Found: | C, 66.35; | H, 6.20; | N, 11.00. |

Example 17

[1*S*-(*1R**,*4R**,*5S**)]-N-(1-(2-(N-2-Imidazolylmethyl)amino)-2-oxoethyl)-2-oxo-3-aza-4-phenylmethyl-5-hydroxy-6-(2-(1-*t*-butylamino-1-oxomethyl)phenyl)hexyl)-2-quinolinyl carboxamide

The desired titled compound was prepared using 0.50 g (0.82 mmol) of the titled compound of Example 15 and 0.28 g (1.64 mmol) of imidazolemethylamine dihydrochloride, substituting HOBT·$H_2O$ and DCC (for 1,1-carbonyldiimidazole) to provide 0.26 g (46%) of a colorless solid.
$^1$H NMR (DMSO-$d_6$): δ 1.33 (s, 9H); 2.69 (m, 5H); 2.95 (dd, J=30 Hz, 14, 2H); 3.62 (br.s, 1H); 3.88 (br.s, 1H); 4.22 (d, J=10 Hz, 2H); 4.80 (br.s, 1H); 6.82 (br.s, 1H); 6.87 (t, J=8 Hz, 1H); 7.05 (t, J=8 Hz, 2H); 7.15-7.25 (m, 4H); 7.30 (t, J=7 Hz, 2H); 7.74 (t, J=7 Hz, 1H); 7.89 (t, J=9 Hz, 1H); 7.98-8.14 (m, 6H); 8.45 (t, J=6 Hz, 1H); 8.58 (d, J=9 Hz, 1H); 8.96 (d, J=9 Hz, 1H).
MS (FD): m/e 691(80), 690(100).

| Analysis for $C_{39}H_{43}N_7O_5$: | | | |
|---|---|---|---|
| Calcd: | C, 67.91; | H, 6.28; | N, 14.21; |
| Found: | C, 67.67; | H, 6.50; | N, 13.98. |

Example 18

[1S-(*1R**,*4R**,*5S**)]-N-(1-(2-(N-2-Benzimidazolylmethyl)amino)-2-oxoethyl)-2-oxo-3-aza-4-phenylmethyl-5-hydroxy-6-(2-(1-*t*-butylamino-1-oxomethyl)phenyl)hexyl)-2-quinolinyl carboxamide
Yield: 0.199 g (82%) of a yellow foam.

$^1H$ NMR (DMSO-$d_6$): δ 1.34 (s, 9H); 2.63-3.30 (m, 5H); 3.60-3.65 (m, 1H); 3.88-3.91 (m, 1H); 4.43 (d, J=5.6 Hz, 2H); 4.84 (dd, J=7.8, 6.0 Hz, 1H); 5.87 (d, J=5.3 Hz, 1H); 6.92-7.47 (m, 14H); 7.69-7.75 (m, 1H); 7.85 (t, J=7.7 Hz, 1H); 8.03-8.20 (m, 6H); 8.57 (d, J=8.6 Hz, 1H); 8.65 (m, 1H); 9.00 (d, J=8.2 Hz, 1H).
MS (FD): m/e 743($M^{+1}$); 740(29); 739(50); 354(100).

| Analysis for $C_{43}H_{45}N_7O_5 \cdot 0.4H_2O$: | | | |
|---|---|---|---|
| Calcd: | C, 69.13; | H, 6.18; | N, 13.12; |
| Found: | C, 68.84; | H, 6.35; | N, 13.50. |

Example 19

[1S-(*1R**,*4R**,*5S**)]-N-(1-(2-Amino-2-oxoethyl)-2-oxo-3-aza-4-phenylmethyl-5-hydroxy-6-(2-(1-*t*-butylamino-1-oxomethyl)phenyl)hexyl)-4-chloroquinolin-2-yl carboxamide
Yield: 29.2 mg (9%).
$^1H$ NMR ($CDCl_3$): δ 1.45 (s, 9H); 2.7-3.1 (m, 6H); 3.7-3.8 (m, 1H); 4.3-4.4 (m, 1H); 4.9-5.0 (m, 1H); 5.5 (m, 1H); 6.0 (m, 2H); 6.9-8.3 (m, 17H); 9.2 (d, 1H).
MS (FD): 644.

| Analysis for $C_{35}H_{38}N_5O_5Cl$: | | | |
|---|---|---|---|
| Calcd: | C, 65.26; | H, 5.95; | N, 10.87; |
| Found: | C, 65.55; | H, 6.04; | N, 10.85. |

Examples 20-22 were prepared substantially in accordance with the procedure detailed in Example 6, above.

Example 20

[1S-(*1R**,*4R**,*5S**)]-N-(1-(2-Methylpropyl)-2-oxo-3-aza-4-phenylmethyl-5-hydroxy-6-(2-(1-*t*-butylamino-1-oxomethyl)phenyl)hexyl)-quinolin-2-yl carboxamide
Yield: 44 mg (61%).

Example 21

[1S-(*1R**,*4R**,*5S**)]-N-(1-(Propyl)-2-oxo-3-aza-4-phenylmethyl-5-hydroxy-6-(2-(1-*t*-butylamino-1-oxomethyl)phenyl)hexyl)-quinolin-2-yl carboxamide
Yield: 42 mg (22%).
MS(FD): m/e 598($M^{+4}$).

Example 22

[1S-(*1R**,*4R**,*5S**)]-N-(1-(2-Amino-2-oxoethyl)-2-oxo-3-aza-4-phenylmethyl-5-hydroxy-6-(2-(1-*t*-butylamino-1-oxomethyl)phenyl)hexyl)-8-trifluoromethyl-quinolin-2-yl carboxamide
Yield: 116 mg (34%).
MS(FD): m/e 678($M^{+1}$).

Example 23

A. [2R-(*2R**,*3S**,*6S**)]-N-*t*-Butyl-2-[2-hydroxy-3-phenylmethyl-4-aza-5-oxo-6-N(*t*-butoxycarbonyl)amino-7-methyl]octyl benzamide

The subtitled compound was prepared substantially in accordance with the procedure detailed in Example 6, using 1.34 g (6.18 mmol) of (S)-2-N-(*t*-butoxycarbonyl)amino-3-methylbutanoic acid and 2.00 (5.88 mmol) of the subtitled intermediate of Preparation 3D to provide 3.95 g of a colorless foam. Half of this foam was purified using column chromatography ($SiO_2$; eluent of 3% MeOH in $CHCl_3$) to provide 1.45 g of the desired subtitled compound. The other half of this foam was reacted without further purification

in Example 56B, below.

| Analysis for $C_{31}H_{45}N_3O_5$: | | | |
|---|---|---|---|
| Calcd: | C, 69.12; | H, 8.23; | N, 7.80; |
| Found: | C, 69.38; | H, 8.43; | N, 7.96. |

B. [2R-*(2R*,3S*,6S*)*]-N-*t*-Butyl-2-[2-hydroxy-3-phenylmethyl-4-aza-5-oxo-6-amino-7-methyl]octyl benzamide

A solution of 2.0 g (max. 3.7 mmol) of the subtitled compound of Example 23A and 0.713 g (3.7 mmol) of toluenesulfonylhydroxide hydrate (TosOH·$H_2O$) in 75 mL of absolute EtOH was heated to 55°C. When the reaction was substantially complete, as indicated by TLC, the reaction mixture was cooled to room temperature and concentrated under reduced pressure to provide a residue. This residue was distributed between 100 mL of EtOAc and 50 mL of a 10% $NH_4OH$. The resulting layers were separated and the organic layer was washed with brine, dried over $MgSO_4$, filtered and then reduced to dryness under reduced pressure to provide 1.0 g of a white foam. This foam was purified using flash chromatography ($SiO_2$, gradient eluent of 2-5% MeoH in $CHCl_3$) to provide 0.97 of the desired subtitled compound.

| Analysis for $C_{26}H_{37}N_3O_3$: | | | |
|---|---|---|---|
| Calcd: | C, 71.04; | H, 8.48; | N, 9.56; |
| Found: | C, 70.85; | H, 8.59; | N, 9.56. |

C. [2R-*(2R*,3S*,6S*,9S*)*]-N-*t*-Butyl-2-[2-hydroxy-3-phenylmethyl-4,7-diaza-5,8-dioxo-6-(1-methylethyl)-9-N(*t*-butoxycarbonyl)amino-10-naphth-1-yl]decyl benzamide

The desired subtitled compound was prepared substantially in accordance with the procedure detailed in Example 6, using 226 mg (0.717 mmol) of (S)-2-N(*t*-butoxycarbonyl)amino-3-naphth-1-ylpropanoic acid and 300 mg (0.683 mmol) of the subtitled intermediate of Example 23B to provide 490 mg (98%) of an off-white foam.

MS (FD): m/e 737($M^+$, 100).

| Analysis for $C_{44}H_{56}N_4O_6$: | | | |
|---|---|---|---|
| Calcd: | C, 71.71; | H, 7.66; | N, 7.60; |
| Found: | C, 71.82; | H, 7.73; | N, 7.55. |

## Example 24

[2R-*(2R*,3S*,6S*,9R*)*]-N-t-Butyl-2-[2-hydroxy-3-phenylmethyl-4,7-diaza-5,8-dioxo-6-(1-methylethyl)-9-N(t-butoxycarbonyl)amino-10-naphth-1-yl]decyl benzamide

The desired titled compound was prepared substantially in accordance with the procedure detailed in Example 6, using 226 mg (0.717 mmol) of (R)-2-N(*t*-butoxycarbonyl)amino-3-naphth-1-ylpropanoic acid and 300 mg (0.683 mmol) of the subtitled intermediate of Example 23B to provide 490 mg (98%) of an off-white foam.

MS (FD): m/e 737($M^+$, 100).

| Analysis for $C_{44}H_{56}N_4O_6$: | | | |
|---|---|---|---|
| Calcd: | C, 71.71; | H, 7.66; | N, 7.60; |
| Found: | C, 71.41; | H, 7.90; | N, 7.68. |

## Example 25

A. [2R-*(2R*,3S*,6S*)*]-N-*t*-Butyl-2-[2-hydroxy-3-phenylmethyl-4-aza-5-oxo-6-N(benzyloxycarbonyl)amino-7-carbamoyl]heptyl benzamide

The desired subtitled compound was prepared substantially in accordance with the procedure detailed

in Example 6, using 2.6 g (10 mmol) of (S)-2-N(benzyloxycarbonyl)amino-3-carbamoylpropanoic acid and 3.4 g (10 mmol) of the subtitled intermediate of Preparation 3D, with the exception that 1.09 mL (10 mmol) of NMM was added to the reaction mixture, to provide 4.4 g (76%) of the desired subtitled compound.

B. [2R-*(2R**,*3S**,*6S**)]-N-*t*-Butyl-2-[2-hydroxy-3-phenylmethyl-4-aza-5-oxo-6-amino-7-carbamoyl]heptyl benzamide

The desired subtitled compound was prepared substantially in accordance with the procedure detailed in Preparation 3D, using 4 g (6.7 mmol) of the subtitled intermediate of Example 25A and 0.5 g of 5% Pd/C.
Yield: 2.4 g (80%).

C. [2R-*(2R**,*3S**,*6S**,*9R**)]-N-*t*-Butyl-2-[2-hydroxy-3-phenylmethyl-4,7-diaza-5,8-dioxo-6-(2-amino-2-oxoethyl)-9-N(*t*-butoxycarbonyl)amino-10-naphth-1-yl]decyl benzamide

The desired subtitled compound was prepared substantially in accordance with the procedure detailed in Example 6, using 630 mg (2.0 mmol) of (R)-2-N(*t*-butoxycarbonyl)amino-3-naphth-1-ylpropanoic acid and 908 mg (2.0 mmol) of the subtitled compound of Example 25B.
Yield: 920 mg (60%).
MS (FD): m/e 751($M^+$, 100).

| Analysis for $C_{43}H_{53}N_5O_7$: | | | |
|---|---|---|---|
| Calcd: | C, 68.69; | H, 7.10; | N, 9.31; |
| Found: | C, 68.66; | H, 7.22; | N, 9.27. |

Example 26

[2R-*(2R**,*3S**,*6S**,*9S**)]-N-*t*-Butyl-2-[2-hydroxy-3-phenylmethyl-4,7-diaza-5,8-dioxo-6-(2-amino-2-oxoethyl)-9-N(*t*-butoxycarbonyl)amino-10-naphth-1-yl]decyl benzamide

The desired titled compound was prepared substantially in accordance with the procedure detailed in Example 6, using 630 mg (2.0 mmol) of (S)-2-N(*t*-butoxycarbonyl)amino-3-naphth-1-ylpropanoic acid and 908 mg (2.0 mmol) of the subtitled compound of Example 25B.
Yield: 895 mg (60%).
MS (FD): m/e 751($M^+$, 100).

| Analysis for $C_{43}H_{53}N_5O_7$: | | | |
|---|---|---|---|
| Calcd: | C, 68.69; | H, 7.10; | N, 9.31; |
| Found: | C, 68.58; | H, 7.11; | N, 9.01. |

Example 27

A. [2R-*(2R**,*3S**,*6S**)]-N-*t*-Butyl-2-[2-hydroxy-3-phenylmethyl-4-aza-5-oxo-6-N(benzyloxycarbonyl)amino-7-imidazol-4-yl]heptyl benzamide

The desired subtitled compound was prepared substantially in accordance with the procedure detailed in Example 6, using 1.79 g (6.18 mmol) of (S)-2-N(benzyloxycarbonyl)amino-3-imidazol-4-ylpropanoic acid and 2.00 g (5.88 mmol) of the subtitled intermediate of Preparation 3D to provide an off-white foam which was used without further purification.
Yield: 3.46 g (96%).

| Analysis for $C_{35}H_{41}N_5O_5$: | | | |
|---|---|---|---|
| Calcd: | C, 68.72; | H, 6.76; | N, 11.45; |
| Found: | C, 65.60; | H, 6.61; | N, 10.94. |

B. [2R-*(2R**,*3S**,*6S**)]-N-*t*-Butyl-2-[2-hydroxy-3-phenylmethyl-4-aza-5-oxo-6-amino-7-imidazol-4-yl]heptyl benzamide

The desired subtitled compound was prepared substantially in accordance with the procedure detailed in Preparation 3D, using 1.9 g of 5% Pd/C and 3.36 g (5.5 mol) of the subtitled intermediate of Example

27A to provide a pale yellow foam.

| Analysis for $C_{27}H_{35}N_5O_3$: | | | |
|---|---|---|---|
| Calcd: | C, 67.90; | H, 7.39; | N, 14.66; |
| Found: | C, 67.94; | H, 7.50; | N, 14.36. |

MS: m/e 478($M^+$).

C. [2R-*(2R*,3S*,6S*,9S*)*]-N-*t*-Butyl-2-[2-hydroxy-3-phenylmethyl-4,7-diaza-5,8-dioxo-6-(imidazol-4-ylmethyl)-9-N(*t*-butoxycarbonyl)amino-10-naphth-1-yl]decyl benzamide

The desired subtitled compound was prepared substantially in accordance with the procedure detailed in Example 6, using 330 mg (1.05 mmol) of (S)-2-N(*t*-butoxycarbonyl)amino-3-naphth-1-ylpropanoic acid and 500 mg (1.05 mmol) of the subtitled compound of Example 27B.

Yield: 612 mg (76%).

MS (FD): m/e 774($M^+$, 100).

| Analysis for $C_{45}H_{54}N_6O_6$: | | | |
|---|---|---|---|
| Calcd: | C, 69.74; | H, 7.02; | N, 10.84; |
| Found: | C, 69.55; | H, 6.86; | N, 10.57. |

The following Examples 28-30 were prepared substantially in accordance with the procedure detailed in Example 6.

## Example 28

[2R-*(2R*,3S*,6S*,9R*)*]-N-*t*-Butyl-2-[2-hydroxy-3-phenylmethyl-4,7-diaza-5,8-dioxo-6-(imidazol-4-ylmethyl)-9-N(*t*-butoxycarbonyl)amino-10-naphth-1-yl]decyl benzamide

The desired titled compound was prepared using 330 mg (1.05 mmol) of (R)-2-N(*t*-butoxycarbonyl)amino-3-naphth-1-ylpropanoic acid and 500 mg (1.05 mmol) of the subtitled compound of Example 27B.

Yield: 612 mg (76%).

MS (FD): m/e 774($M^+$, 100).

| Analysis for $C_{45}H_{54}N_6O_6$: | | | |
|---|---|---|---|
| Calcd: | C, 69.74; | H, 7.02; | N, 10.84; |
| Found: | C, 69.75; | H, 6.98; | N, 10.88. |

## Example 29

[3'''S-(*3'''R*,4'''S**)]-N-[1'-Oxo-1'-(3"-[1'''-oxo-2'''-aza-3'''-phenylmethyl-4'''-hydroxy-5'''-(2'''-N-*t*-butylcarbamido)phenyl]pentyl-4"-ethyl)phenyl]methyl-1,2,3,4-tetrahydroisoquinoline

The titled compound was prepared using 300 mg (0.97 mmol) of the subtitled intermediate of Preparation 11 and 341 mg (1.18 mmol) of the subtitled intermediate of Preparation 3D.

Yield: 410 mg (67%) of an off-white foam.

$^1H$ NMR ($CDCl_3$): δ 1.07 (t, J=7.6 Hz, 3H); 1.45 (s, 9H); 2.54 (q, J=7.5 Hz, 2H); 2.70-3.30 (m, 5H); 3.55 (br.s, 1H); 3.91-3.95 (m, 1H); 4.40-4.65 (m, 2H); 4.83 (br.s, 1H); 5.97 (s, 1H); 6.32 (br.d, J=9.2 Hz, 1H); 6.90-7.60 (m, 16H).

IR ($CDCl_3$): 3009, 1645, 1514, 1455, 1236,1213 $cm^{-1}$.

MS (FD): m/e 632($M^+$, 100).

## Example 30

[3S-*(3R*,4aR*,8aR*,2'S*,3'R*)*]-2-[2'-Hydroxy-3'-phenylmethyl-4'-aza-5'-oxo-5'-(3"-[1''',2''',3''',4'''-tetrahydroisoquinolin-1'''-ylcarbonyl]-6"-methyl)phenyl]pentyl-decahydroisoquinoline-3-N-*t*-butylcarboxamide

The titled compound was prepared using 366 mg (1.24 mmol) of the subtitled intermediate of Preparation

10C and 500 mg (1.24 mmol) of the subtitled intermediate of Preparation 9B.
Yield: 489 mg (58%) of an off-white foam.
$[a]_D$ -69.1017° (*c* 1.013, MeOH).
$^1$H NMR ($CDCl_3$): δ 1.13 (s, 9H); 1.20-2.10 (m, 14H); 2.20-2.35 (m, 4H); 2.50-3.05 (m, 7H); 3.37-3.60 (m, 2H); 3.80-4.10 (m, 2H); 4.40-4.70 (m, 2H); 4.85 (br.s, 1H); 5.67 (br.s, 1H); 6.72 (br.d, J = 8.4Hz, 1H); 6.95-7.34 (m, 12H). IR (KBr): 3600-3150 (br.); 3009, 1627, 1514, 1455, 1247, 1047 $cm^{-1}$.
MS (FD): m/e 679($M^+$), 578(100).

| Analysis for $C_{42}H_{54}N_4O_4$: | | | |
|---|---|---|---|
| Calcd: | C, 74.30; | H, 8.02; | N, 8.25; |
| Found: | C, 74.07; | H, 8.00; | N, 8.22. |

Example 31

A. [2R-(*2R**,*3S**,*6R**)]-N-*t*-Butyl-2-[2-hydroxy-3-phenylmethyl-4-aza-5-oxo-6-N(*t*-butoxycarbonyl)amino-7-benzyloxycarbonyl]heptyl benzamide

The subtitled compound was prepared substantially in accordance with the procedure detailed in Example 6, using 1.0 g (2.9 mmol) of the subtitled intermediate of Preparation 3D and 0.95 g (2.9 mmol) of (2R)-2-N(*t*-butoxycarbonyl)amino-4-oxo-4-benzyloxybutanoic acid to provide 1.9 g (87%) of a white solid (m.p 67-72°C).

| Analysis for $C_{37}H_{47}N_3O_7$: | | | |
|---|---|---|---|
| Calcd: | C, 68.82; | H, 7.34; | N, 6.51; |
| Found: | C, 69.16; | H, 7.50; | N, 6.56. |

B. [2R-(*2R**,*3S**,*6R**)]-N-*t*-Butyl-2-[2-hydroxy-3-phenylmethyl-4-aza-5-oxo-6-amino-7-benzyloxycarbonyl]heptyl benzamide

To a cold (0°C) solution of 2.89 g (5.53 mmol) of the subtitled compound of Example 31A in $CH_2Cl_2$, was added trifluoroacetic acid ($CF_3COOH$). The resulting reaction mixture was stirred for approximately one hour and then concentrated under reduced pressure to provide a foam. This foam was slurried in toluene and then concentrated under reduced pressure to provide 2.4 g of crude material which was used without further purification.

C. [2R-(*2R**,*3S**,*6R**)]-N-*t*-Butyl-2-[2-hydroxy-3-phenylmethyl-4-aza-5-oxo-6-N(methylsulfonyl)amino-7-benzyloxycarbonyl]heptyl benzamide

To a solution containing 0.75 g (0.97 mmol) of the subtitled compound of Example 31B and $Et_3N$ in $CH_2Cl_2$, was added 75.1 μL (0.970 mmol) of methanesulfonylchloride, under $N_2$. When the reaction was substantially complete, as indicated by TLC, the reaction mixture was reduced to dryness under reduced pressure to provide a residue.
Yield: 0.33 g (54%) of a white solid.
$^1$H NMR ($CDCl_3$): δ 1.45 (s, 9H); 2.42 (dd, J=6.12 Hz, 1H); 2.76 (s, 3H); 2.63-3.10 (m, 5H); 3.80 (m, 1H); 4.13 (m, 1H); 4.38 (m, 1H); 5.05 (s, 2H); 5.80 (d, J=8 Hz, 1H); 5.99 (d, J=6 Hz, 1H); 6.10 (s, 1H); 7.06-7.41 (m, 15H).
MS (FD): m/e 623($M^+$).
IR ($CHCl_3$): 3026, 1660, 1643, 1602, 1517 $cm^{-1}$.
UV (EtOH): 203nm (E=44,949).

| Analysis for $C_{33}H_{41}N_3O_7S$: | | | |
|---|---|---|---|
| Calcd: | C, 63.54; | H, 6.62; | N, 6.74; |
| Found: | C, 63.73; | H, 6.56; | N, 6.52. |

D. [2R-(*2R**,*3S**,6*R**)]-N-*t*-Butyl-2-[2-hydroxy-3-phenylmethyl-4-aza-5-oxo-6-N(methylsulfonyl)amino-7-carboxy]heptyl benzamide

To a solution of 0.13 g (0.21 mmol) of the subtitled compound of Example 31C in MeOH, were added

0.2 g (3.2 mmol) of ammonium formate and 50 mg of 5% Pd/C. The reaction mixture was allowed to react at reflux temperature for approximately 2 hours. When the reaction was complete, as determined by TLC, the reaction mixture was cooled, diluted with EtOAc and then filtered. The solution was then combined with 50 mL of $H_2O$, the resulting layers were then separated and the organic layer was reduced to dryness under reduced pressure.
Yield: 0.10 g (91%) of a white solid.

Example 32

A. [2*R*-(2*R**,3*S**,6*S**)]-N-*t*-Butyl-2-[2-hydroxy-3-phenylmethyl-4-aza-5-oxo-6-amino-7-carbamoyl]heptyl benzamide

The desired subtitled compound was prepared substantially in accordance with the procedure detailed in Preparation 3D, using 5% Pd/C and 4 g (6.7 mmol) of the subtitled intermediate of Example 25A.
Yield: 2.4 g (80%) of a solid.
MS (FAB): m/e 455 ($M^{+1}$).

B. [2*R*-(2*R**,3*S**,6*S**)]-N-*t*-Butyl-2-[2-hydroxy-3-phenylmethyl-4-aza-5-oxo-6-N(naphth-1-ylethylsulfonyl)amino-7-carbamoyl]heptyl benzamide

The desired subtitled compound was prepared substantially in accordance with the procedure detailed in Example 31C using 118 mg (0.462 mmol) of naphth-1-ylethylsulfonylchloride and 200 mg (0.440 mmol) of the subtitled compound of Example 32A (in the presence of NMM rather that $Et_3N$) to provide 126 mg (42%) of a white foam.
MS (FD): m/e 673 ($M^{+1}$).

| Analysis for $C_{37}H_{44}N_4O_6S$: | | | |
|---|---|---|---|
| Calcd: | C, 66.05; | H, 6.59; | N, 8.33; |
| Found: | C, 66.30; | H, 6.72; | N, 8.22. |

Example 33

1,6-Diphenyl-2(S),5(S)-di[N-[2(S)-N(benzyloxycarbonyl)amino-3-methyl-butanoyl]amino]-3,4-dihydroxyhexane

To a cold (0°C) solution of (S)-2-N(benzyloxycarbonyl)amino-3-methyl butanoic acid, 2-chloro-4,6-dimethoxy-1,3,5-triazene and 36 μL of NMM, was added a solution containing 0.142 mmol of the titled intermediate of Preparation 12 and 36 mL of NMM in $CH_2Cl_2$. The reaction mixture was warmed to room temperature and then reacted for approximately 5 hours. When the reaction was substantially complete, as indicated by TLC, the mixture was poured into 75 mL of a 2:1 EtOAc/0.2N HCl mixture. The resulting layers were separated and the organic layer was washed with half-dilute solution $NaHCO_3$, dried over $Na_2SO_4$, filtered and then concentrated under reduced pressure to provide a solid. This solid was purified using flash chromatography (silica; eluent of 30% hexane in EtOAc).
Yield: 44 mg (44%).
$^1H$ NMR ($CD_3OD$): δ 0.6 (d, J=6 Hz, 6H); 0.75 (d, J=6 Hz, 6H); 1.85 (m, 2H); 2.80 (m, 4H); 3.75 (m, 2H); 4.05 (m, 2H); 5.02 (s, 4H); 5.42 (d, J=8 Hz, 2H); 7.05 (m, 10H); 7.25 (m, 10H).
MS (FD): m/e 767 ($M^+$).

Example 34

1,6-Diphenyl-2(S),5(S)-di[N-[2(S)-N(quinolin-2-ylmethoxycarbonyl)amino-3-methylbutanoyl]amino]-3,4-dihydroxyhexane

The titled compound was prepared substantially in accordance with the procedure detailed in Example 33, using 102 mg (0.273 mmol) of the titled intermediate of Preparation 12, 184 mg (0.608 mmol) of (S)-2-N(quinolin-2-ylmethoxycarbonyl)-amino-3-methyl butanoic acid, 107 mg (0.608 mmol) of 2-chloro-4,6-dimethoxy-1,3,5-triazene and 69 μL (0.628 mmol) of NMM, with the exception that the reaction was run in $CH_2Cl_2$.
Yield: 155 mg (65%).
$^1H$ NMR ($CD_3OD$): δ 0.7 (m, 12H); 1.80 (m, 2H); 2.78 (m, 4H); 3.40 (m, 2H); 3.75 (d, J=6 Hz, 2H); 4.58 (m, 2H); 5.32 (s, 4H); 7.10 (m, 10H); 5.32 (s, 4H); 7.10 (m, 10H); 7.55 (m, 4H); 7.75 (t, J=6 Hz, 2H); 7.85 (d, J=6 Hz, 2H); 7.95 (d, J=6 Hz, 2H); 8.30 (d, J=6 Hz, 2H).

MS (FD): m/e 868 ($M^+$).

| Analysis for $C_{50}H_{56}N_6O_8$: | | | |
|---|---|---|---|
| Calcd: | C, 69.11; | H, 6.49; | N, 9.67; |
| Found: | C, 68.89; | H, 6.52; | N, 9.70. |

Example 35

1,6-Diphenyl-2(S),5(S)-di[N-[2(S)-N(pyrid-2-ylmethoxycarbonyl)amino-3-methylbutanoyl]amino]-3,4-dihydroxyhexane

The titled compound was prepared substantially in accordance with the procedure detailed in Example 34, using 72.7 mg (0.195 mmol) of the titled intermediate of Preparation 12, 110 mg (0.436 mmol) of (S)-2-N(pyrid-2-ylmethoxycarbonyl)amino-3-methyl butanoic acid, 76 mg (0.433 mmol) of 2-chloro-4,6-dimethoxy-1,3,5-triazene and 50 μL (0.455 mmol) of NMM.

Yield: 57.2 mg (38%).

$^1H$ NMR ($CD_3OD$): δ 0.72 (t, J=8 Hz, 12H); 1.84 (m, 2H); 2.81 (d, J=9 Hz, 4H); 3.41 (s, 2H); 3.76 (d, J=9 Hz, 2H); 4.55 (br.t, J=9 Hz, 2H); 5.17 (s, 4H); 7.00-7.40 (m, 12H); 7.45 (d, J=9 Hz, 2H); 7.84 (t, J=9 Hz, 2H); 8.48 (br.d, J=2 Hz, 2H).

IR ($CHCl_3$): 3428; 3025; 2973; 1723; 1667; 1602; 1507 $cm^{-1}$.

MS (FD): 770($M^{+1}$).

Example 36

Acetyl-β-naphthylalanine-valine-statine-leucine-β-naphthylalanine-amide ($CH_3C(O)$-bNA-Val-Sta-Leu-bNA-$NH_2$)

A. Preparation of Resin and Incorporation of *t*-Boc-Amino Acids

Before adding the *t*-Boc-amino acid, 0.45 g of *p*-methylbenzhydrylamine hydrochloride resin (containing 0.33 mmol of the free amino group) was neutralized three times with 10 mL of 5% N,N-diisopropylethylamine (DIEA) in $CH_2Cl_2$ (each wash), and then washed three times with 10 mL of $CH_2Cl_2$ (each wash). Then, 5 mL of a solution containing 2.5 equivalents of *t*-Boc-β-naphthylalanine (*t*-Boc-bNA) in $CH_2Cl_2$ was added to the washed resin. Next, a solution containing 2.5 equivalents of DCC in 1 mL of $CH_2Cl_2$ was added to the resin/*t*-Boc-amino acid mixture . The resulting reaction mixture was allowed to react at room temperature for one to three hours with occasional stirring. The coupling of the *t*-Boc-amino acid to the resin was tested using a ninhydrin test.

B. Removal of the *t*-Boc protecting group

Following the coupling reaction, the resin from Example 36A was washed three times with 10 mL of $CH_2Cl_2$ (each wash) and then combined with 15 mL of 50% $CF_3COOH$ in $CH_2Cl_2$. The resultant reaction mixture was allowed to react for approximately one minute and then the supernatant was removed from the resin and another 15 mL of 50% $CF_3COOH$ in $CH_2Cl_2$ was added. The resultant reaction mixture was allowed to react for approximately thirty minutes and then the supernatant was removed and the resin was again washed three times with 10 mL of $CH_2Cl_2$ (each wash). The ninhydrin test was used to monitor the reaction.

C. Neutralization

Before the next *t*-Boc-amino acid was coupled, the peptide-resin from Example 36B was neutralized with 15 mL of a 5% DIEA in $CH_2Cl_2$ solution. After approximately one minute, the supernatant was removed and a second 15 mL of 5% DIEA in $CH_2Cl_2$ was added. The resulting mixture was allowed to react for approximately five minutes and then the supernatant was removed and the peptide-resin was washed six times with $CH_2Cl_2$.

D. Sequential Coupling to Form Final Product

The next *t*-Boc-amino acid solution was added to the resin substantially in accordance with the procedure detailed in Example 36A, 36B and 36C, above. This procedure was sequentially repeated using the appropriate *t*-Boc-protected amino acids to provide the subtitled compound.

The only change in the procedure occurred during the *t*-Boc-statine coupling. When adding *t*-Boc-statine, 1.25 equivalents of the amino acid and 1.25 equivalents of the condensing reagent DCC were used. The coupling reaction for *t*-Boc-statine was allowed to run for 3 or more hours. Alternatively, larger excesses of *t*-Boc-statine or longer coupling times can be used.

E. Acetylation

To the neutralized peptide-resin from Example 36D, above, was added approximately 5 mL of acetic anhydride to cover the peptide-resin, followed by adding 1 mL of 5% DIEA in $CH_2Cl_2$. The resulting reaction mixture was allowed to react for approximately fifteen minutes. The reaction was substantially complete, as indicated by the ninhydrin test. A light yellow color resulted from the ninhydrin test indicating that the amino terminus of the peptide was protected by an acetyl group.

F. Cleavage of Peptide from Resin

The peptide was cleaved from the peptide resin by stirring in a solution of 10 parts (v/v) hydrofluoric acid (HF) containing 10% anisole to 1 part resin for 45 minutes at 0°C . After substantially all of the HF was removed using reduced pressure, the resulting mixture was washed with anhydrous $Et_2O$. The free peptide was extracted from the residue with glacial HOAc, 50% HOAc and then $H_2O$. The extracts were combined, lyophilized and then the titled peptide compound was purified using reverse-phase HPLC.

The peptide gave the following amino acid composition upon hydrolysis: Val, 1.0(1); Leu, 1.1(1); Sta, present; bNA, 1.8(2); which corresponds to the proper ratios for the titled compound. The peptide was also analyzed by MS(FAB) which confirmed the molecular weight (822) of the peptide.

Examples 37-40 were prepared substantially in accordance with the procedure detailed in Example 36.

| Example | Compound |
|---|---|
| 37 | $CH_3C(O)$-NA-Nva- Sta- Nva-NA-$NH_2$ |
| 38 | $CH_3C(O)$-NA-Nva-AHPPA-Nva-NA-$NH_2$ |
| 39 | $CH_3C(O)$-NA-Val- Sta -Abu-NA-$NH_2$ |
| 40 | NA-Val-AHPPA-Leu-NA-$NH_2$ |

The compounds prepared in Examples 36-40 were analyzed for amino acid composition upon hydrolysis and for molecular weight by MS(FAB). The results are set forth in Table 1, below.

Table 1
Amino acid composition and Molecular weight

| | | Amino Acid | Analysis | Molecular | Weight |
|---|---|---|---|---|---|
| Example | Residue | Theo. | Actual | Theo. | MH+ |
| 37 | NA | 2.00 | 2.14 | 808 | 809 |
| | Nva | 2.00 | 1.85 | | |
| | AHPPA | 1.00 | PRES | | |
| 38 | NA | 2.00 | 2.08 | 842 | 843 |
| | Nva | 2.00 | 1.92 | | |
| | Sta | 1.00 | PRES | | |
| 39 | Val | 1.00 | 1.01 | 794 | 795 |
| | Abu | 1.00 | 0.98 | | |
| | NA | 2.00 | 2.01 | | |
| | Sta | 1.00 | PRES | | |
| 40 | Val | 1.00 | 0.88 | 814 | 815 |
| | NA | 2.00 | 1.93 | | |
| | Leu | 1.00 | 1.12 | | |
| | AHPPA | 1.00 | PRES | | |

PRES - present

The following Examples 41-48 were prepared substantially in accordance with the procedure detailed in Example 36, using 0.136 mmol of an N-Boc-O-benzyl-L-threonine-4-(oxymethyl)-phenylacetamido methyl (Pam) resin, 5 mmol each of the appropriate *t*-Boc-amino acids and 1 mmol of *t*-Boc-statine. All coupling reactions were allowed to react for approximately one hour at room temperature.

With respect to the compounds prepared in Examples 42, 44, 46, 47, and 48, above, after the *t*-Boc-glycine was added, the protecting group was removed and neutralized substantially as detailed above in Example 36B-C, the resin was washed twice with 1% HOAc in DMF and then mixed with 3 mL of 50% acetone in DMF. To the resultant mixture, was then added a 1M solution of sodium cyanoborohydride in DMF. The resultant reaction mixture was allowed to react at room temperature for approximately one hour. After the reaction is complete, the supernatant was removed and the desired oligopeptide was cleaved from the resin substantially in accordance with the procedure detailed above in Reaction 36F. The reaction was monitored using the ninhydrin test. The reaction was complete when the resin turned reddish.

| Example | Compound |
|---|---|
| 41 | $CH_3C(O)$-Gly-Val-Val-Sta-Ala-Thr-OH |
| 42 | $(CH_3)_2CH$-Gly-Val-Val-Sta-Ala-Thr-OH |
| 43 | Gly-Val-Val-Sta-Ala-Thr-OH |
| 44 | $(CH_3)_2CH$-Gly-Sta-Ile-Ala-Thr-OH |
| 45 | Gly-Val-Sta-Ala-Thr-OH |
| 46 | $(CH_3)_2CH$-Gly-Val-Sta-Ala-Thr-OH |
| 47 | $(CH_3)_2CH$-Gly-Val-Val-Ile-Sta-Thr-OH |
| 48 | $(CH_3)_2CH$-Gly-Val-Sta-Ile-Ala-Thr-OH |

The compounds prepared in Examples 41-48 were analyzed for amino acid composition upon hydrolysis and for molecular weight by MS(FAB). The results are set forth in Table 2, below.

Table 2
Amino acid composition and Molecular weight

| Example | Residue | Amino Acid Theo. | Analysis Actual | Molecular Theo. | Weight Meas. |
|---|---|---|---|---|---|
| 41 | Gly | 1.0 | 1.066 | 644.7 | 645 |
| | Val | 2.0 | 1.529 | | |
| | Ala | 1.0 | 1.000 | | |
| | Thr | 1.0 | 0.957 | | |
| | Sta | 1.0 | PRES | | |
| 42 | Gly | 1.0 | PRES | 666.2 | 667 |
| | Val | 2.0 | 1.84 | | |
| | Ala | 1.0 | 1.15 | | |
| | Thr | 1.0 | 1.010 | | |
| | Sta | 1.0 | PRES | | |
| 43 | Gly | 1.0 | 1.000 | 602.6 | 603 |
| | Val | 2.0 | 1.78 | | |
| | Ala | 1.0 | 1.13 | | |
| | Thr | 1.0 | 0.990 | | |
| | Sta | 1.0 | PRES | | |
| 44 | Gly | 1.0 | PRES | 559.6 | 560 |
| | Ala | 1.0 | 1.000 | | |
| | Ile | 1.0 | 0.826 | | |
| | Thr | 1.0 | 0.967 | | |
| | Sta | 1.0 | PRES | | |
| 45 | Gly | 1.0 | 1.020 | 503.5 | 504 |
| | Val | 1.0 | 1.050 | | |
| | Ala | 1.0 | 1.020 | | |
| | Thr | 1.0 | 0.910 | | |
| | Sta | 1.0 | PRES | | |

Table 2 (continued)

| Example | Residue | Amino Acid Analysis Theo. | Actual | Molecular Weight Theo. | Meas. |
|---|---|---|---|---|---|
| 46 | Gly | 1.0 | PRES | 567.8 | 568 |
| | Val | 1.0 | 1.06 | | |
| | Ala | 1.0 | 1.03 | | |
| | Thr | 1.0 | 0.92 | | |
| | Sta | 1.0 | PRES | | |
| 47 | Gly | 1.0 | PRES | 686.8 | 687 |
| | Val | 1.0 | 1.609 | | |
| | Ile | 1.0 | 1.811 | | |
| | Thr | 1.0 | 1.000 | | |
| | Sta | 1.0 | PRES | | |
| 48 | Gly | 1.0 | PRES | 659 | 660 |
| | Val | 1.0 | 0.94 | | |
| | Ile | 1.0 | 1.21 | | |
| | Ala | 1.0 | 0.96 | | |
| | Thr | 1.0 | 0.90 | | |
| | Sta | 1.0 | PRES | | |

PRES - present

The following compounds were prepared substantially in accordance with the procedures detailed above. Specifically, the epoxides shown in Examples 49-74 were prepared using the procedure described in Example 1. The compounds shown in Examples 82-113, 140-145, 149-152, 170-206, 210, 212-214, 218-239, 242-243, 249-258 and 275 were prepared using the general procedures described in Example 6, 8, and 25A. The compounds shown in Examples 124-139 and 146 were generally prepared using the procedure described in Example 31. The compounds shown in Examples 114-123, 147, 148, 153, 154, 158-163 and 240-241 were generally prepared using the procedure described in Example 31, substituting acetyl chloride (or other appropriate acyl chloride) for methanesulfonylchloride. The compounds shown in Examples 155-157, 164-169, 207-209, 211 and 215-217 were generally prepared using the procedure described in Example 16. The compounds shown in Examples 244-248 and 274 were generally prepared using the procedures described in Examples 28 and 29, using intermediate compounds prepared according to the Preparation 10C. The compounds shown in Examples 259-262 were prepared using the procedure described in Example 33-35. Finally, the compounds shown in Examples 263-273 were prepared using the procedure described in Example 36.

Table 1

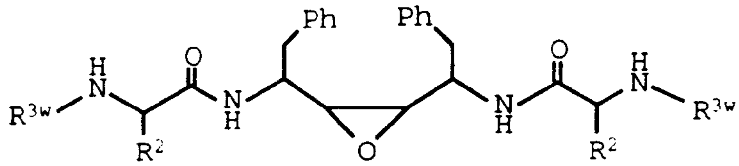

| Ex. | $R^{3w}$ | $R^2$ | Analytical Data |
|---|---|---|---|
| 49 | 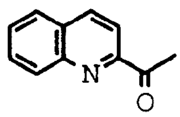 | $-CH_2CN$ | MS: m/e 785 ($M^+$+1) |
| 50 | 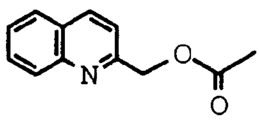 | $-CH(CH_3)_2$ | $^1$H NMR: δ 8.05 (m, 2); 7.72 (m, 2); 7.45 (m, 2); 7.10 (m, 10); 5.35 (s, 4); 4.15 (m, 2); 3.95 (m, 2); 3.10 (d, 1); 2.80 (m, 5); 2.0 (m, 2); 0.8 (m, 12) |
| 51 | 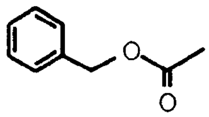 | $-CH_2C(O)NH_2$ | Yield: 7 mg (80-85%) |
| 52 | 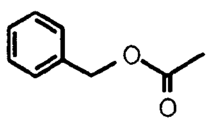 | $-CH_2CN$ | MS: m/e 743 ($M^+$+1) |
| 53 | 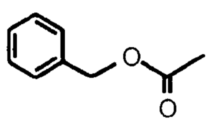 | 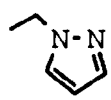 | |
| 54 | 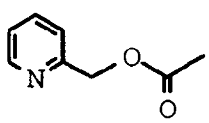 | $-CH(CH_3)_2$ | MS: m/e 751 ($M^+$+1) |
| 55 | 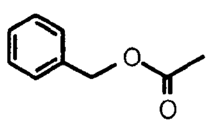 | 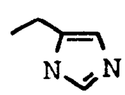 | $^1$H NMR: δ 7.50 (s, 2); 7.10 (m, 20); 6.68 (s, 2); 4.95 (s, 4); 4.05 (m, 4); 3.20 (m, 1); 2.60 (m, 8) |
| 56 | 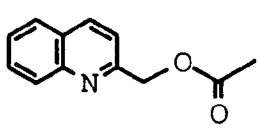 | $-CH_2CH_3$ | MS: m/e 823 ($M^+$+1) |

Table 1 (Cont'd)

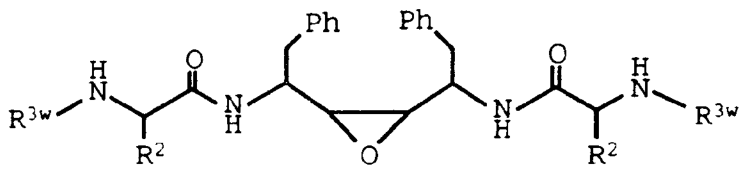

| Ex. | $R^{3w}$ | $R^2$ | PChem |
|---|---|---|---|
| 57 | 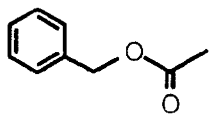 | $-CH_2SCH_3$ | MS: m/e 784 ($M^+$) |
| 58 | 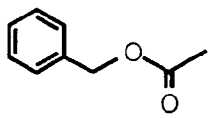 | $-CH_2CH_2C(O)NH_2$ | MS: m/e 807 ($M^++1$) |
| 59 | 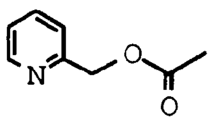 | $-CH_2CH(CH_3)_2$ | MS: m/e 778 ($M^+$) |
| 60 | 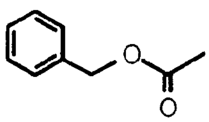 | $-CH(CH_3)CH_2CH_3$ | MS: m/e 777 ($M^++1$) |
| 61 | 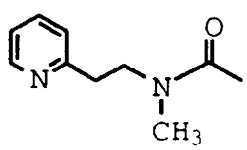 | $-CH(CH_3)_2$ | $^1H$ NMR: δ 8.42 (m, 2); 7.70 (m, 2); 7.20 (m, 14); 4.22 (m, 3); 4.0 (q, 1); 3.85 (d, 1); 3.6 (m, 4); 3.22 (m, 1); 2.95 (m, 4); 2.82 (s, 6); 2.80 (m, 4); 1.95 (m, 2); 0.8 (m, 12) |
| 62 | 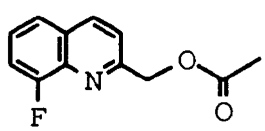 | $-CH(CH_3)_2$ | MS: m/e 888 ($M^++2$) |
| 63 | 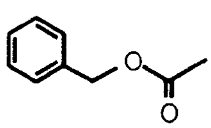 | $-CH_3$ | MS: m/e 694 ($M^++2$) |

Table 1 (Cont'd)

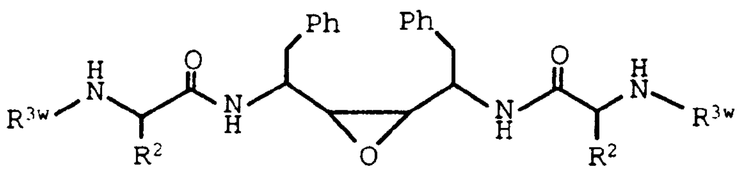

| Ex. | $R^{3w}$ | $R^2$ | PChem |
|---|---|---|---|
| 64 | 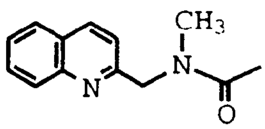 | $-CH_2CH_2CH_3$ | MS: m/e 878 ($M^+$+2) |
| 65 | 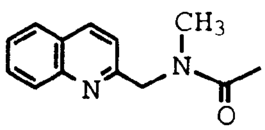 | $-CH_2CH_2CH_2CH_3$ | MS: m/e 906 ($M^+$+2) |
| 66 | 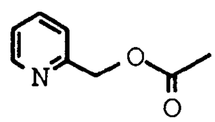 | $-C(CH_3)_3$ | MS: m/e 780 ($M^+$+2) |
| 67 | 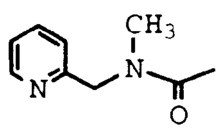 | $-CH(CH_3)CH_2CH_3$ | MS: m/e 807 ($M^+$+3) |
| 68 | 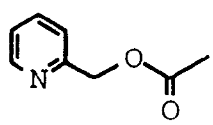 | $-CH_2CH_2CH_2CH_3$ | MS: m/e 780 ($M^+$+2) |
| 69 | 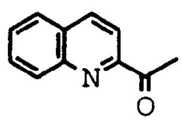 | $-CH(CH_3)_2$ | MS: m/e 791 ($M^+$+1) |
| 70 | 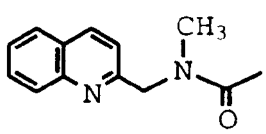 | $-CH_2CN$ | MS: m/e 872 ($M^+$+2) |
| 71 | 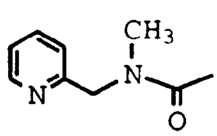 | $-CH_2CH_3$ | MS: m/e 749 ($M^+$+1) |
| 72 | 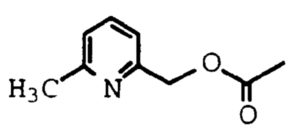 | $-CH(CH_3)_2$ | MS: m/e 780 ($M^+$+2) |

Table 1 (Cont'd)

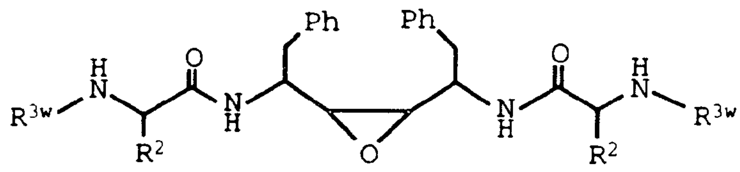

| Ex. | $R^{3w}$ | $R^2$ | PChem |
|---|---|---|---|
| 73 | 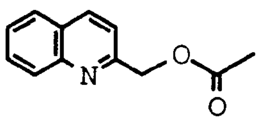 | $-C(CH_3)_3$ | MS: m/e 879 ($M^+$+1) |
| 74 | 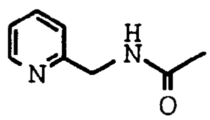 | $-CH(CH_3)CH_2CH_3$ | MS: m/e 777 ($M^+$+1) |

Table 2

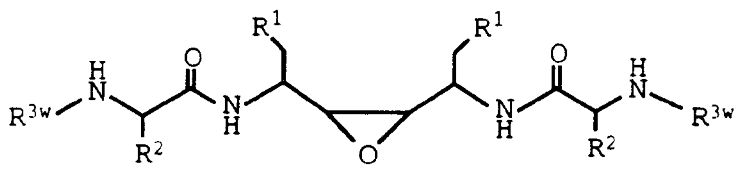

| Ex. | $R^{3w}$ | $R^2$ | $R^1$ | PChem |
|---|---|---|---|---|
| 75 | 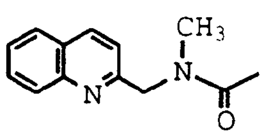 | $-CH_2CH_3$ | 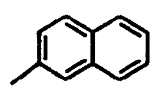 | MS: m/e 948 ($M^+$) |
| 76 | 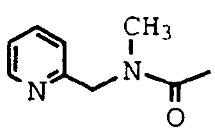 | $-CH(CH_3)CH_2CH_3$ | 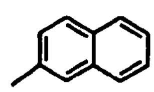 | MS: m/e 904 ($M^+$) |
| 77 | 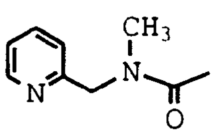 | $-CH_2CH_3$ | 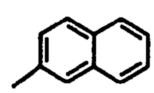 | MS: m/e 850 ($M^+$+2) |
| 78 | 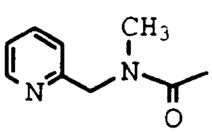 | $-CH_2CH_3$ | 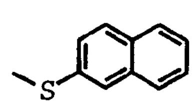 | MS: m/e 912 ($M^+$+1) |

Table 3

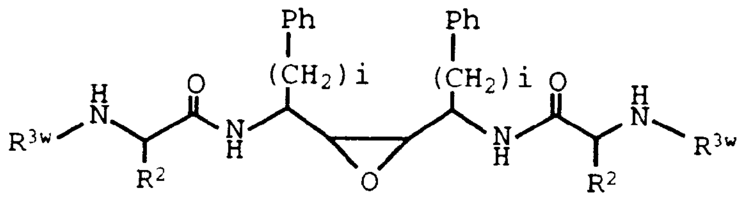

| Ex | $R^{3w}$ | $R^2$ | i | PChem |
|---|---|---|---|---|
| 79 | 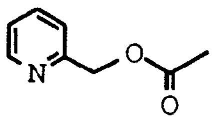 | $-CH(CH_3)_2$ | 2 | MS: m/e 778 ($M^+$) |
| 80 | 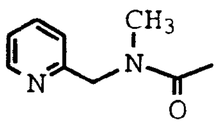 | $-CH_2CH_3$ | 3 | MS: m/e 806 ($M^+$+2) |
| 81 | 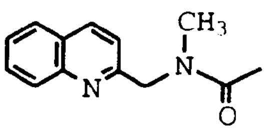 | $-CH_2CH_3$ | 3 | $^1H$ NMR ($CD_3OD$): δ 8.2 (m, 2); 7.95 (m, 2); 7.83 (m, 2); 7.72 (t, 2); 7.55 (t, 2); 7.40 (d, 2); 7.10 (m, 10); 4.70 (m, 4); 4.40 (t, 1); 4.15 (m, 2); 3.98 (m, 1); 3.85 (m, 1); 3.38 (m, 1); 3.02 (s, 3); 2.97 (s, 3); 2.55 (m, 4); 1.80 (m, 4); 1.58 (m, 8); 0.97 (t, 6) |

## Table 4

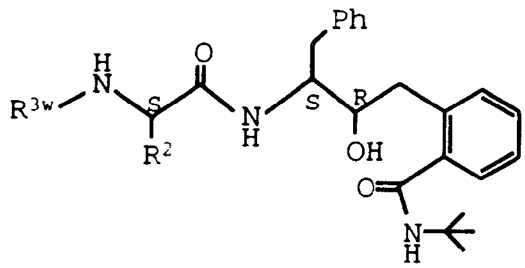

| Ex | $R^{3w}$ | $R^2$ | PChem |
|---|---|---|---|
| 82 | 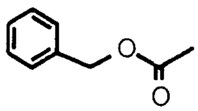 | $-CH_2OH$ | MS(FD): m/e 562 ($M^+$); 220 (100) |
| 83 | 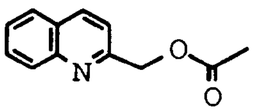 | $-CH(CH_3)CH_2CH_3$ | MS(FD): m/e 639 ($M^+$) |
| 84 | 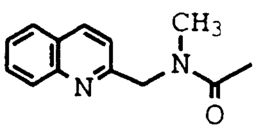 | $-CH(CH_3)CH_2CH_3$ | MS(FD): m/e 652 ($M^+$) |
| 85 | 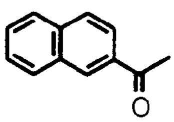 | $-CH_2CH_2C(O)NH_2$ | MS(FD): m/e 623 ($M^+$) |
| 86 | 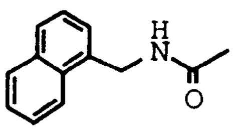 | $-CH_2CN$ | MS(FD): m/e 621 ($M^{+2}$) |
| 87 | 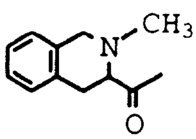 | $-CH_2CN$ | MS(FD): m/e 612 ($M^{+2}$) |
| 88 | 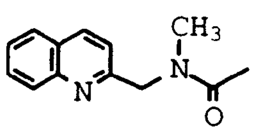 | $-CH_2CN$ | MS(FD): m/e 637 ($M^{+3}$) |
| 89 | 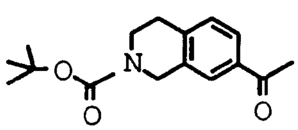 | $-CH_2CN$ | MS(FD): m/e 697 ($M^{+2}$) |

Table 4 (Cont'd)

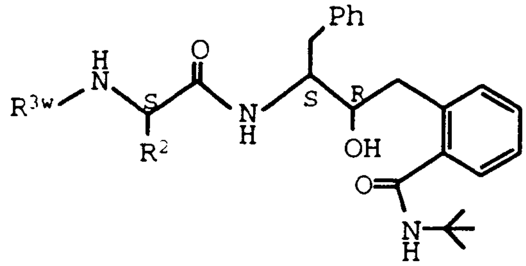

| Ex | $R^{3w}$ | $R^2$ | PChem |
|---|---|---|---|
| 90 | 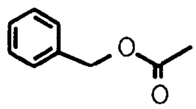 | 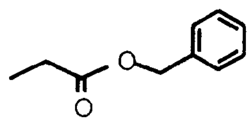 | MS(FD): m/e 680 ($M^+$) |
| 91 | 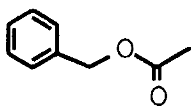 | 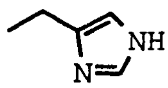 | Analysis for $C_{35}H_4N_5O_5$:<br>Calcd: C, 68.72; H, 6.76; N, 11.45;<br>Found: C, 65.60; H, 6.61; N, 10.94. |
| 92 | 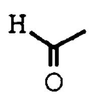 | 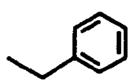 | MS(FD): m/e 516 ($M^+$) |
| 93 | 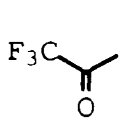 | 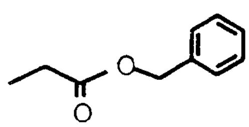 | Yield: 80 mg (53%); white solid |
| 94 | 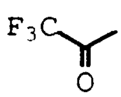 | 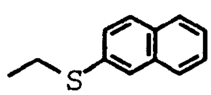 | MS(FD): m/e 516 ($M^+$) |

Table 4A

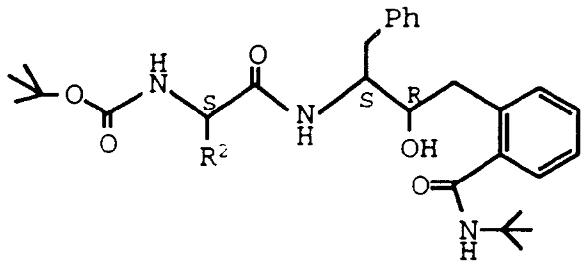

| Example | $R^2$ | PChem |
|---|---|---|
| 95 | 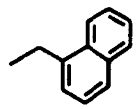 | Analysis for $C_{31}H_{45}N_3O_5$: Calcd: C, 69.12; H, 8.23; N, 7.80; Found: C, 69.38; H, 8.43; N, 7.96. |
| 96 | 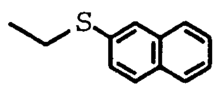 | MS(FD): m/e 669 ($M^+$) |
| 97 | 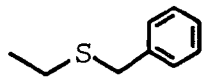 | MS(FD): m/e 634 ($M^+$) |
| 98 | 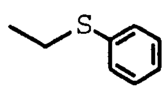 | Analysis for $C_{35}H_{45}N_3O_5S$: Calcd: C, 67.82; H, 7.32; N, 6.78; Found: C, 67.57; H, 7.20; N, 6.52. |
| 99 | 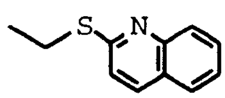 | MS(FD): m/e 670 ($M^+$) |
| 100 | 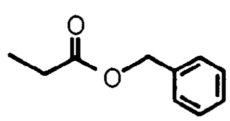 | Analysis for $C_{37}H_{47}N_3O_7$: Calcd: C, 68.82; H, 7.34; N, 6.54; Found: C, 68.65; H, 7.20; N, 6.77. |
| 101 | 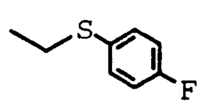 | MS(FD): m/e 637 ($M^+$) |
| 102 | 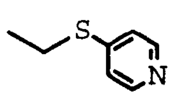 | MS(FD): m/e 621 ($M^+$) |
| 103 | 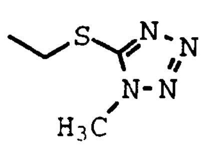 | MS(FD): m/e 626 ($M^+$) |

Table 4B

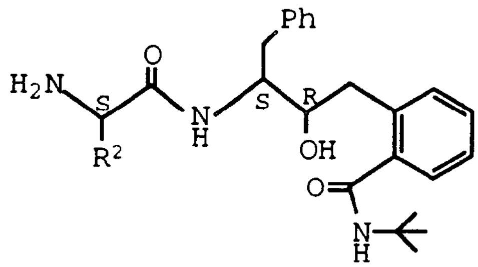

| Example | $R^2$ | PChem |
|---|---|---|
| 104 | 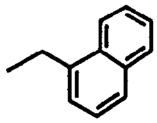 | Analysis for $C_{26}H_{37}N_3O_3$<br>Calcd: C, 71.04; H, 8.48; N, 9.56;<br>Found: C, 70.85; H, 8.59; N, 9.56. |
| 105 | 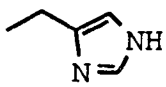 | MS: m/e 478 ($M^+$) |
| 106 | 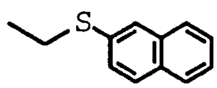 | MS(FD): m/e 569 ($M^+$) |
| 107 | 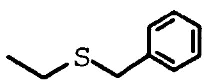 | MS(FD): m/e 534 ($M^+$) |
| 108 | 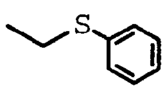 | Analysis for $C_{30}H_{37}N_3O_3S$:<br>Calcd: C, 69.33; H, 7.18; N, 8.08;<br>Found: C, 69.60; H, 7.43; N, 8.07. |
| 109 | 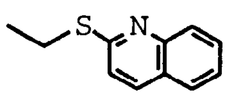 | Analysis for $C_{33}H_{38}N_4O_3S$:<br>Calcd: C, 69.45; H, 6.71; N, 9.82;<br>Found: C, 69.59; H, 6.58; N, 9.61. |
| 110 | 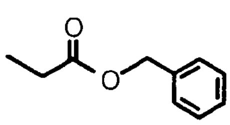 | Yield: 0.75g (42%) |
| 111 | 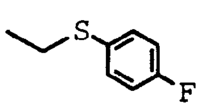 | MS(FD): m/e 538 ($M^+$) |
| 112 | 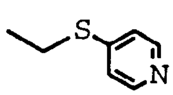 | MS(FD): m/e 521 ($M^+$) |
| 113 | 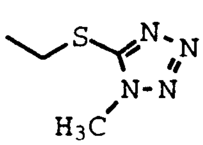 | MS(FD): m/e 526 ($M^+$) |

Table 4C

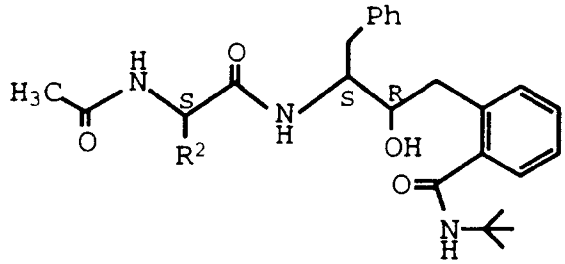

| Example | $R^2$ | PChem |
|---|---|---|
| 114 | 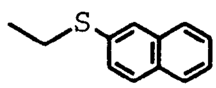 | MS(FD): m/e 611 ($M^+$) |
| 115 | 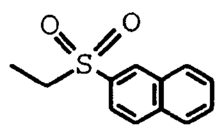 | MS(FD): m/e 644 ($M^+$) |
| 116 | 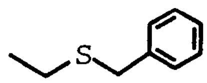 | MS(FD): m/e 575 ($M^+$) |
| 117 | 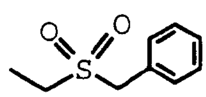 | MS(FD): m/e 608 ($M^+$) |
| 118 | 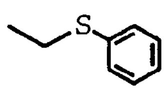 | MS(FD): m/e 561 ($M^+$) |
| 119 | 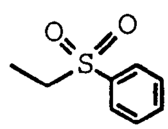 | MS(FD): m/e 594 ($M^+$) |
| 120 | $-CH_2CH_2SCH_3$ | MS(FD): m/e 514 ($M^+$) |
| 121 | 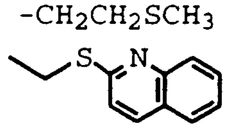 | MS(FD): m/e 612 ($M^+$) |
| 122 | 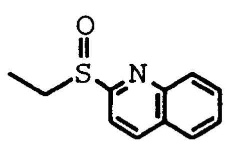 | Analysis for $C_{35}H_{40}N_4O_5S$: Calcd: C, 66.86; H, 6.41; N, 8.91; Found: C, 67.13; H, 6.37; N, 8.63. |
| 123 | 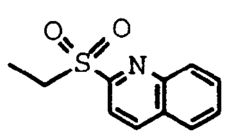 | Analysis for $C_{35}H_4N_4O_6S$: Calcd: C, 65.20; H, 6.25; N, 8.69; Found: C, 65.49; H, 6.26; N, 8.61. |

Table 4D

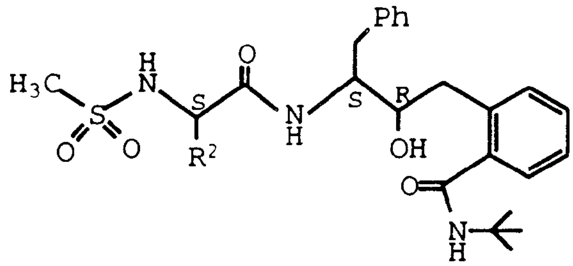

| Example | $R^2$ | PChem |
|---|---|---|
| 124 | 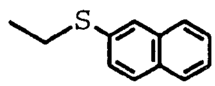 | MS(FD): m/e 647 ($M^+$) |
| 125 | 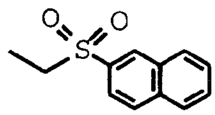 | MS(FD): m/e 680 ($M^+$) |
| 126 | 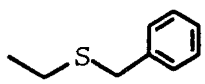 | MS(FD): m/e 611 ($M^+$) |
| 127 | 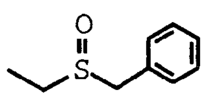 | MS(FD): m/e 628 ($M^+$) |
| 128 | 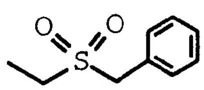 | MS(FD): m/e 644 ($M^+$) |
| 129 | 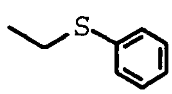 | MS(FD): m/e 597 ($M^+$) |
| 130 | 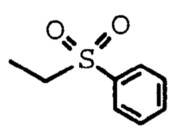 | MS(FD): m/e 630 ($M^+$) |
| 131 | 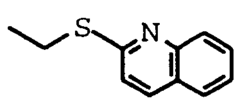 | MS(FD): m/e 649 ($M^+$) |
| 132 | 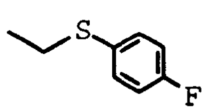 | MS(FD): m/e 615 ($M^+$) |

Table 4D (Cont'd)

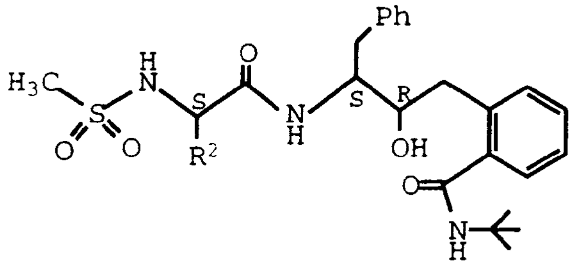

| Example | $R^2$ | PChem |
|---|---|---|
| 133 | 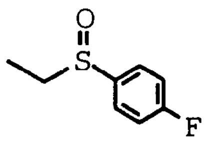 | MS(FD): m/e 632 ($M^+$) |
| 134 | 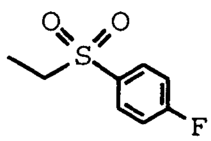 | Analysis for $C_{31}H_{38}FN_3O_7S_2$:<br>Calcd: C, 57.48; H, 5.91; N, 6.49;<br>Found: C, 57.19; H, 5.86; N, 6.23. |
| 135 | 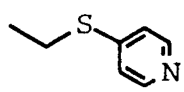 | MS(FD): m/e 598 ($M^+$) |
| 136 | 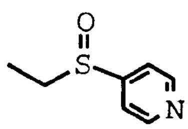 | Yield: 30 mg (36%); colorless oil |
| 137 | 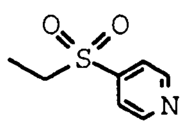 | Yield: 44 mg; white solid |
| 138 | 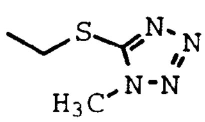 | MS(FD): m/e 604 ($M^+$) |
| 139 | 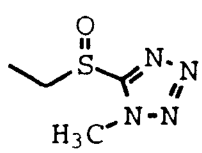 | MS(FD): m/e 620 ($M^+$) |

## Table 5

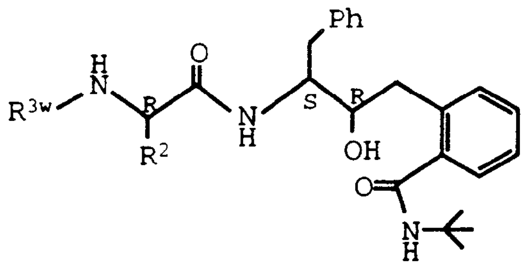

| Ex. | $R^{3w}$ | $R^2$ | PChem |
|---|---|---|---|
| 140 | 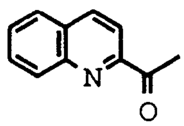 | 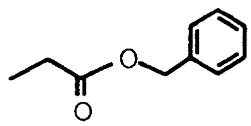 | MS(FD): m/e 701 ($M^+$) |
| 141 | 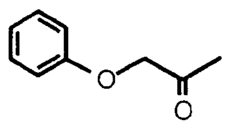 | 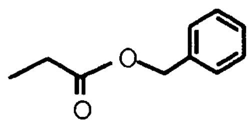 | MS(FD): m/e 680 ($M^+$) |
| 142 | 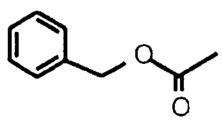 | $-CH_2SO_2NH_2$ | MS(FD): m/e 625 ($M^+$) |
| 143 | 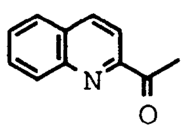 | $-CH_2SO_2NH_2$ | MS(FD): m/e 646 ($M^+$) |
| 144 | 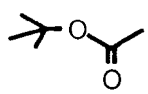 | 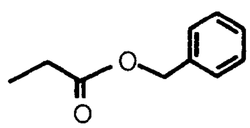 | Analysis for $C_{37}H_{47}N_3O_7$: Calcd: C, 68.82; H, 7.34; N, 6.51; Found: C, 69.16; H, 7.50; N, 6.56. |
| 145 | -H | 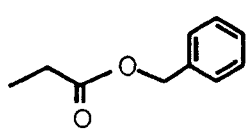 | |
| 146 | 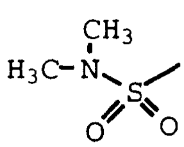 | 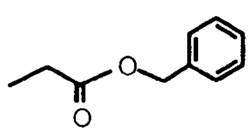 | MS(FD): m/e 653 ($M^+$) |

Table 5 (cont'd)

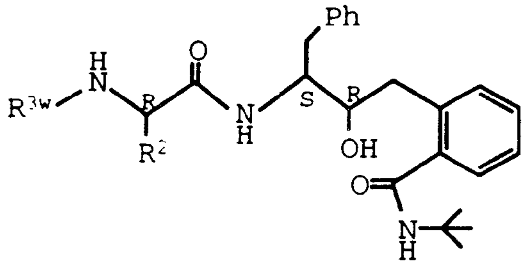

| Ex | $R^{3w}$ | $R^2$ | PChem |
|---|---|---|---|
| 147 | 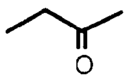 | 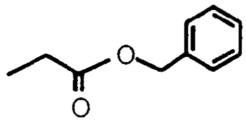 | MS(FD): m/e 602 ($M^+$) |
| 148 | 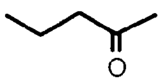 | 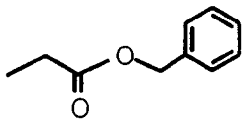 | MS(FD): m/e 616 ($M^+$) |
| 149 | 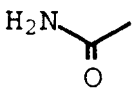 | 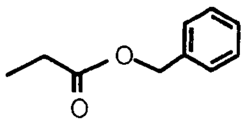 | MS(FD): m/e 589 ($M^+$) |
| 150 | 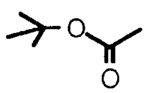 | 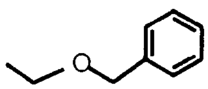 | Yield: 0.52g (68%) |
| 151 | -H | 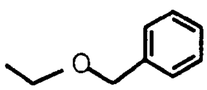 | MS(FD): m/e 518 ($M^+$) |
| 152 | 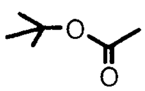 | 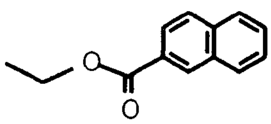 | Yield: 570 mg (80%) |

Table 5A

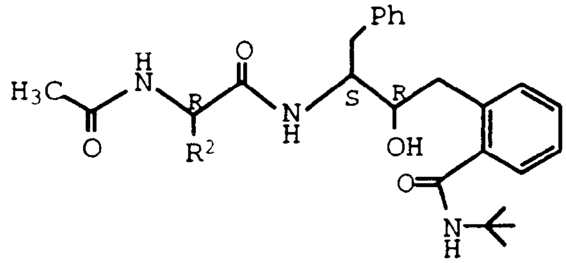

| Example | $R^{3w}$ | PChem |
|---|---|---|
| 153 | 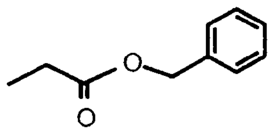 | MS(FAB): mass 588.3051 |
| 154 | $-CH_2CO_2H$ | Yield: 0.51g (55%) |
| 155 | 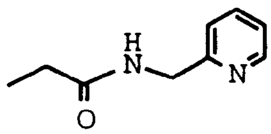 | MS(FD): m/e 588 ($M^+$) |
| 156 | 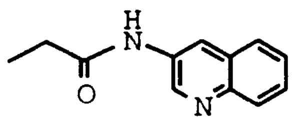 | MS(FD): m/e 623 ($M^+$) |
| 157 | 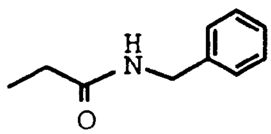 | MS(FD): m/e 587 ($M^+$) |
| 158 | 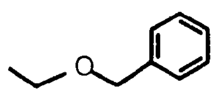 | MS(FD): m/e 560 ($M^+$) |
| 159 | 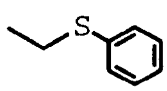 | MS(FD): m/e 561 ($M^+$) |
| 160 | 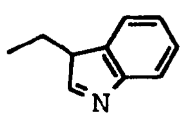 | MS(FD): m/e 568 ($M^+$) |
| 161 | 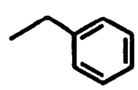 | MS(FD): m/e 530 ($M^+$) |
| 162 | 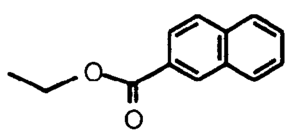 | Analysis for $C_{37}H_{41}N_3O_6$:<br>Calcd: C, 71.25; H, 6.62; N, 6.74;<br>Found: C, 71.32; H, 6.70; N, 6.67. |
| 163 | 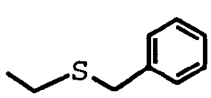 | MS(FD): m/e 575 ($M^+$) |

Table 5B

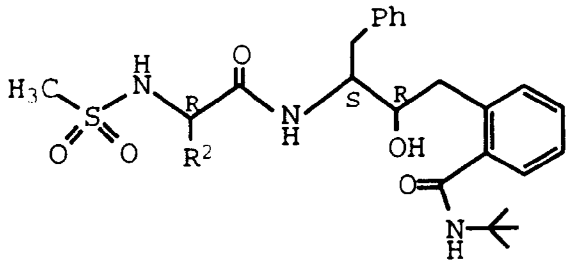

| Example | $R^{3w}$ | PChem |
|---|---|---|
| 164 | $-CH_2CO_2H$ | Yield: 0.10g (91%) |
| 165 | 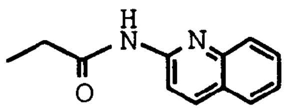 | MS(FD): m/e 660 ($M^+$) |
| 166 | 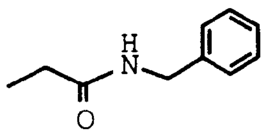 | MS(FD): m/e 623 ($M^+$) |
| 167 | 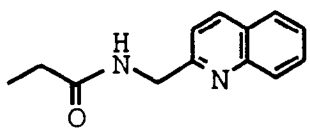 | MS(FD): m/e 674 ($M^+$) |
| 168 | 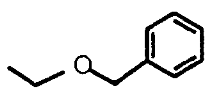 | MS(FD): m/e 596 ($M^+$) |
| 169 | 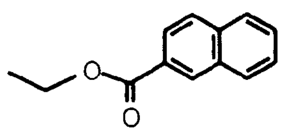 | Yield: 240mg (71%) |

Table 6

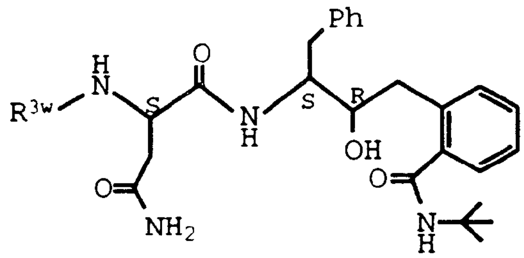

| Ex. | $R^{3w}$ | PChem |
|---|---|---|
| 170 | 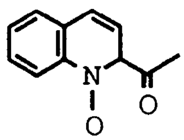 | IR (KBr): 3311, 3065, 2966, 1656, 1520, 1453, 1366, 735, 701 $cm^{-1}$ |
| 171 | 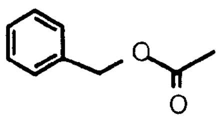 | MS(FD): m/e 589 ($M^+$), 589 (100) |
| 172 | 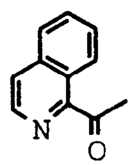 | MS(FD): m/e 610 ($M^+$), 220 (100) |
| 173 | 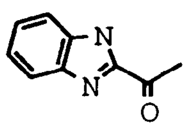 | MS(FD): m/e 599 ($M^+$), 599 (100) |
| 174 | 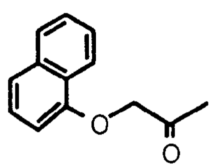 | MS(FD): m/e 639 ($M^+$), 639 (100) |
| 175 | 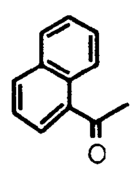 | MS(FD): m/e 609 ($M^{+1}$) |
| 176 | 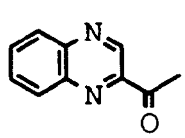 | MS(FD): m/e 612 ($M^{+2}$) |

Table 6 (Cont'd)

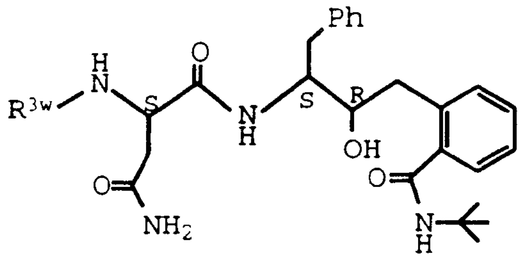

| Ex. | $R^{3w}$ | PChem |
|---|---|---|
| 177 | 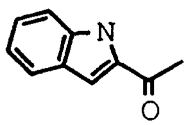 | MS(FD): m/e 599 ($M^{+2}$) |
| 178 | 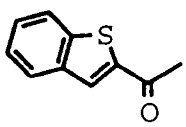 | MS(FD): m/e 616 ($M^{+2}$) |
| 179 | 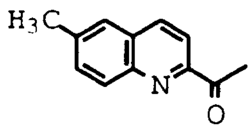 | MS(FAB): m/e 623 ($M^{+1}$) |
| 180 | 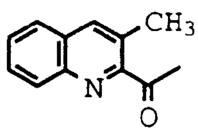 | MS(FAB): m/e 623 ($M^{+1}$) |
| 181 | 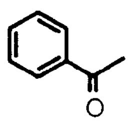 | MS(FAB): m/e 558 ($M^{+1}$) |
| 182 | 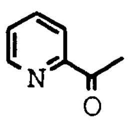 | MS(FAB): m/e 559 ($M^{+1}$) |
| 183 | 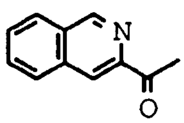 | MS(FAB): m/e 609 ($M^{+1}$) |
| 184 | 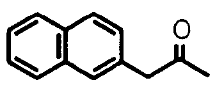 | MS(FD): m/e 623 ($M^{+1}$) |

Table 6 (cont'd)

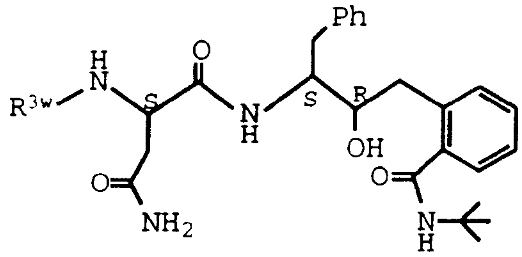

| Ex. | $R^{3w}$ | PChem |
|---|---|---|
| 185 | 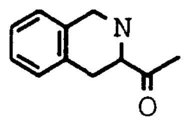 | MS(FD): m/e 614 ($M^{+2}$) |
| 186 | 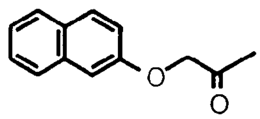 | MS(FD): m/e 640 ($M^{+1}$) |
| 187 | 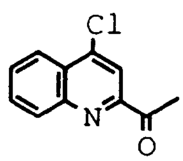 | MS(FD): m/e 644 |
| 188 | 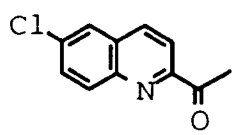 | MS(FAB): m/e 644 |
| 189 | 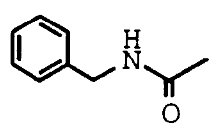 | MS(FD): m/e 588 (M) |
| 190 | 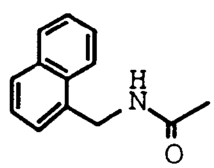 | MS(FD): m/e 638 ($M^{+1}$) |
| 191 | 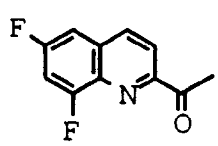 | MS(FD): m/e 646 ($M^{+1}$) |

Table 6 (Cont'd)

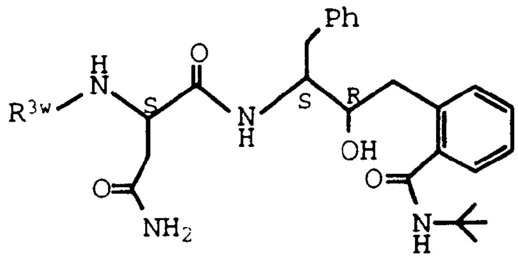

| Ex. | $R^{3w}$ | PChem |
|---|---|---|
| 192 | 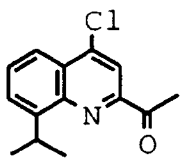 | MS(FD): m/e 686 |
| 193 | 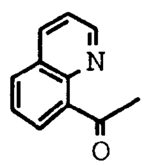 | MS(FD): m/e 644 |
| 194 | 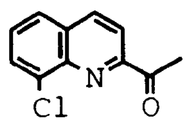 | MS(FD): m/e 644 |
| 195 | 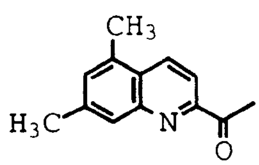 | MS(FD): m/e 638, 546 |
| 196 | 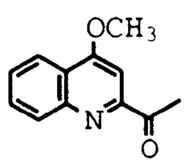 | MS(FD): 640, 420, 220 |
| 197 | 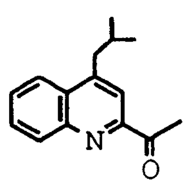 | MS(FD): m/e 666, 573, 445 |
| 198 | -H | MS(FAB): m/e 455 $(M^{+1})$ |

Table 6 (Cont'd)

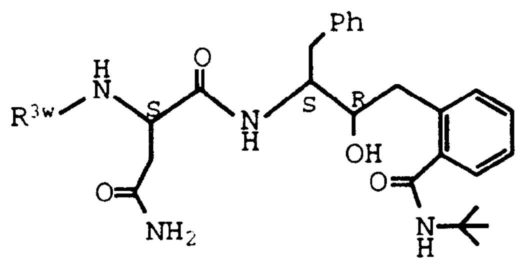

| Ex. | $R^{3w}$ | PChem |
|---|---|---|
| 199 | 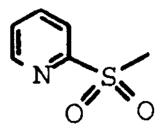 | MS(FD): m/e 596 ($M^+$) |
| 200 | 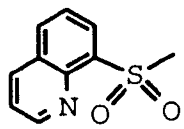 | MS(FD): m/e 646 ($M^+$) |
| 201 | 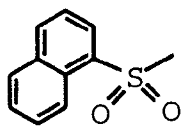 | MS(FD): m/e 645 ($M^{+1}$) |
| 202 | 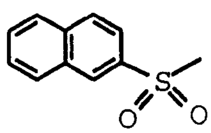 | MS(FD): m/e 645 ($M^{+1}$) |
| 203 | 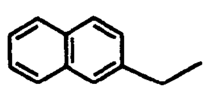 | Analysis for $C_{37}H_{42}N_3O_6S$:<br>Calcd: C, 65.63; H, 6.43; N, 8.50;<br>Found: C, 65.52; H, 6.45; N, 8.43. |

Table 7 (Cont'd)

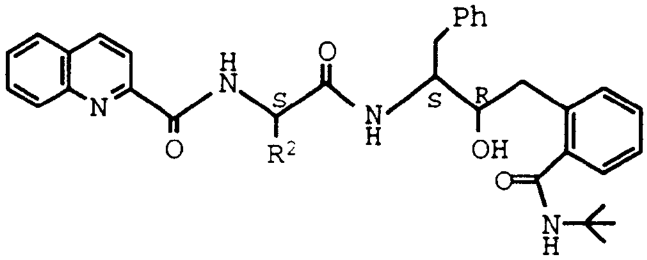

| Ex. | $R^2$ | PChem |
|---|---|---|
| 204 | $-CH(CH_3)_2$ | MS(FAB): m/e 595 ($M^+$); 128 (100) |
| 205 | 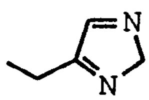 | MS(FD): m/e 633 ($M^+$); 633 (100) |
| 206 | $-CH_2CH_2CO_2H$ | MS(FD): m/e 626 (15); 625 (35); 220 (8) |
| 207 | 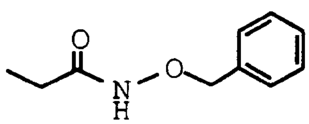 | MS(FD): m/e 717 ($M^{+1}$ 24) |
| 208 | 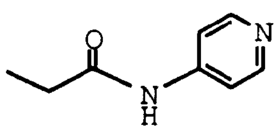 | MS(FD): m/e 688 ($M^{+1}$,100); 220 (22) |
| 209 | 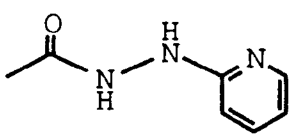 | MS(FD): m/e 702 ($M^-$,100) |
| 210 | $-CH_2CH_2C(O)NH_2$ | MS(FD): m/e 624 ($M^{+2}$) |
| 211 | 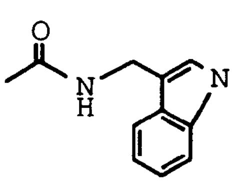 | MS(FD): m/e 739 ($M^{+1}$,35); 738 (100) |

Table 7

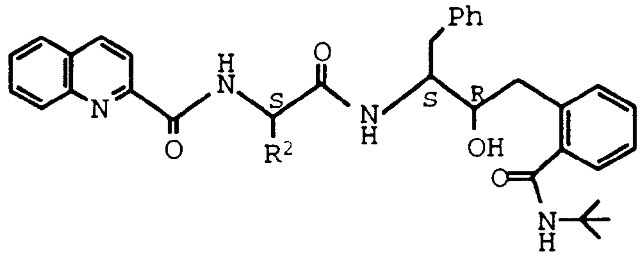

| Example | $R^2$ | PChem |
|---|---|---|
| 212 | $-CH_2CN$ | MS(FD): m/e 594 ($M^{+2}$); 501, 372, 224 |
| 213 | 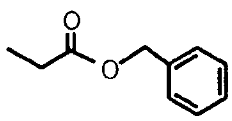 | $^1$H NMR : δ 1.48 (s, 9H); 2.78-3.12 (m, 6H); 3.71 (m, 1H); 4.30 (m, 1H); 5.05 (m, 1H); 5.16 (s, 2H); 5.98 (s, 1H); 6.76-6.85 (m, 2H); 6.92 (t, J=9 Hz, 2H); 7.17-7.43 (m, 11H); 7.70 (t, J=9 Hz, 1H); 7.85 (t, J=9 Hz, 1H); 7.95 (d, J=9 Hz, 1H); 8.18 (d, J=9 Hz, 1H); 8.27 (d, J=9 Hz, 1H); 8.39 (d, J=9 Hz, 1H); 9.00 (d, J=12 Hz, 1H). |
| 214 | 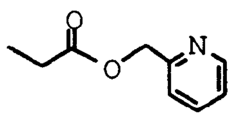 | MS(FD): m/e 702 ($M^+$) |
| 215 | 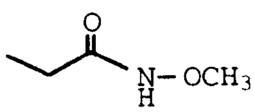 | MS(FD): m/e 640 ($M^{+1}$,38); 610 (63),593 (58) |
| 216 | 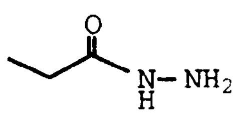 | MS(FD): m/e 625 ($M^+$,100) |
| 217 | 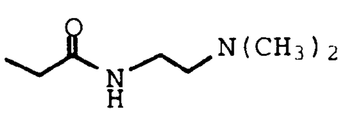 | MS(FD): m/e 681 ($M^{+1}$) |

Table 8

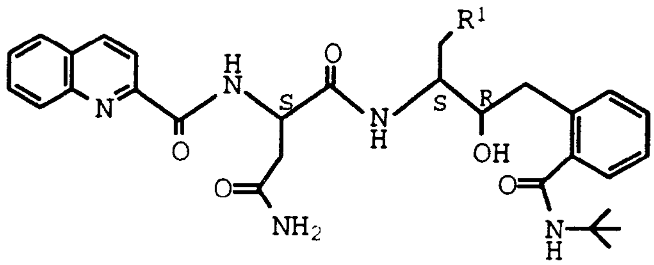

| Example | $R^1$ | PChem |
|---|---|---|
| 218 | 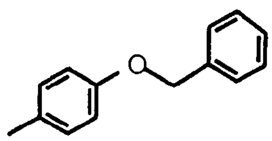 | MS(FD): m/e 716 ($M^+$, 100) |
| 219 | 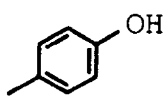 | MS(FD): m/e 626 ($M^+$, 100) |
| 220 | 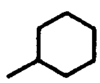 | MS(FD): m/e 616 (100); 396 (15); 395 (33); 394 (8); 221 (18); 220 (100); 192 (20); 191 (88) |
| 221 | 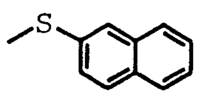 | MS(FD): m/e 692 ($M^+$); 691 (100) |
| 222 | 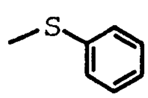 | MS(FD): m/e 642 ($M^+$, 100); |

Table 9

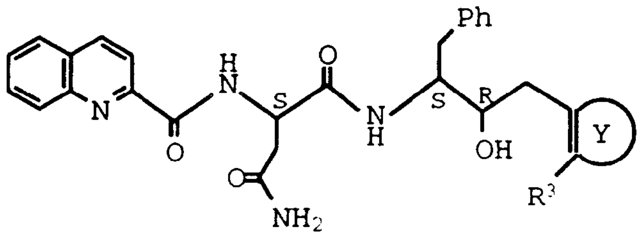

| Example | 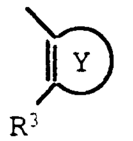 | PChem |
|---|---|---|
| 223 | 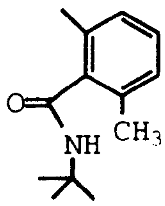 | MS(FD): m/e 624 ($M^+$, 100) |
| 224 | 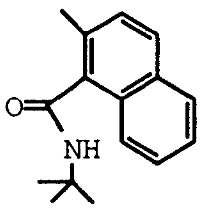 | MS(FD): m/e 660 ($M^+$, 100) |
| 225 | 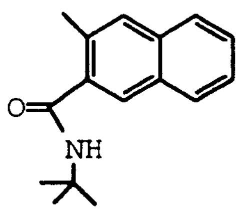 | MS(FD): m/e 661 ($M^+$, 100) |
| 226 | 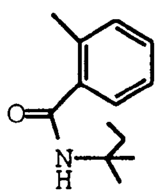 | MS(FD): m/e 624 ($M^+$, 100) |
| 227 | 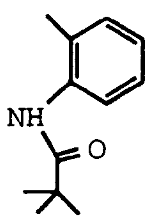 | MS(FD): m/e 610 ($M^+$, 100) |

Table 9 (Cont'd)

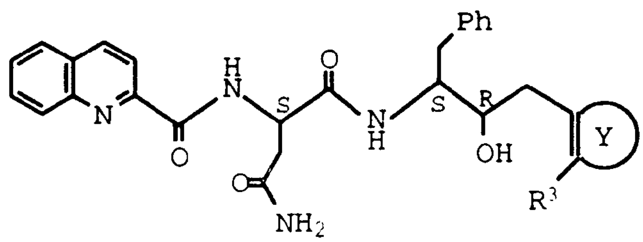

| Example | 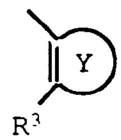 | PChem |
|---|---|---|
| 228 | 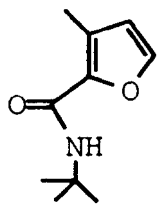 | MS(FAB): mass 600.2839 (M+H) |
| 229 | 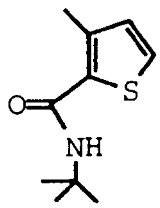 | MS(FAB): mass 616.2591 (M+H) |
| 230 | 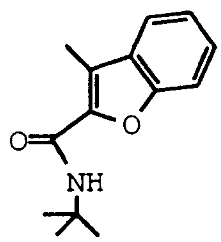 | MS(FD): m/e 650 ($M^+$), |
| 231 | 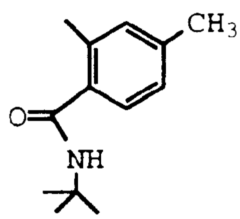 | MS(FAB): 624; 607; 355) |

Table 9 (Cont'd)

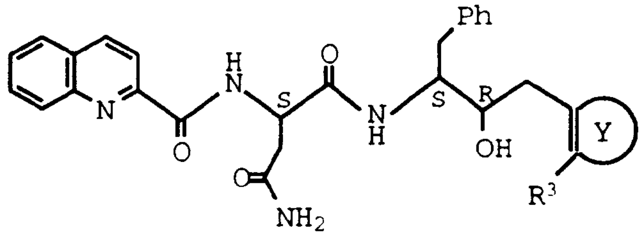

| Example | 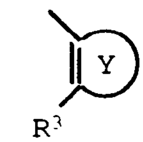 | PChem |
|---|---|---|
| 232 | 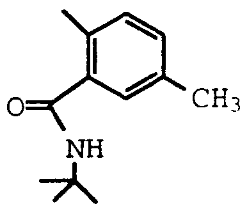 | $^1H$ NMR: δ 1.45 (s, 9H); 2.26 (s, 3H); 2.73-2.80 (m, 2H); 2.94-2.99 (m, 2H); 3.13-3.23 (m, 2H); 3.90-3.95 (m, 1H); 4.29-4.40 (m, 1H); 4.89-4.97 (m, 1H); 6.68 (s, 1H); 6.79 (s, 1H); 6.85-7.15 (m, 10H); 7.49-7.60 (m, 3H); 7.72 (t, J=7.5 Hz, 1H); 7.79 (d, J=8 Hz, 1H); 8.03-8.14 (m, 2H); 8.18 (d, J=8 Hz, 1H); 8.85 (d, J=6.3 Hz, 1H). |
| 233 | 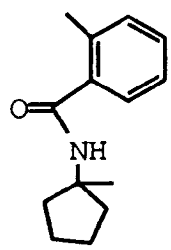 | MS(FAB): 636 ($M^+$, 100) |

Table 10

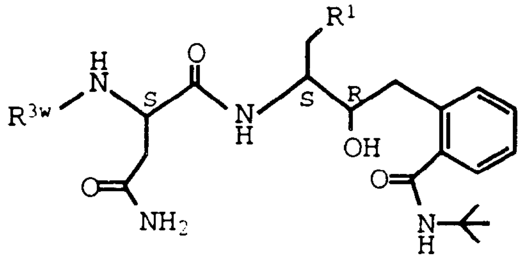

| Example | $R^{3w}$ | $R^1$ | PChem |
|---|---|---|---|
| 234 | 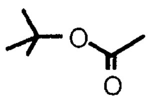 | 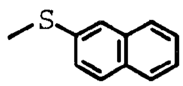 | MS(FD): m/e 637 ($M^+$), 636 (100) |
| 235 | -H | 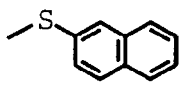 | MS(FD): m/e 537 ($M^+$), 536 (100) |
| 236 | 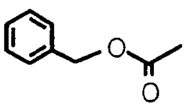 | 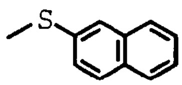 | MS(FD): m/e 671 ($M^+$), 670 (100) |
| 237 | 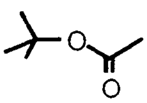 | 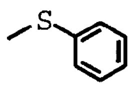 | MS(FD): m/e 587 ($M^+$, 100) |
| 238 | -H | 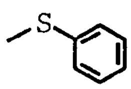 | MS(FD): m/e 487 ($M^+$, 100) |
| 239 | 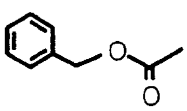 | 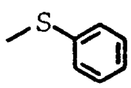 | MS(FD): m/e 621 ($M^+$, 100) |
| 240 | 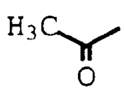 | 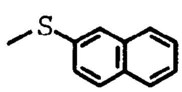 | MS(FD): m/e 579 ($M^+$), 578 (100) |
| 241 | 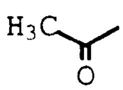 | 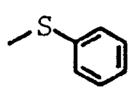 | MS(FD): m/e 529 ($M^+$), 528 (100) |

Table 11

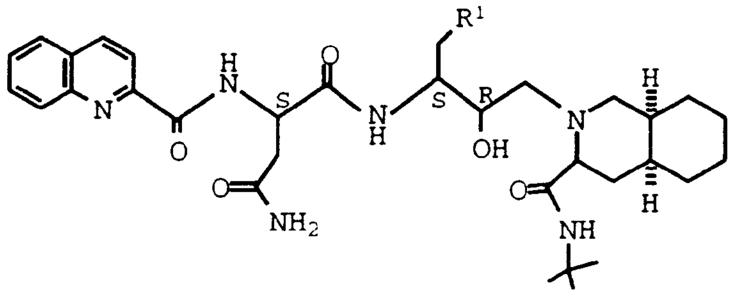

| Example | $R^1$ | PChem |
|---|---|---|
| 242 | 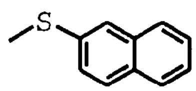 | MS(FD): m/e 652 ($M^+$, 100) |
| 243 | 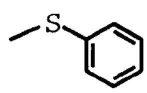 | MS(FAB): m/e 703 ($M^+$); 602 |

Table 12

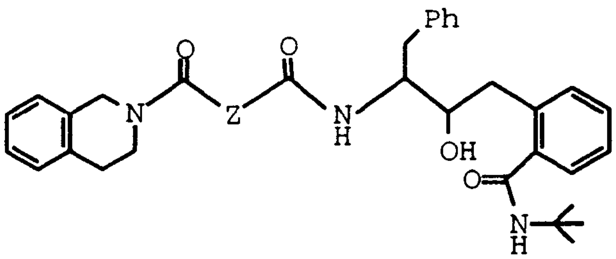

| Example | Z | PChem |
|---|---|---|
| 244 | 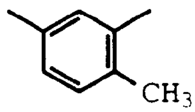 | MS(FD): m/e 618 ($M^+$, 100) |
| 245 | 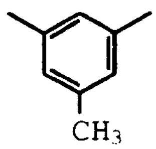 | MS(FD): m/e 618 ($M^+$, 100) |
| 246 | 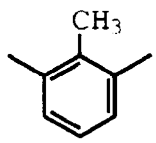 | MS(FD): m/e 618 ($M^+$); 220 (100) |
| 247 | 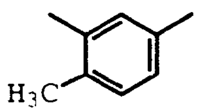 | MS(FD): m/e 618 ($M^+$, 100) |
| 248 | 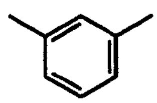 | MS(FD): m/e 604 ($M^+$, 100) |

Table 13

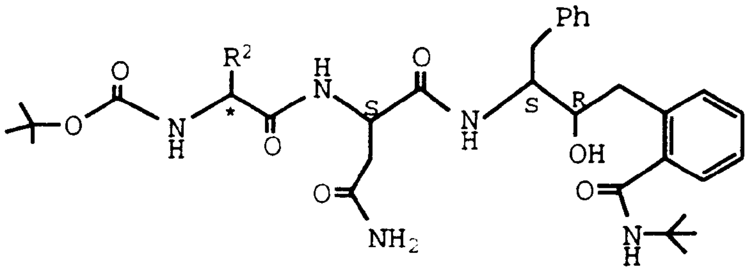

| Ex. | $R^2$ | * | PChem |
|---|---|---|---|
| 249 | 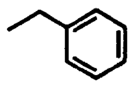 | S | Analysis for $C_{39}H_{51}N_5O_7$ Calcd: C, 66.74; H, 7.32; N, 9.98; Found: C, 65.59; H, 7.44; N, 9.76. |
| 250 | 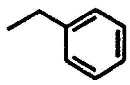 | R | Yield: 100 mg (33%) |
| 251 | 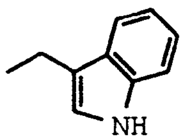 | S | MS(FD): m/e 740 ($M^+$-1) |
| 252 | 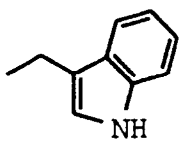 | R | MS(FD): m/e 741 ($M^+$) |
| 253 | 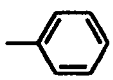 | S | Analysis for $C_{38}H_{49}N_5O_7$ Calcd: C, 66.36; H, 7.18; N, 10.18; Found: C, 65.63; H, 7.10; N, 10.28. |
| 254 | 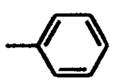 | R | Analysis for $C_{38}H_{49}N_5O_7$ Calcd: C, 66.36; H, 7.18; N, 10.18; Found: C, 66.20; H, 7.23; N, 10.31 |

Table 14

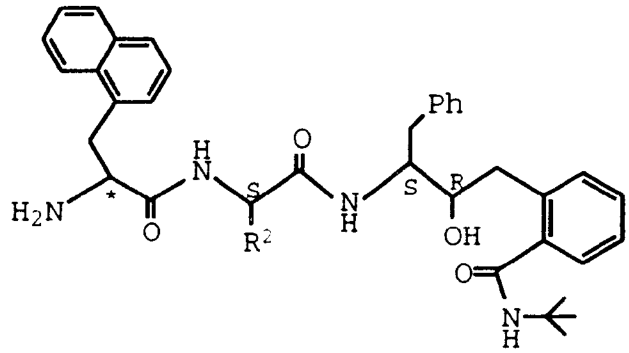

| Example | $R^2$ | * | PChem |
|---|---|---|---|
| 255 | $-CH(CH_3)_2$ | S | MS(FD): m/e 636 ($M^+$, 100) |
| 256 | $-CH(CH_3)_2$ | R | MS(FD): m/e 636 ($M^+$, 100) |
| 257 | $-CH_2C(O)NH_2$ | S | MS(FD): m/e 652 ($M^+$, 100) |
| 258 | $-CH_2C(O)NH_2$ | R | Analysis for $CH_{39}H_{48}N_4O_4$<br>Calcd: C, 70.13; H, 6.81; N, 10.76;<br>Found: C, 70.38; H, 7.01; N, 10.79. |

Table 15

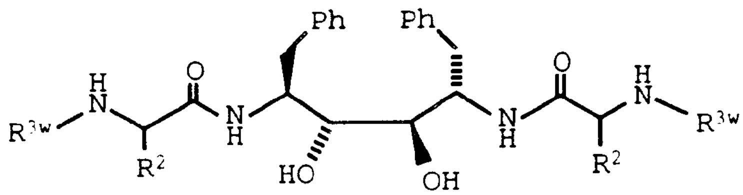

| Example | $R^{3w}$ | $R^{2w}$ | PChem |
|---|---|---|---|
| 259 | 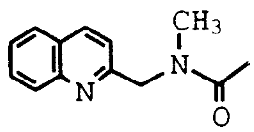 | $-CH_2CH_3$ | Yield: 105 mg (50.2%) |
| 260 | 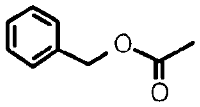 | $-CH_2CH(CH_3)_2$ | MS: 795 ($M^+$) |
| 261 | 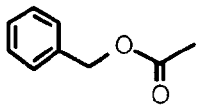 | $-CH_2CH_2CH_3$ | Yield: 16.1 mg (10.6%) |
| 262 | 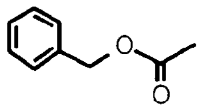 | $-CH_2CN$ | Yield: 40 mg (4%) |

Table 16

| Example | Compound | PChem |
|---|---|---|
| 263 | $CH_3C(O)$- NA-Val-Sta-Leu- $NA\text{-}NH_2$ | MS(FAB): m/e 824 |
| 264 | $CH_3C(O)$- NA-Val-Sta-Gly- $NA\text{-}NH_2$ | MS(FAB): m/e 768 |
| 265 | $CH_3C(O)$- NA-Val-Sta-Ala- $NA\text{-}NH_2$ | MS(FAB): m/e 781 |
| 266 | $CH_3C(O)$- NA-Val-Sta-Val- $NA\text{-}NH_2$ | MS(FAB): m/e 809 |
| 267 | $CH_3C(O)$- NA-Val-Sta-Ile- $NA\text{-}NH_2$ | MS(FAB): m/e 823 |
| 268 | $CH_3C(O)$- NA-Val-Sta-Nva- $NA\text{-}NH_2$ | MS(FAB): m/e 809 |
| 269 | $CH_3C(O)$- NA-Val-Sta-Nle- $NA\text{-}NH_2$ | MS(FAB): m/e 823 |
| 270 | NA-Val-Sta-Nva- $NA\text{-}NH_2$ | MS(FAB): m/e 767 |
| 271 | NA-Val-Sta-Nva- NA-OH | MS(FAB): m/e 768 |
| 272 | $CH_3C(O)$- NA-Val-Sta-Nva- NA-OH | MS(FAB): m/e 810 |
| 273 | $CH_3C(O)$-bNA-Val-Sta-Leu-$bNA\text{-}NH_2$ | MS(FAB): m/e 823 |
| 274 | 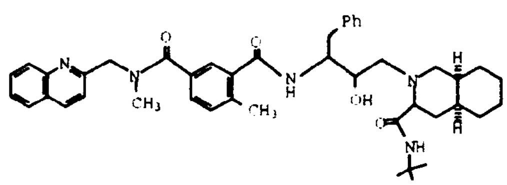 | MS(FD): m/e 719 ($M^+$); 617(100) |
| 275 | 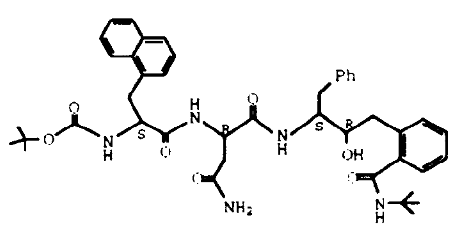 | MS(FD): m/e 751 ($M^+$, 100) |

As noted above, the present invention provides a process for identifying a βAP production inhibitor, comprising:

(1) administering an aspartyl protease inhibitor to a test system;

(2) measuring βAP production in the test system; and

(3) comparing βAP production in the test system to βAP production in a control test system in which no such aspartyl protease inhibitor has been administered.

This aspect of the present invention is carried out as follows. First, test compounds which inhibit exemplary aspartyl proteases, particularly cathepsin D, are identified as aspartyl protease inhibitors. As used herein, the term "test system" refers to an assay that is designed to measure aspartyl protease inhibitory activity. A typical test system used for such identification is a non-cellular assay where turnover of the protease substrate is monitored upon the administering of a protease inhibitor. The turnover of the protease inhibitor is then compared to the turnover of substrate in a control test system in which no such aspartyl protease inhibitor has been administered. As used herein, the term "control test system" refers to an identical test system that is run side-by-side with the test system with the exception no aspartyl protease inhibitor is administered. This non-cellular assay is used to identify those test compounds having suitable aspartyl protease inhibitory activity in a non-cellular environment.

An example of a non-cellular assay that may be used to determine *in vitro* aspartyl protease inhibitory activity reacts partially purified human brain cathepsin D with a substrate, e.g., porcine renin tetradecapeptide. The resultant reaction mixture is divided into portions and each portion is incubated for a different period of time. After the reaction is stopped, the turnover of substrate in each portion is determined using reverse-phase HPLC. Protease inhibition activity may be calculated based on the rates of substrate turnover. This assay is exemplified in Assay 1, below.

A second non-cellular assay for determining *in vitro* aspartyl protease inhibitory activity utilizes a microtiter plate, where a suitable aspartyl protease, such as cathepsin D is added to each well together with controls. A suitable fluorometric substrate is added to each well. The resultant reaction mixture is allowed to react for a preselected time, e.g., 30 minutes. After enzyme activity is terminated, the substrate or its product may be determined fluorometrically and then the protease inhibition activity may be calculated based on the rates of substrate turnover. This assay is exemplified in Assay 2, below.

A third non-cellular assay for determining *in vitro* aspartyl protease inhibitory activity is generally described in Jupp, R.A. et al., (1990) Biochem. J., 265:871-878 and Rao et al., J. Med. Chem. (1993), in press. The assay is carried out by incubating a test compound with a suitable aspartyl protease, such as cathepsin D, in the presence of a chromogenic substrate. The inhibition constant, $K_i$, value may then be determined by quantifying the competitive inhibition of the hydrolysis of the chromogenic substrate. A variety of chromogenic substrates that are useful for assaying aspartyl protease activity are discussed in Dunn, et al., (1986) Biochem. J., 237:899-906. A preferred chromogenic substrate for a cathepsin D assay is disclosed in Scarborough, P.E. et al., Protein Science, (1993), 2:264-276.

Concentrations of substrate solutions and cleavage products may be verified using amino acid analysis. The enzyme preparations may be titrated with a tightly binding inhibitor to quantitate the active concentration of the enzyme. Enzyme concentrations are typically in the range of from 1-6 nM. Stock solutions of the test compound are prepared at concentrations ranging from 1.0 to 5.0 mg/mL, and then 50-100 μL portions of these stock solutions may be further diluted to a concentration in the range of 5 to 300 μM for rate determinations.

First, the percentage of inhibition of the initial rate of reaction in the presence of 4mM of the test compound may be determined relative to a control reaction in order to provide an estimate of the test compound's potency. Then, the test compounds are assayed over a concentration range of, for example, 5 to 300 μM for rate determinations.

A decrease in absorbance in the range of 284-324 nm may be monitored using a spectrophotometer. Absorbance readings are typically taken at various timepoints. The initial velocities are then plotted versus the initial substrate concentration. The kinetic parameter, $K_m$ for the chromogenic substrate may be calculated from these data. Estimates of $K_i$ may then be determined using the Dixon construction. Dixon, M., (1953) Biochem J., 55:170-171.

If the ratio of the total active enzyme concentration to the estimated $K_i$ value ($[E]_t/K_i$) is less than 0.2, then the precise $K_i$ value may be computed by using a non-linear curve fitting program based on the equation:

$$v = V_{max}[S]/[S] + K_m(1 + [I]/K_i)$$

where $v$ is the initial rate, [S] is the initial substrate concentration, [I] is the inhibitor concentration and $K_m$ and $K_i$ are defined above. If the ratio, $[E]_t/K_i$, is between 0.2 and 10, then the precise $K_i$ value may be calculated using the method described in Henderson. P.J.F., (1972) Biochem J., 127:321-333. This Assay is exemplfied in Assay 3.

It will be desirable to select those test compounds having aspartyl protease inhibitory activity for further testing in a cellular environment in order to identify which aspartyl protease inhibitors are most suitable for use as βAP production inhibitors. The initial assessment of aspartyl protease inhibitory activity may be based on a relative ranking of those test compounds tested, but will more typically be based on an absolute assessment of inhibitory activity, typically involving the measurement of an inhibitory concentration, i.e., the concentration needed to reach an arbitrary level of aspartyl protease inhibition, such as 50%. The term "IC50" refers to the concentration needed to reach 50% inhibition.

Such IC50 values can be measured using either of the above protocols by first determining the aspartyl protease activity in the absence of potential protease inhibitors in order to determine a control value. The aspartyl protease activity is then measured in the presence of varying concentrations of each test compound, and the concentration which results in an inhibition of protease activity of 50% is the IC50 value. All test compounds which result in an IC50 value at or below a selected threshold value may be designated suitable aspartyl protease inhibitors and may be further tested according to the methods of the present invention. This threshold value is arbitrary, but will usually be a low concentration, typically below 10 μg/mL, more usually 5 μg/mL, often 1 μg/mL, and sometimes 250 ng/mL, or below.

Upon identification of aspartyl protease inhibitors, further screening will be performed to select those aspartyl protease inhibitors which inhibit the intracellular production of βAP. The βAP production inhibition activity is typically measured in a cellular assay or in an animal model, or both.

The cellular assay preferably utilizes a cell line which has been transfected to overproduce βAP, such as a cell line which has been transfected with the Swedish or other mutation responsible for AD or other βAP-related condition. Typically these assays measure βAP production in a test system in which the aspartyl protease inhibitor has been administered and in a control test system in which no such aspartyl protease inhibitor has been administered in order to measure a reduction in βAP production relative to the control. These assays may measure βAP production or the presence of a cellular characteristic which is related to βAP production. Aspartyl protease inhibitors which inhibit or otherwise affect intracellular βAP production will typically be tested further.

The test system used to measure the βAP production inhibition activity is typically a cellular assay or an animal model. Preferably, the βAP production inhibition activity is obtained by direct measurement of βAP production in a cellular assay, as described in WO9410569, the full disclosure of which is incorporated herein by reference. This assay is exemplified in Assay 4, below.

For example, mammalian cell lines, typically human cell lines, are grown under conditions which result in the secretion of detectable amounts of βAP into the conditioned culture media, typically in the range of from about 0.1 ng/mL to 10 ng/mL. By growing cells under conditions which result in the accumulation of βAP in the conditioned culture medium, and exposing the cultured cells to an aspartyl protease inhibitor, it is possible to measure the effect of the aspartyl protease inhibitor on βAP production. An aspartyl protease inhibitor which diminishes βAP production is useful for the therapeutic treatment of AD and other βAP-related conditions.

Typical cell lines used in the cellular assay include human and animal cell lines, such as the 293 human kidney cell line, human neuroglioma cell lines, human HeLa cells, primary human endothelial cells (e.g., HUVEC cells), primary human fibroblasts or lymphoblasts, primary human mixed brain cells, including neurons, astrocytes, and neuroglia, Chinese hampster ovary (CHO) cells, and the like. Preferred are cell lines capable of expressing APP variants which overproduce βAP. As used herein, the term "overproduce" means that the amount of βAP produced from the variant APP will be greater than the amount produced from any or all of the normal APP isoforms, e.g., the 695, 751, and 770 amino acid isoforms which have been previously described. Particularly preferred APP variants are those having one or several amino acid substitutions directly amino-terminal of the βAP cleavage site. For example, K293 cells which express an APP containing the Swedish mutation produce approximately six-fold to eight-fold more βAP than cells expressing normal APP.

β-amyloid peptide may be measured in the conditioned culture medium by any technique which is sufficiently selective and sensitive to identify substantially intact βAP in the presence of other APP fragments which may be present. Immunological detection techniques may be employed using binding substances specific for βAP, such as antibodies, antibody fragments, recombinant antibodies, and the like, which possess the requisite specificity and sensitivity to βAP. Antibodies which are monospecific for the junction region of βAP are capable of distinguishing βAP from other fragments. As used herein the term "βAP junction region" refers to that region of βAP which is centered at the site between amino acid residues 16 and 17 ($Lys_{16}$ and $Leu_{17}$). The βAP junction region is a target for normal proteolytic processing of APP. Such normal processing results in a variety of APP fragments which are potentially immunologically cross-reactive with the intact βAP molecule. Antibodies raised against a synthetic peptide consisting of amino acid residues 13-28 of βAP have been found to display the requisite specificity.

Suitable detection techniques include ELISA, Western blotting, radioimmunoassay, and the like. A typical cellular assay for assaying βAP production is described in WO9321526. This assay relies on the measurement of a fragment of APP (other than the βAP fragment) which is produced and secreted into the cell culture as a result of βAP production. The secreted fragments comprise a substantially intact amino-terminal sequence of APP terminating within five amino acids of the carboxy-terminal residue (methionine in the case of the normal APP sequence) which lies adjacent to the βAP region and intact APP. In particular, the secreted fragments may consist essentially of sequences which terminate in $Met_{596}$ and $Lys_{595}$ of the 695 amino acid isoform of APP, with corresponding numbering for the other isoforms and corresponding amino acids for the mutant APP forms, such as $Lys_{595}$-$Met_{596}$ to $Asn_{595}$-$Leu_{596}$ for the Swedish mutation.

A preferred detection technique includes a two-site or "sandwich" assay employing a junction-specific antibody as the capture antibody which is bound to a solid phase and a second labelled antibody which binds to an epitope other than that bound by the capture antibody. The second labelled antibody preferably recognizes the amino-terminus of βAP and may be conveniently raised against a synthetic peptide consisting essentially of amino acid residues 1-16 of βAP.

Alternatively, βAP production may be monitored in an animal model, such as the mouse animal model disclosed in WO9119810. An animal model that expresses another APP isotype and/or variant may also be used to screen aspartyl protease inhibitors. Testing in an animal model will typically be performed in addition to the cellular assay described above. Thus, the present invention also provides a process of identifying βAP production inhibitors, comprising (1) measuring βAP production in a cellular assay; and (2) measuring βAP production in a body fluid of a test animal. The βAP production in a body fluid of a test animal may be measured by assaying for the presence of βAP or a βAP fragment in a body fluid of the test animal before and after the administering of an aspartyl protease inhibitor.

The present invention provides methods for inhibiting β-amyloid production in cells, comprising administering to the cells an aspartyl protease inhibitor selected by the process described above. For example, the aspartyl protease inhibitor may be added to a cell culture in order to inhibit βAP production by the cultured cells. Alternatively, the aspartyl protease inhibitor may be administered to a mammal in need thereof in order to inhibit the production of β-amyloid and subsequently the deposition of amyloid plaque associated with AD and other βAP-related diseases.

The following Assays are offered by way of illustration, not by way of limitation. These Assays were carried out to demonstrate the ability of the compounds used in the methods of the present invention to inhibit aspartyl proteases, particularly cathepsin D.

## Assay 1

### CATHEPSIN D INHIBITION ACTIVITY (NON-CELLULAR ASSAY)

Human brain cathepsin D was purified from frozen post-mortem cortex substantially in accordance with the method of Nixon and Morotta, (1984) J. Neurochem. 43:507-516. The cortex material was prepared and applied to a DEAE-Sepharose® column. The flow through fraction was dialyzed against 50mM Tris-HCl, pH 7.5, and applied to concanavalin A Sepharose® and eluted with 50mM Tris, pH 7.5, 0.5M methyl α-D-mannopyranoside. The eluted cathepsin D activity was dialyzed against 25mM Tris, pH 7.5, and stored at -40°C (Con A pool) until use.

For each assay, 50 μL of the Con A pool was diluted to 270 μL in 200mM sodium citrate, pH 4.5, 150mM sodium chloride (NaCl), and incubated for 10 minutes at 4°C with 15 μL of compound stock diluted in dimethylsulfoxide (DMSO). The substrate, porcine renin tetradecapeptide ($CH_3C(O)$-Asp-Arg-Val-Tyr-Ile-His-Pro-Phe-His-Leu-Leu-Val-Tyr-Ser [SEQ ID NO:3], Bachem Bioscience, Inc., Philadelphia, PA), was added to each reaction mixture to yield a final concentration of 44 μM in a total volume of 300 μL. The samples were incubated at 37°C for 0, 10, 20, or 30 minutes, and then boiled for 5 minutes to stop the reaction. The samples were centrifuged in a microcentrifuge for 10 minutes at 14,000 rpm at 4°C. After centrifugation, 200 μL of each reaction was injected onto a C18 RPLC column (Vydac, Hesperia, CA) with a Perkin Elmer ISS 100 autosampler and eluted with a gradient eluent of 0-50% $CH_3CN$ in 0.1% $CF_3CO_2H$ at 2 mL/min. Peptide product peaks were monitored at 220nm with a Perkin Elmer LC 95 detector and integrated with Rainin Dynamax software in order to quantitate activity.

The compounds prepared in Examples 41-48 and pepstatin were assayed for cathepsin D inhibition activity using Assay 1. Results are presented in Assay Table 1 as an IC50 value which was determined by comparing cathepsin D activity in the presence of each aspartyl protease inhibitor with the cathepsin D activity in a control where no such aspartyl protease inhibitor was administered.

### Assay Table 1
### Cathepsin D Activity (Non-cellular)

| Compound | Structure | IC50 (nM) |
|---|---|---|
| Pepstatin | Iva-Val-Val-Sta-Ala-Sta | 0.1 |
| Example 41 | $CH_3C(O)$-Gly-Val-Val-Sta-Ala-Thr-OH | 4 |
| Example 42 | $(CH_3)_2CH$-Gly-Val-Val-Sta-Ala-Thr-OH | 8 |
| Example 43 | Gly-Val-Val-Sta-Ala-Thr-OH | 53 |
| Example 44 | $(CH_3)_2CH$-Gly-Sta-Ile-Ala-Thr-OH | >100 |
| Example 45 | Gly-Val-Sta-Ala-Thr-OH | >250 |
| Example 46 | $(CH_3)_2CH$-Gly-Val-Sta-Ala-Thr-OH | >250 |
| Example 47 | $(CH_3)_2CH$-Gly-Val-Val-Ile-Sta-Thr-OH | >250 |
| Example 48 | $(CH_3)_2CH$-Gly-Val-Sta-Ile-Ala-Thr-OH | >250 |

Assay 2

CATHEPSIN D INHIBITION ACTIVITY (NON-CELLULAR FLUOROMETIC ASSAY)

A fluorometric assay was adapted from the method disclosed by Murakami et al., (1981) Anal. Biochem. 110:232-239 for measuring renin activity. Human liver cathepsin D (Athens Research and Technology, Athens, GA) was diluted in assay buffer, 200mM NaOAc, pH 4.5, 150mM NaCl to 500 ng/mL and then 100 µL of this cathepsin D solution was added to each well of a 96 well plate with the exception of control wells which received just 100 µL of assay buffer. Compound stocks were prepared by dissolving an aspartyl protease inhibitor in DMSO for each concentration tested in the assay and then 5 µL of compound stock was added to each of the wells prepared above. Blank and enzyme control wells each received 5 µL of the DMSO vehicle.

Following a ten minute incubation at 25°C to allow enzyme/compound interaction, 5 µL of 500µM fluorometric substrate (Suc-Arg-Pro-Phe-His-Leu-Leu-Val-Try-AMC [SEQ ID NO:4], Bachem Biosciences, Philadelphia, PA) in DMSO was added per well to initiate the reaction. After incubation at 37°C for 30 minutes, cathepsin D activity was terminated by the addition of 100 µL per well of 400 mU/mL microsomal leucine aminopeptidase (EC 3.4.11.2, Sigma, St. Louis, MO) in 1M Tris-HCl, pH 8.0.

The plates were then analyzed in a fluorometer (CytoFluor 2350, Millipore, Bedford, MA) with an excitation wavelength of 360nm and an emission wavelength of 460nm, in order to check for background fluorescence due to test compounds. Following a two hour incubation at 37°C, to allow the aminopeptidase to release the fluorophore, 7-amido-4-methylcoumarin (AMC) from the products of cathepsin D cleavage, the plates were again analyzed in the fluorometer. In order to check for potential false positives, i.e., inhibitors of microsomal leucine aminopeptidase, residual aminopeptidase activity was monitored directly in each well by the addition of 20 µL/well of 2.5mM Leu-pNA (Bachem Biosciences, Philadelphia, PA) in 10% DMSO. Aminopeptidase activity was measured as an increase in the absorbance at 405nm in a $UV_{max}$ microplate reader (Molecular Devices, Menlo Park, CA).

The compounds prepared in Examples 9, 12, 13, 20-23, 23A, 24, 30, 41-43, 45, and 48 were assayed at 10 µg/mL for cathepsin D inhibition activity using Assay 2. Cathepsin D activity was linear under these conditions and the results are expressed as percent inhibition of the control activity in Assay Table 2, below. All results presented are the mean and standard deviation of at least four replicate assays.

Assay Table 2

| Cathepsin D activity in non-cellular fluorometric assay | |
|---|---|
| Example | Cathepsin D Inhibition |
| 9 | 44.4 ± 1.9% |
| 12 | 12.4 ± 3.7% |
| 13 | 91.4 ± 0.4% |
| 20 | 94.8 ± 0.6% |
| 21 | 78.3 ± 3.8% |
| 23 | 96.3 ± 0.9% |
| 23A | 32.5 ± 4.7% |
| 24 | 92.7 ± 0.5% |
| 30 | 69.2 ± 3.5% |
| 41 | 98.3 ± 0.1% |
| 42 | 89.5 ± 1.3% |
| 43 | 87.9 ± 2.4% |
| 45 | 30.6 ± 4.8% |
| 48 | 79.9 ± 2.7% |

Assay 3

CATHEPSIN D INHIBITION ACTIVITY (NON-CELLULAR ASSAY)

An assay for measuring aspartyl protease activity using a chromogenic substrate was used to determine the inhibition constant, $K_i$, for the test compounds Frozen human placental tissue was thawed and homogenized in 10mM Tris-HCl, pH 7.4, and 0.5% Brij 35 in a Waring blender. The homogenate was centrifuged at 10,000 rpm for 30 minutes in a Sorvall GSA rotor. The supernatant was adjusted to pH 3.7 using 5.7N HCl and then sodium acetate was added to a concentration of 0.1M. The resultant mixture was incubated for 30 minutes at 0°C, resulting in the formation of a precipitate. This precipitate was removed by centrifugation (30 minutes, 10,000 rpm). The acidic supernatent was applied to a chromatography column (pepstatinyl agarose equilibrated with 0.1M sodium acetate, pH 3.5, 0.1% Brij 35, and 1M NaCl), washed until the $OD_{280}$ of the effluent returned to baseline, and then eluted with 50mM Tris-HCl, pH 8.6, 0.1% Brij 35, and 1M NaCl. The eluent was dialyzed against 10mM sodium phosphate, pH 7.0, 0.1% Brij 35, and 0.1M NaCl, and then applied to a 1 mL DEAE-Sephacel column equilibrated with the same buffer. Cathepsin D was recovered in the breakthrough fraction.

The enzyme mixture (125 μL buffer (0.1M sodium formate at pH 3.7), 1 μL of a 1mM solution of test compound, 4 μL of DMSO, enzyme and water to provide a total volume of 230 μL) was vortexed and prewarmed for four minutes before the addition of the chromogenic substrate. After warming, the enzyme mixture was added to 20 μL of a 625 μL dilution of substrate solution. A control reaction mixture was similarly prepared without the addition of the the test compound. The percent of inhibition of the initial rate of reaction in the presence of a particular concentration of test compound (i.e. 4 μM) may be determined for the test compound by monitoring the reaction mixture in parallel to the control reaction.

The decrease in absorbance in the range of 284-324 nm was followed on a Hewlett-Packard 8452A diode array spectrophotometer equipped with a multi-cell transport and thermostatted at 37°C. Absorbance readings were taken at 0.1 second intervals every 2 nm over the targeted range. Each cuvette was sampled five times; the resultant readings were averaged for each time point. For each cuvette, the reading were repeated with a cycle time of 17.2 seconds for approximately 1000 seconds.

The initial velocity was calculated from the slope during the linear phase of the reaction and then plotted versus substrate concentration at the start of the reaction. These data were fitted to the standard Michaelis-Menten equation by Marquardt analysis to yield the calculated value, $K_m$, for the substrate. The values of $K_i$ were estimated using the Dixon construction, and then calculated using a nonlinear curve-fitting program ($[E]_t/K_i < 0.2$) or the Henderson method ($0.2 < [E]_t/K_i < 10$) as described above.

The compounds prepared in Examples 1-11, 14-19, 22-28 and 30-37 were assayed for cathepsin D inhibition activity using Assay 3. The results are presented in Assay Tables 3A and 3B.

Assay Table 3A

| Example | $K_i$ (μ M) |
|---|---|
| 1 | 1.16 ± 0.26 |
| 2 | 0.75 ± 0.14 |
| 4 | 6.20 |
| 5 | 0.24 |
| 6 | 1.80 ± 0.26 |
| 7 | 0.42 ± 0.06 |
| 8 | 1.17 |
| 9 | 0.82 |
| 10 | 5.51 |
| 17 | 2.3 ± 0.2 |
| 18 | 2.2 ± 0.2 |
| 22 | 0.9 ± 0.1 |
| 23 | 0.073 |
| 24 | 0.042 |
| 25 | 1.44 ± 0.26 |
| 26 | 2.30 ± 0.41 |
| 33 | 0.023 ± 0.003 |
| 34 | 0.018 |
| 35 | 0.086 |
| 36 | 0.00039 ± 0.00004 |
| 37 | 0.00054 ± 0.00007 |
| 38 | 0.00047 ± 0.00007 |
| 39 | 0.00053 ± 0.00009 |
| 40 | 0.00098 ± 0.00012 |

Assay Table 3B

| Example | % inhibition at 4μ M |
|---|---|
| 3 | 40 |
| 11 | 32 |
| 14 | 22 |
| 15 | 49 |
| 16 | 36 |
| 19 | 48 |
| 27 | 40 |
| 28 | 6 |
| 30 | 28 |
| 31 | 43 |
| 32 | 12 |

Assay 4

βAP PRODUCTION INHIBITION (CELLULAR ASSAY)

Two cell lines (human kidney cell line 293 and Chinese hamster ovary cell line CHO) were stably transfected with the gene for APP-751 containing the double mutation $Lys_{651}$-$Met_{652}$ to $Asn_{651}$-$Leu_{652}$ (APP-751 numbering) commonly called the Swedish mutation using the method described in Citron et al., (1992) Nature 360:672-674. The transfected cell lines were designated as 293 751 SWE and CHO 751 SWE, and were plated in Corning 96 well plates at $2.5x10^4$ or $1x10^4$ cells per well respectively in Dulbecco's minimal essential media plus 10% fetal bovine serum. Following overnight incubation at 37°C in an incubator equilibrated with 10% carbon dioxide ($CO_2$), the media were removed and replaced with 200 μL per well of media containing an aspartyl protease inhibitor. After a two hour pretreatment period, the media were again removed and replaced with fresh media containing the aspartyl protease inhibitor and the cells were incubated for an additional two hours.

Aspartyl protease inhibitor stocks were prepared in DMSO such that at the final concentration used in the treatment, the concentration of DMSO did not exceed 0.5%. After treatment, plates were centrifuged in a Beckman GPR at 1200 rpm for five minutes at room temperature to pellet cellular debris from the conditioned media. From each well, 100μL of conditioned media were transferred into an ELISA plate precoated with antibody 266 against βAP-13-28 (Seubert et al., *supra*.) and stored at 4°C overnight. An ELISA assay employing labelled antibody 6C6 (against βAP-1-16) was run the next day to measure the amount of βAP produced.

Cytotoxic effects of the compounds were measured by a modification of the method of Hansen et al., (1989) J. Immun. Meth. 119:203-210. To the cells remaining in the tissue culture plate, was added 25 μL of a 3,(4,5-dimethylthiazol-2-yl)2,5-diphenyltetrazolium bromide (MTT) stock solution (5 mg/mL) to a final concentration of 1 mg/mL. Cells were incubated at 37°C for one hour, and cellular activity was stopped by the addition of an equal volume of MTT lysis buffer (20% w/v sodium dodecylsulfate in 50% DMF, pH 4.7). Complete extraction was achieved by overnight shaking at room temperature. The difference in the $OD_{562nm}$ and the $OD_{650nm}$ was measured in a Molecular Devices $UV_{max}$ microplate reader as an indicator of the cellular viability.

The results of the βAP ELISA were fit to a standard curve and expressed as ng/mL βAP peptide. In order to normalize for cytotoxicity, these βAP results were divided by the MTT results and expressed as a percentage of the results from a drug-free control.

The compounds prepared above in Examples 9, 12, 13, 20-23, 23A, 24, 30, 41-43, 45, and 48 were assayed for βAP production inhibition activity in cells at 10 μg/mL using Assay 4. The results presented in Assay Table 4, below, are the mean and standard deviation of at least six replicate assays.

Assay Table 4

| βAP production inhibition activity in cells | |
|---|---|
| Example | βAP Production Inhibition |
| 9 | 44.4 ± 7.7% |
| 12 | 12.4 ± 6.0% |
| 13 | 91.4 ± 3.9% |
| 20 | 94.8 ± 3.1% |
| 21 | 78.3 ± 1.5% |
| 23 | 96.3 ± 2.8% |
| 23A | 32.5 ± 3.3% |
| 24 | 92.7 ± 1.4% |
| 30 | 69.2 ± 2.2% |
| 41 | -6.2 ± 6.2% |
| 42 | 13.1 ± 7.5% |
| 43 | 16.2 ± 8.5% |
| 45 | 3.3 ± 1.1% |
| 48 | 1.6 ± 19.8% |

The compounds of the present invention can be administered for prophylactic and/or therapeutic treatment of diseases related to the deposition of βAP, such as AD, Down's syndrome, and advanced aging of the brain. In therapeutic applications, the compounds are administered to a host already suffering from the disease. The compounds will be administered in an amount sufficient to inhibit further deposition of βAP plaque. The specific dose of compound administered according to this invention to obtain therapeutic and/or prophylactic effects will, of course, be determined by the particular circumstances surrounding the case, including, for example, the compound administered, the route of administration, the condition being treated, the individual being treated and the like. A typical daily dose will contain a dosage level of from about 0.01 mg/kg to about 50 mg/kg of body weight of an active compound of this invention. Preferred daily doses generally will be from about 0.05 mg/kg to about 20 mg/kg and ideally from about 0.1 mg/kg to about 10 mg/kg.

For prophylactic applications, the compounds of the present invention are administered to a host susceptible to Alzheimer's Disease or a βAP-related disease, but not already suffering from such disease. Such hosts may be identified by genetic screening and clinical analysis, as described in the medical literature. See e.g., Goate, (1991) Nature 349:704-706. The compounds will be able to inhibit or prevent the formation of the βAP plaque at a symptomatically early stage, preferably preventing even the initial stages of the β-amyloid disease.

The compounds can be administered by a variety of routes including oral, rectal, transdermal, subcutaneous, intravenous, intramuscular and intranasal. The compounds of the present invention are preferably formulated prior to administration.

The active ingredient in such formulations comprises from 0.1% to 99.9% by weight of the formulation. By "pharmaceutically acceptable" it is meant that the carrier, diluent or excipient is compatible with the other ingredients of the formulation and not deleterious to the recipient thereof.

For example, a typical pharmaceutical composition for intramuscular injection would contain about one μ g to one mg of the compound in from one to four milliliters of sterile buffered water. The typical pharmaceutical composition for intravenous infusion would contain about one to one hundred milligram of the compound in from one hundred to five hundred milliliters of sterile Ringer's solution.

The pharmaceutical formulations are prepared by known procedures using known and readily available ingredients. In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the

form of tablets, pills, powders, lozenges, sachets, cachets, elixirs, suspensions, emulsions, solutions, syrups, aerosols, (as a solid or in a liquid medium), ointments containing, for example, up to 10% by weight of the active compound, soft and hard gelatin capsules, suppositories, sterile injectable solutions, sterile packaged powders and the like.

Frequently, it will be desirable or necessary to introduce the pharmaceutical compositions directly or indirectly to the brain. Direct techniques usually involve placement of a drug delivery catheter into the host's ventricular system to bypass the blood-brain barrier. Indirect techniques, which are generally preferred, involve formulating the compositions to provide for drug latentiation by the conversion of hydrophilic drugs into lipid-soluble drugs. Latentiation is generally achieved through blocking of the hydroxyl, carboxyl, and primary amine groups present on the drug to render the drug more lipid-soluble and amenable to transportation across the blood-brain barrier. Alternatively, the delivery of hydrophilic drugs can be enhanced by intra-arterial infusion of hypertonic solutions which can transiently open the blood-brain barrier.

## Claims

1. The use of an aspartyl protease inhibitor, or a pharmaceutically acceptable salt thereof, for the manufacture of a medicament for inhibiting β-amyloid peptide (βAP) production.

2. The use according to claim 1 wherein the aspartyl protease inhibitor is a cathepsin D inhibitor.

3. The use according to claim 1 wherein the aspartyl protease inhibitor is a compound of formula I

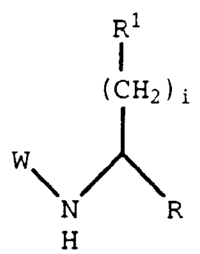

(I)

wherein:

i is 1, 2, 3, or 4;

$R^1$ is $C_1$-$C_6$ alkyl, aryl, cyclohexyl or -S-$R^{1x}$, where

$R^{1x}$ is aryl, or cyclohexyl;

W is

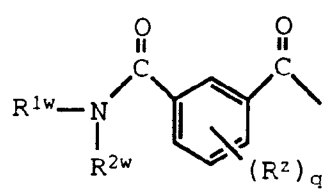

;

where

$R^{1w}$ and $R^{2w}$ are independently selected from hydrogen, $C_1$-$C_6$ alkyl, aryl($C_1$-$C_4$)alkyl, heterocycle($C_1$-C4)alkyl, unsaturated heterocycle($C_1$-$C_4$)alkyl, or $R^{1w}$ and $R^{2w}$ are combined with the nitrogen atom to which they are attached to form a heterocycle or unsaturated heterocycle with the proviso that the nitrogen atom may not be quaternized;

q is 0, 1, 2, 3, or 4;

$R^z$ is hydrogen, halo, $C_1$-$C_6$ alkyl, $C_1$-$C_6$ alkoxy, $C_1$-$C_6$ alkylthio, or amino;

or W is

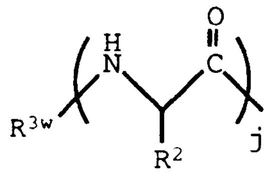
;

where

j is 1, or 2;

$R^{3w}$ is hydrogen, formyl, carbamoyl, or $-(B)_e-X-R^0$, where

B is -C(O)-, or $-S(O)_k-$;

k is 0, 1, or 2;

e is 0, or 1;

X is $-(CH_2)_g-$, $-(CH_2)_m-O-(CH_2)_n-$, or $-(CH_2)_m-NR^x-(CH_2)_n-$, where

g is 0, 1, or 2;

m and n are independently 0, 1, or 2;

$R^x$ is hydrogen, or $C_1$-$C_4$ alkyl;

$R^0$ is $C_1$-$C_6$ alkyl, $C_1$-$C_4$ alkoxycarbonyl, formyl, $C_2$-$C_6$ alkanoyl, amino, trifluoromethyl, $C_1$-$C_4$ alkylamino, di($C_1$-$C_4$)alkylamino, aryl, heterocycle, or unsaturated heterocycle;

each $R^2$ is independently an amino acid side chain, $-CH_2CH_3$, $-CH_2CH_2CH_3$, $-CH_2(CH_2)_2CH_3$, $-C(CH_3)_3$, $-CH_2SO_2NH_2$, $-CH_2CN$, $-(CH_2)_r-X^1-R^{2a}$, $-(CH_2)_s-C(O)NR^{2b}R^{2c}$, or $-(CH_2)_t-S(O)_k-[1-N(R^{2d})$-tetrazol-5-yl], where

r, s and t are independently 0, 1, or 2;

$X^1$ is a bond, divalent($C_1$-$C_4$)alkyl, divalent($C_2$-$C_4$)alkenyl, divalent($C_2$-$C_4$)alkynyl, -C(O)-O-, -O-C(O)-, $-C(O)NR^{2b}-$, $-NR^{2b}-C(O)-$, $-NR^{2b}-$, -C(O)-, -O-, or $-S(O)_k-$;

$R^{2a}$ is $C_1$-$C_6$ alkyl, aryl, aryl($C_1$-$C_4$)alkyl, heterocycle, heterocycle($C_1$-$C_4$)alkyl, unsaturated heterocycle, or unsaturated heterocycle($C_1$-$C_4$)alkyl;

$R^{2b}$ is hydrogen, or $C_1$-$C_4$ alkyl;

$R^{2c}$ is amino, $C_1$-$C_6$ alkoxy, $C_1$-$C_6$ alkyl, $-(CH_2)_m$-di($C_1$-$C_4$)alkylamino, aryl, aryl($C_1$-$C_4$)alkyl, heterocycle, heterocycle($C_1$-$C_4$)alkyl, unsaturated heterocycle, or unsaturated heterocycle($C_1$-$C_4$)alkyl;

$R^{2d}$ is hydrogen, $C_1$-$C_6$ alkyl, aryl, unsaturated heterocycle, unsaturated heterocycle($C_1$-$C_4$)-alkyl, or aryl($C_1$-$C_4$)alkyl;

R is

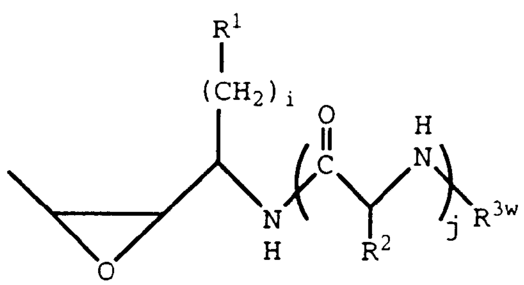
,

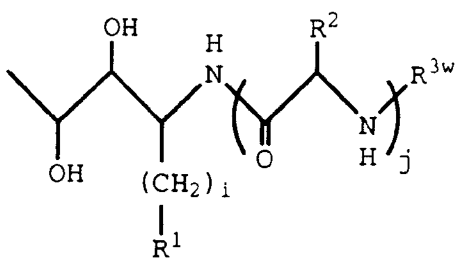
,

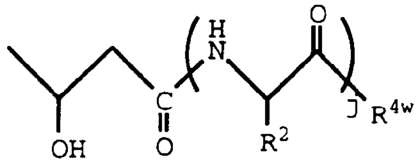 ,

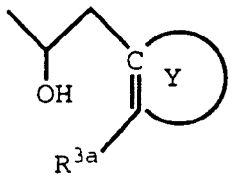 ,

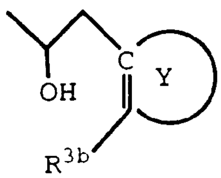

or

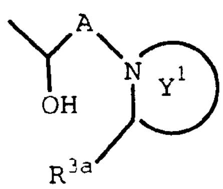 ;

where:

A is $-CH_2-$ or

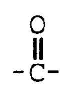 ;

Y is aryl or unsaturated heterocycle;

$Y^1$ is heterocycle;

$R^{4w}$ is hydroxy or amino;

$R^{3a}$ is a group having the formula

1) $-C(O)-NR^4R^4$,

2)

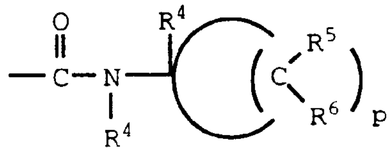 ,

or

3)

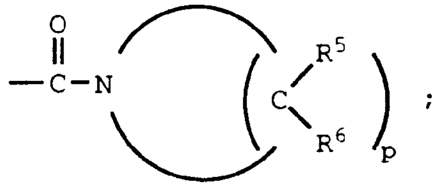

$R^{3b}$ is a group having the structure:

1)

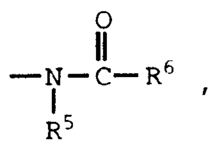

2)

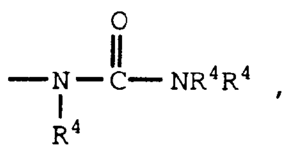

or

3)

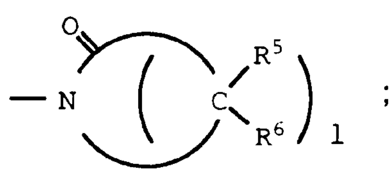

p is 4 or 5;

l is 3, 4 or 5;

$R^4$ at each occurrence is independently hydrogen, $C_1$-$C_6$ alkyl or hydroxy($C_1$-$C_4$)alkyl;

$R^5$ and $R^6$ are independently selected from hydrogen, hydroxy, $C_1$-$C_6$ alkyl, $C_1$-$C_6$ alkoxy, amino, $C_1$-$C_4$ alkylamino, hydroxy($C_1$-$C_4$)alkyl, carboxy, $C_1$-$C_4$ alkoxycarbonyl, carbamoyl, N-($C_1$-$C_4$)alkylcarbamoyl, aryl, heterocycle or unsaturated heterocycle;

with the provisos that:

(1) when R is

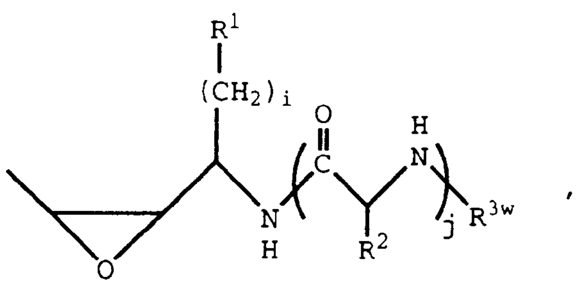

then

W is

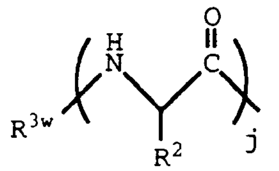

;

$R^{3w}$ is formyl, carbamoyl or $-(B)_e-X-R^0$, where

e is 1; and

B is -C(O)-;

(2) when R is

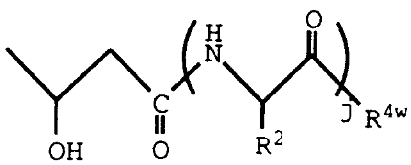

;

then

W is

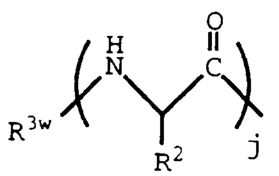

;

and

j is 2; and

(3) when R is

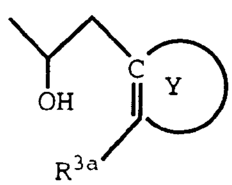

,

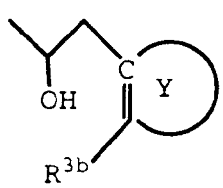

or

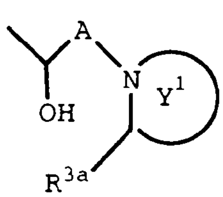

;

then

i is 1; and

$R^1$ is aryl, cyclohexyl, or -S-$R^{1x}$, where

$R^{1x}$ is aryl, or cyclohexyl;

or a pharmaceutically acceptable salt thereof.

4. The use according to claim 3 wherein the aspartyl protease inhibitor is a compound of formula I wherein:

i is 1;

$R^1$ is $-CH(CH_3)_2$, or phenyl;

W is

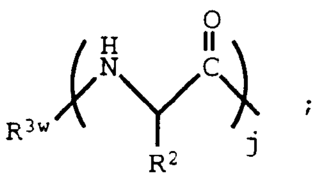

where

$R^{3w}$ is hydrogen or -B-X-$R^0$, where

B is -C(O)-, or $-S(O)_2-$;

X is $-(CH_2)_g-$, or $-(CH_2)_m-O-(CH_2)_n-$, where

g is 0;

m and n are independently 0, 1, or 2;

$R^0$ is $C_1$-$C_6$ alkyl, aryl, or unsaturated heterocycle;

$R^2$ is an amino acid side chain, $-CH_2CH_3$, $-CH_2CH_2CH_3$, $-CH_2(CH_2)_2CH_3$, $-CH_2CN$, or $-CH_2-R^{2a}$, where

$R^{2a}$ is aryl

R is

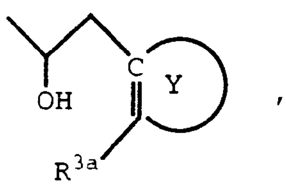

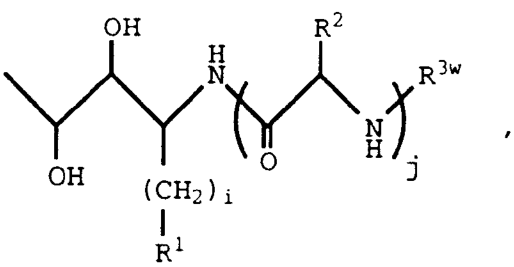

or

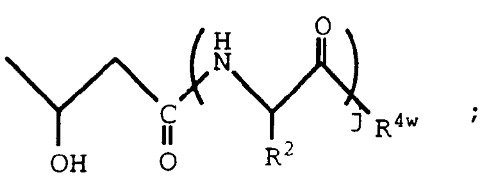

where:

$R^{4w}$ is amino;

Y is phenyl; and
$R^{3a}$ is -C(O)NH(*t*-butyl);
with the proviso that when R is

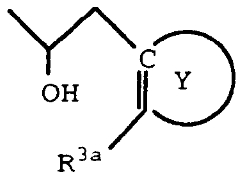 ;

then j is 2;
or a pharmaceutically acceptable salt thereof.

**5.** The use according to claim 1 wherein the aspartyl protease inhibitor is a compound of formula II

$$R^7\text{-Gly-}(R^8)_f\text{-Thr-OH} \qquad II$$

wherein:
$R^7$ is $C_1$-$C_6$ alkyl, formyl or $C_2$-$C_6$ alkanoyl;
f is 1-6;
each $R^8$ is independently an amino acid residue, statine or a statine derivative;
with the provisos that:
(1) when f is 1, then $R^8$ must be statine or a statine derivative;
(2) when f is 3-6, then the total number of statine and statine derivatives in $-(R^8)_f-$ cannot exceed 2;
or a pharmaceutically acceptable salt thereof.

**6.** The use of an aspartyl protease inhibitor, or a pharmaceutically acceptable salt thereof, for the manufacture of a medicament for preventing or treating Alzheimer's Disease (AD).

**7.** The use of an aspartyl protease inhibitor, or a pharmaceutically acceptable salt thereof, for the manufacture of a medicament for treating or preventing βAP-related diseases.

**8.** A process for identifying a βAP production inhibitor, comprising
(1) administering an aspartyl protease inhibitor to a test system;
(2) measuring βAP production in the test system; and
(3) comparing βAP production in the test system to βAP production in a control test system in which no such aspartyl protease inhibitor has been administered.

**9.** A process according to claim 8, wherein the aspartyl protease inhibitor is identified by measuring the activity of an aspartyl protease in a non-cellular assay in presence and absence of a test compound.

**10.** A process according to claim 8, wherein βAP production is measured by
(1) exposing the aspartyl protease inhibitor to a cellular assay; and
(2) measuring a cellular characteristic which is related to βAP production.

**11.** A process of identifying a βAP production inhibitor, comprising:
(1) identifying an aspartyl protease inhibitor;
(2) measuring βAP production in a cellular assay; and
(3) measuring βAP production in a body fluid of a test animal.

European Patent Office

# EUROPEAN SEARCH REPORT

Application Number
EP 94 30 5833

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | EP-A-0 569 777 (MILES INC) *as a whole, i.e. pages 5, 28, 33-34, claim 19* | 1,2,6-11 | A61K31/47 A61K31/44 A61K31/415 A61K31/165 A61K31/335 A61K31/27 A61K31/215 A61K31/38 A61K31/40 A61K31/18 A61K31/495 A61K31/17 A61K31/195 C07K5/02 C07K5/06 C07K7/02 C12Q1/37 G01N33/68 |
| P,X | WO-A-93 16101 (ATHENA NEUROSCIENCES, INC.) *pages 4, 9, 13 and 14 and claims 1 and 5* | 1,2,6-11 | |
| X | WO-A-93 07872 (THE UNIVERSITY OF NOTTINGHAM) | 1,2,6,7 | |
| Y | * example 8 * | 3-5 | |
| Y | J. BIOL. CHEM., vol.268, no.22, 5 August 1993 pages 16602 - 16609 R. SIMAN ET. AL. 'Processing of the beta-amyloid precursor' * abstract * | 3-5 | |
| Y | BIOCHEMISTRY, vol.26, 1987 pages 8083 - 8086 A. MATUS ET AL. 'Age-related increase in a cathepsin D like protease that degrades brain microtubule-associated proteins' * the whole document * | 3-5 | **TECHNICAL FIELDS SEARCHED (Int.Cl.6)** A61K C07K C12Q G01N |
| Y | EP-A-0 526 009 (ELI LILLY AND COMPANY) * the whole document * | 3-5 | |
| P,Y | EP-A-0 575 097 (ELI LILLY AND COMPANY) * the whole document * | 3-5 | |
| P,Y | EP-A-0 604 184 (ELI LILLY AND COMPANY) * the whole document * | 3-5 | |
| P,Y | EP-A-0 604 186 (ELI LILLY AND COMPANY) * the whole document * | 3-5 | |

-/--

The present search report has been drawn up for all claims

| Place of search | Date of completion of the search | Examiner |
|---|---|---|
| MUNICH | 23 December 1994 | Foerster, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 5833

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 431 784 (ELI LILLY AND COMPANY)<br>--- | 3-5 | A61K38/55 |
| Y | J. BIOL. CHEM.,<br>vol.266, no.22, 1991<br>pages 14548 - 14553<br>A. TOMASELLI ET AL. 'Actin, troponin C, Alzheimer amyloid precursor protein and pro-interleukin 1 beta as substrates of the protease from Human Immundeficiency Virus'<br>* the whole document *<br>----- | 3-5 | |
| | | | TECHNICAL FIELDS SEARCHED (Int.Cl.6) |

The present search report has been drawn up for all claims

| Place of search | Date of completion of the search | Examiner |
|---|---|---|
| MUNICH | 23 December 1994 | Foerster, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)